(19) Europäisches Patentamt
European Patent Office
Office européen des brevets

(11) EP 1 431 280 A1

(12) **EUROPEAN PATENT APPLICATION**
published in accordance with Art. 158(3) EPC

(43) Date of publication:
**23.06.2004 Bulletin 2004/26**

(21) Application number: **02760763.9**

(22) Date of filing: **28.08.2002**

(51) Int Cl.7: **C07C 307/02**, C07C 327/44, C07C 317/46, A61K 31/18, A61K 31/277, A61P 5/24, A61P 13/08, A61P 35/00, A61P 43/00

(86) International application number:
**PCT/JP2002/008665**

(87) International publication number:
**WO 2003/020690 (13.03.2003 Gazette 2003/11)**

(84) Designated Contracting States:
**AT BE BG CH CY CZ DE DK EE ES FI FR GB GR IE IT LI LU MC NL PT SE SK TR**
Designated Extension States:
**AL LT LV MK RO SI**

(30) Priority: **29.08.2001 JP 2001260002**

(71) Applicant: **Kyowa Hakko Kogyo Co., Ltd**
**Tokyo 100-8185 (JP)**

(72) Inventors:
- **INO, Yoji, Kyowa Hakko Co., Ltd**
  **Shunto-gun, Shizuoka 411-8731 (JP)**
- **AMISHIRO, Nobuyoshi**
  **Champaign, IL 61822 (US)**
- **MURAMATSU, Kozue,**
  **Kyowa Hakko Kogyo Co. ,Ltd.**
  **Shunto-gun, Shizuoka 411-8731 (JP)**
- **SHIOTSU, Yukimasa,**
  **Kyowa Hakko Kogyo Co., Ltd**
  **Shunto-gun, Shizuoka 411-8731 (JP)**
- **MURAKATA, Chikara,**
  **Kyowa Hakko Kogyo Co., Ltd.**
  **Shunto-gun, Shizuoka 411-873 (JP)**

(74) Representative: **VOSSIUS & PARTNER**
**Siebertstrasse 4**
**81675 München (DE)**

(54) **ARYL SULFAMATE DERIVATIVES**

(57) The present invention provides an aryl sulfamate derivative represented by Formula (I)

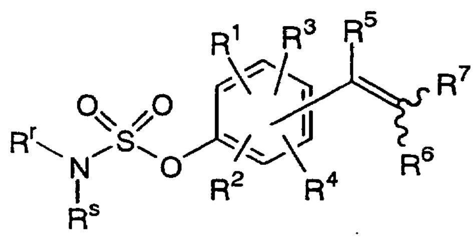

(I)

(wherein $R^r$ and $R^s$, which may be the same or different, each represent a hydrogen atom or lower alkyl, $R^1$, $R^2$, $R^3$ and $R^4$, which may be the same or different, each represent a hydrogen atom, lower alkyl, a halogen atom, nitro, cyano, azido, or the like, $R^5$ represents a hydrogen atom, substituted or unsubstituted alkyl, substituted or unsubstituted lower cycloalkyl, or the like, $R^6$ represents a hydrogen atom, substituted or unsubstituted alkyl, a halogen atom, or the like, or $R^5$ and $R^6$ are joined to form a bond, and $R^7$ represents $X^1NR^{23}R^{24}$ or $COR^{26}$), or a pharmaceutically acceptable salt thereof.

EP 1 431 280 A1

## Description

Technical field

The present invention relates to aryl sulfamate derivatives or pharmaceutically acceptable salts thereof which have an inhibitory activity against steroid sulfatase, and which are useful in treating or preventing steroid hormone dependent diseases, or which have a memory enhancement action, and which are useful in treating or preventing memory disorders.

Background art

In post-menopausal women, the estrogen levels in breast tumors are at least ten times higher than that in the blood plasma. The high estrogen levels in breast tumors are thought to result from estrone sulfate being hydrolyzed to estrone by steroid sulfatase (estrone sulfatase). Therefore, steroid sulfatase inhibitors are thought to be effective therapeutic agents in the treatment of estrone dependent breast cancer. In addition, it is thought that steroid sulfatase inhibitors are also useful in the prevention or treatment of other diseases to which estrones are related, such as endometrial cancer, ovarian cancer, endometriosis, adenomyosis of the uterus, or the like. Furthermore, because steroid sulfatase also relates to the biosynthesis of androgen, steroid sulfatase inhibitors are thought to be useful also for the prevention or treatment of diseases to which androgens are related, such as prostate cancer or the like.

Although estrone-3-sulfamate (EMATE) is reported as a typical steroid sulfatase inhibitor [International Journal of Cancer, vol. 63, p. 106 (1995); US Patent No. 5,616,574], because EMATE has an estrogenic effect, it is shown that it is not useful in the treatment of estrone dependent diseases [Cancer Research, vol. 56, p. 4950 (1996)].

Additionally, so far many non-steroidal steroid sulfatase inhibitors have been discovered [Expert Opinion on Therapeutic Patents, vol. 9, p. 1083 (1999)].

Examples include tyramine derivatives (Compound A1) [Cancer Research, vol. 57, p. 702 (1997); The Journal of Steroid Biochemistry and Molecular Biology, vol. 59, p. 41 (1996); The Journal of Steroid Biochemistry and Molecular Biology, vol. 68; p. 31 (1999); The Journal of Steroid Biochemistry and Molecular Biology, vol. 69, p. 227 (1999); US Patent No. 5,567,831], cinnamic acid derivatives (Compound A2 and Compound A3) (US Patent No. 6,011,024), diethyl stilbestrol derivatives [The Journal of Steroid Biochemistry and Molecular Biology, vol. 69, p. 227 (1999)], and the like. In addition, several steroid sulfatase inhibitors have been disclosed more recently (for example Compound A4) (WO 01/04086, WO 01/02349).

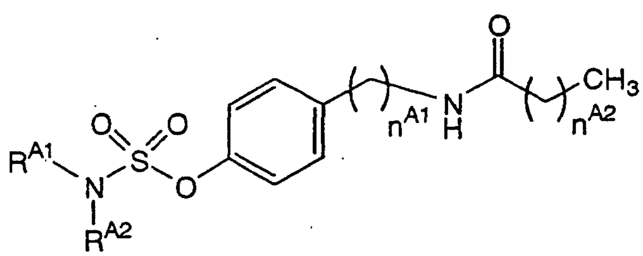

(A1)

(wherein $R^{A1}$ and $R^{A2}$ represent a hydrogen atom or lower alkyl, $n^{A1}$ represents an integer of from 0 to 4, and $n^{A2}$ represenrs an integer of from 5 to 14)

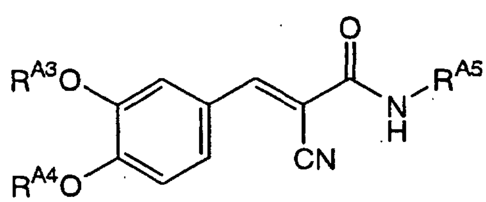

(A2)

[wherein at least one of $R^{A3}$and $R^{A4}$ represents $SO_2NR^{A6}R^{A7}$ (wherein $R^{A6}$ and $R^{A7}$, which may be the same or different, each represent a hydrogen atom or methyl), and the other represents a hydrogen atom or $SO_2NR^{A6}R^{A7}$ (wherein $R^{A6}$ and $R^{A7}$ have the same meanings as previously described, respectively), and $R^{A5}$ represents a hydrogen atom or lower alkyl having 1 to 6 carbon atoms]

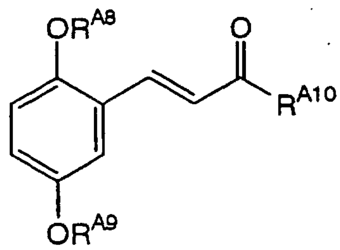

(A3)

[wherein at least one of $R^{A8}$ and $R^{A9}$ represents $SO_2NR^{A11}R^{A12}$ (wherein $R^{A11}$ and $R^{A12}$, which may be the same or different, each represent a hydrogen atom or methyl), and the other represents a hydrogen atom or $SO_2NR^{A11}R^{A12}$ (wherein $R^{A11}$ and $R^{A12}$ have the same meanings as previously described, respectively), and $R^{A10}$ represents methoxy or ethyl]

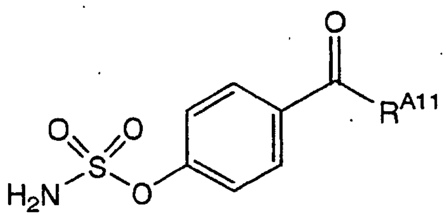

(A4)

(wherein $R^{A11}$ represents a hydrogen atom, substituted or unsubstituted alkyl or the like)

In addition, it is known that steroid sulfamate has a memory enhancement action (US Patent No. 5,556,847).

As reported in WO99/33858, compounds that have not only an inhibitory activity against steroid sulfatase but also an anti-estrogenic activity (an inhibitory activity against the action of estrogen or an inhibitory activity against estrogen biosynthesis) are thought to be useful in the treatment or prevention of steroid hormone dependent diseases. In addition, for aryl sulfamate derivatives having an inhibitory activity against steroid sulfatase, that the phenol derivative (a desulfamoylated form) generated by hydrolysis of sulfamate in a living body has an anti-estrogenic activity is also thought to be useful.

<u>Disclosure of the invention</u>

The object of the present invention is to provide aryl sulfamate derivatives or pharmaceutically acceptable salts thereof, which have an inhibitory activity against steroid sulfatase and which are useful in treating or preventing steroid hormone dependent diseases.

The present invention relates to the following (1) ~ (40).

(1) An aryl sulfamate derivative represented by Formula (I)

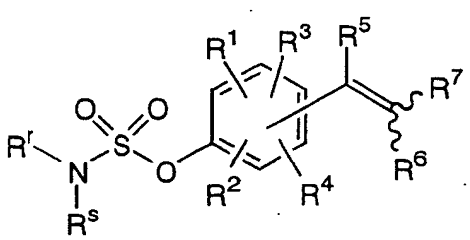

(I)

{wherein $R^r$ and $R^s$, which may be the same or different, each represent a hydrogen atom or lower alkyl, $R^1$, $R^2$, $R^3$ and $R^4$, which may be the same or different, each represent a hydrogen atom, lower alkyl, a halogen atom, nitro, cyano, azido, $OR^8$ [wherein $R^8$ represents a hydrogen atom, lower alkyl, lower alkanoyl or $SO_2NR^{ra}R^{sa}$ (wherein $R^{ra}$ and $R^{sa}$ have the same meanings as the previously described $R^r$ and $R^s$, respectively)], $COR^9$ [wherein $R^9$ represents a hydrogen atom, lower alkyl, hydroxy, lower alkoxy or $NR^{10}R^{11}$ (wherein $R^{10}$ and $R^{11}$, which may be the same or different, each represent a hydrogen atom or lower alkyl)] or $NR^{12}R^{13}$ [wherein $R^{12}$ represents a

hydrogen atom or lower alkyl, and $R^{13}$ represents a hydrogen atom, lower alkyl, lower alkanoyl, or $SO_2NR^{rb}R^{sb}$ (wherein $R^{rb}$ and $R^{sb}$ have the same meanings as the previously described $R^r$ and $R^s$, respectively)], $R^5$ represents a hydrogen atom, substituted or unsubstituted alkyl, substituted or unsubstituted lower cycloalkyl, a halogen atom, nitro, cyano, azido, isocyano, substituted or unsubstituted lower alkynyl, substituted or unsubtitued lower alkenyl, hydroxy, substituted or unsubstituted alkoxy, $OSO_2NR^{rc}R^{sc}$ (wherein $R^{rc}$ and $R^{sc}$ have the same meanings as the previously described $R^r$ and $R^s$, respectively), $COR^{14}$ [wherein $R^{14}$ represents a hydrogen atom, substituted or unsubstituted alkyl, substituted or unsubstituted lower cycloalkyl, substituted or unsubstituted aryl, a substituted or unsubstituted heterocyclic group, $OR^{15}$ (wherein $R^{15}$ repreesnts a hydrogen atom, substituted or unsubstituted alkyl, substituted or unsubstituted lower cycloalkyl, substituted or unsubstituted aryl, or a substituted or unsubstituted heterocyclic group) or $NR^{16}R^{17}$ (wherein $R^{16}$ and $R^{17}$, which may be the same or different, each represent a hydrogen atom, substituted or unsubstituted alkyl, substituted or unsubstituted lower cycloalkyl, substituted or unsubstituted aryl, or a substituted or unsubstituted heterocyclic group)], $NR^{18}R^{19}$ (wherein $R^{18}$ and $R^{19}$, which may be the same or different, each represent substituted or unsubstituted alkyl), $NR^{20}COR^{21}$ (wherein $R^{20}$ and $R^{21}$, which may be the same or different, each represent a hydrogen atom, substituted or unsubstituted alkyl, substituted or unsubstituted lower cycloalkyl, substituted or unsubstituted aryl, or a substituted or unsubstituted heterocyclic group) or $SO_2R^{22}$ (wherein $R^{22}$ represents substituted or unsubstituted alkyl), $R^6$ represents a hydrogen atom, substituted or unsubstituted alkyl, substituted or substituted lower cycloalkyl, a halogen atom, nitro, cyano, azido, isocyano, substituted or unsubstituted lower alkynyl, substituted or unsubstituted lower alkenyl, hydroxy, substituted or unsubstituted alkoxy, substituted or unsubstituted aryl, a substituted or unsubstituted heterocyclic group, $OSO_2NR^{rcx}R^{scx}$ (wherein $R^{rcx}$ and $R^{scx}$ have the same meanings as the previously described $R^r$ and $R^s$, respectively), $COR^{14x}$ (wherein $R^{14x}$ has the same meaning as the previously described $R^{14}$), $NR^{18x}R^{19x}$ (wherein $R^{18x}$ and $R^{19x}$ have the same meanings as the previously described $R^{18}$ and $R^{19}$, respectively), $NR^{20x}COR^{21x}$ (wherein $R^{20x}$ and $R^{21x}$ have the same meanings as the previously described $R^{20}$ and $R^{21}$, respectively) or $SO_2R^{22x}$ (wherein $R^{22x}$ has the same meaning as the previously described $R^{22}$), or $R^5$ and $R^6$ are joined to form a bond, and $R^7$ represents $X^1NR^{23}R^{24}$ [wherein $X^1$ represents C=O or C=S, $R^{23}$ represents a hydrogen atom, substituted or unsubstituted alkyl, substituted or unsubstitued lower cycloalkyl, substituted or unsubstituted aryl, or a substituted or unsubstituted heterocyclic group, and $R^{24}$ represents a hydrogen atom, substituted or unsubstituted alkyl, substituted or unsubstituted lower cycloalkyl, substituted or unsubstituted aryl, a substituted or unsubstituted heterocyclic group or $OR^{25}$ (wherein $R^{25}$ represents a hydrogen atom, substituted or unsubstituted alkyl, substituted or unsubstituted lower cycloalkyl, substituted or unsubstituted aryl, or a substituted or unsubstituted heterocyclic group)] or $COR^{26}$ [wherein $R^{26}$ represents a hydrogen atom, substituted or unsubstituted alkyl, substituted or unsubstituted lower cycloalkyl, substituted or unsubstituted aryl, substituted or a unsubstituted heterocyclic group or $OR^{27}$ (wherein $R^{27}$ represents a hydrogen atom, substituted or unsubstitued alkyl, substituted or unsubstituted lower cycloalkyl, substituted or unsubstituted aryl, or a substituted or unsubstituted heterocyclic group)] with the proviso that (1) when $-CR^5=CR^6R^7$ is placed on the benzene ring at the ortho position to $-OSO_2NR^r=R^s$, one of $R^1$, $R^2$, $R^3$ and $R^4$ is $NH_2$ and is placed on the benzene ring at the para position to $-OSO_2NR^rR^s$, the rest of $R^1$, $R^2$, $R^3$ and $R^4$ as well as $R^5$ are hydrogen atoms and $R^6$ is cyano, then $R^7$ is not carboxy, (2) when one of $R^1$, $R^2$, $R^3$ and $R^4$ is hydroxy or $OSO_2NR^{ra}R^{sa}$ (wherein $R^{ra}$ and $R^{sa}$ have the same meanings as previously described, respectively) and the rest of $R^1$, $R^2$, $R^3$ and $R^4$ are hydrogen atoms, then said hydroxy or said $OSO_2NR^{ra}R^{sa}$ is only placed on the benzene ring at the meta position to $-OSO_2NR^rR^s$, and (3) when $R^5$ is hydroxy, then $R^7$only represents $X^1NR^{23}R^{24}$ (wherein $X^1$, $R^{23}$ and $R^{24}$ have the same meanings as previously described, respectively) or $CO_2R^{27}$ (wherein $R^{27}$ has the same meaning as previously described)}, or a pharmaceutically acceptable salt thereof.

(2) An aryl sulfamate derivative represented by Formula (IA)

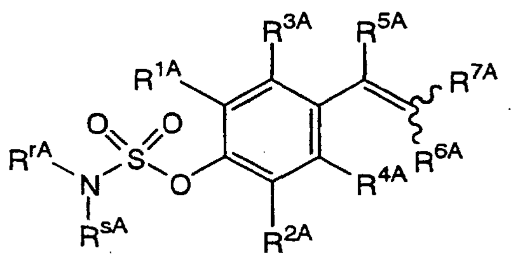

(IA)

[wherein $R^{rA}$, $R^{sA}$, $R^{3A}$, $R^{4A}$, $R^{5A}$, $R^{6A}$ and $R^{7A}$ have the same meanings as the previously described $R^r$, $R^s$, $R^3$, $R^4$, $R^5$, $R^6$ and $R^7$, respectively, and $R^{1A}$ and $R^{2A}$, which may be the same or different, each represent a hydrogen atom, lower alkyl, a halogen atom, nitro, cyano, azido, $OR^{8A}$ (wherein $R^{8A}$ represents lower alkyl or lower alkanoyl),

$COR^{9A}$ (wherein $R^{9A}$ has the same meaning as the previously described $R^9$) or $NR^{12A}R^{13A}$ (wherein $R^{12A}$ and $R^{13A}$ have the same meanings as the previously described $R^{12}$ and $R^{13}$, respectively), with the proviso that when $R^{5A}$ is hydroxy, $R^{7A}$ only represents $X^{1A}NR^{23A}R^{24A}$ (wherein $X^{1A}$, $R^{23A}$ and $R^{24A}$ have the same meanings as the previously described $X^1$, $R^{23}$ and $R^{24}$, respectively) or $CO_2R^{27A}$ (wherein $R^{27A}$ has the same meaning as the previously described $R^{27}$)], or a pharmaceutically acceptable salt thereof.

(3) The aryl sulfamate derivative or the pharmaceutically acceptable salt thereof according to (2), wherein $R^{1A}$ and $R^{2A}$ are hydrogen atoms.

(4) The aryl sulfamate derivative or the pharmaceutically acceptable salt thereof according to (2), wherein at least one of $R^{1A}$ and $R^{2A}$ is a halogen atom.

(5) The aryl sulfamate derivative or the pharmaceutically acceptable salt thereof according to (3) or (4), wherein $R^{5A}$ is a hydrogen atom, substituted or unsubstituted alkyl, substituted or unsubstituted lower cycloalkyl, a halogen atom, nitro, cyano, azido, isocyano, substituted or unsubstituted lower alkynyl, substituted or unsubstituted lower alkenyl, substituted or unsubstituted alkoxy, $OSO_2NR^{rc}R^{sc}$ (wherein $R^{rc}$ and $R^{sc}$ have the same meanings as previously described, respectively), $COR^{14}$ (wherein $R^{14}$ has the same meaning as previously described), $NR^{18}R^{19}$ (wherein $R^{18}$ and $R^{19}$ have the same meanings as previously described, respectively), $NR^{20}COR^{21}$ (wherein $R^{20}$ and $R^{21}$ have the same meanings as previously described, respectively) or $SO_2R^{22}$ (wherein $R^{22}$ has the same meaning as previously described), and $R^{6A}$ is a hydrogen atom, substituted or unsubstituted alkyl, substituted or unsubstituted lower cycloalkyl, a halogen atom, nitro, cyano, azido, isocyano, substituted or unsubstituted lower alkynyl, substituted or unsubstituted lower alkenyl, substituted or unsubstituted alkoxy, substituted or unsubstituted aryl, a substituted or unsubstituted heterocyclic group, $OSO_2NR^{rcx}R^{scx}$ (wherein $R^{rcx}$ and $R^{scx}$ have the same meanings as previously described, respectively), $COR^{14x}$ (wherein $R^{14x}$ has the same meaning as previously described), $NR^{18x}R^{19x}$ (wherein $R^{18x}$ and $R^{19x}$ have the same meanings as previously described, respectively), $NR^{20x}COR^{21x}$ (wherein, $R^{20x}$ and $R^{21x}$ have the same meanings as previously described, respectively) or $SO_2R^{22x}$ (wherein $R^{22x}$ has the same meaning as previously described).

(6) The aryl sulfamate derivative or the pharmaceutically acceptable salt thereof according to (3) or (4), wherein $R^{5A}$ is hydroxy.

(7) The aryl sulfamate derivative or the pharmaceutically acceptable salt thereof according to (3) or (4), wherein $R^{6A}$ is hydroxy.

(8) The aryl sulfamate derivative or the pharmaceutically acceptable salt thereof according to any one of (3) to (5), wherein $R^{6A}$ is a hydrogen atom, substituted or unsubstituted alkyl, a halogen atom, nitro, cyano, azido, isocyano, substituted or unsubstituted alkoxy, $OSO_2NR^{rcx}R^{scx}$ (wherein $R^{rcx}$ and $R^{scx}$ have the same meanings as previously described, respectively), $COR^{14x}$ (wherein $R^{14x}$ has the same meaning as previously described), $NR^{10x}COR^{21x}$ (wherein $R^{20x}$ and $R^{21x}$ have the same meanings as previously described, respectively) or $SO_2R^{22x}$ (wherein $R^{22x}$ has the same meaning as previously described).

(9) The aryl sulfamate derivative or the pharmaceutically acceptable salt thereof according to any one of (3) to (5), wherein $R^{6A}$ is a hydrogen atom or substituted or unsubstituted alkyl.

(10) The aryl sulfamate derivative or the pharmaceutically acceptable salt thereof according to any one of (3), (4) and (6), wherein $R^{6A}$ is a hydrogen atom or substituted or unsubstituted alkyl.

(11) The aryl sulfamate derivative or the pharmaceutically acceptable salt thereof according to (9), wherein $R^{5A}$ is cyano, a halogen atom, a hydrogen atom or substituted or unsubstituted alkyl.

(12) The aryl sulfamate derivative or the pharmaceutically acceptable salt thereof according to (3) or (4), wherein $R^{5A}$ and $R^{6A}$ are joined to form a bond.

(13) The aryl sulfamate derivative or the pharmaceutically acceptable salt thereof according to (8), wherein $R^{5A}$ is a hydrogen atom or substituted or unsubstituted alkyl.

(14) The aryl sulfamate derivative or the pharmaceutically acceptable salt thereof according to any one of (3), (4) and (7), wherein $R^{5A}$ is a hydrogen atom or substituted or unsubstituted alkyl.

(15) The aryl sulfamate derivative or the pharmaceutically acceptable salt thereof according to any one of (3) to (5), wherein $R^{6A}$ is cyano or a halogen atom.

(16) The aryl sulfamate derivative or the pharmaceutically acceptable salt thereof according to (6), wherein $R^{6A}$ is cyano.

(17) The aryl sulfamate derivative or the pharmaceutically acceptable salt thereof according to (15), wherein $R^{5A}$ is a hydrogen atom or substituted or unsubstituted alkyl.

(18) The aryl sulfamate derivative or the pharmaceutically acceptable salt thereof according to (3) or (4), wherein $R^{5A}$ is a hydrogen atom or alkyl.

(19) The aryl sulfamate derivative or the pharmaceutically acceptable salt thereof according to any one of (2) to (18), wherein $R^{7A}$ is $X^1NR^{23}R^{24}$ (wherein $X^1$, $R^{23}$ and $R^{24}$ have the same meanings as previously described, respectively).

(20) The aryl sulfamate derivative or the pharmaceutically acceptable salt thereof according to any one of (2) to

(18), wherein $R^{7A}$ is $CONR^{23}R^{24}$ (wherein $R^{23}$ and $R^{24}$ have the same meanings as previously described, respectively).
(21) The aryl sulfamate derivative or the pharmaceutically acceptable salt thereof according to any one of (2) to (18), wherein $R^{7A}$ is $CONR^{23a}R^{24a}$ (wherein $R^{23a}$ and $R^{24a}$,which may be the same or different, each represent a hydrogen atom or substituted or unsubstituted alkyl).
(22) The aryl sulfamate derivative or the pharmaceutically acceptable salt thereof according to (18), wherein $R^{7A}$ is $CONR^{23b}R^{24b}$ (wherein $R^{23b}$ and $R^{24b}$, which may be the same or different, each represent a hydrogen atom or alkyl having 7 to 20 carbon atoms).
(23) The aryl sulfamate derivative or the pharmaceutically acceptable salt thereof according to (18), wherein $R^{7A}$ is $CONR^{23c}R^{24c}$ (wherein $R^{23c}$ and $R^{24c}$, which may be the same or different, each represent a hydrogen atom or alkyl having 1 to 6 carbon atoms).
(24) The aryl sulfamate derivative or the pharmaceutically acceptable salt thereof according to any one of (2) to (23), wherein $R^{3A}$ and $R^{4A}$ are hydrogen atoms.
(25) The aryl sulfamate derivative or the pharmaceutically acceptable salt thereof according to any one of (2) to (24), wherein $R^{rA}$ and $R^{sA}$ are hydrogen atoms.
(26) A steroid sulfatase inhibitor comprising the aryl sulfamate derivative or the pharmaceutically acceptable salt thereof according to any one of (1) to (25).
(27) A therapeutic or prophylactic agent for a disease selected from the group consisting of breast cancer, endometrial cancer, ovarian cancer, and prostate cancer, comprising at least one of the aryl sulfamate derivatives or the pharmaceutically acceptable salts thereof according to any one of (1) to (25).
(28) An anti-tumor agent comprising at least one of the aryl sulfamate derivatives or the pharmaceutically acceptable salts thereof according to any one of (1) to (25).
(29) A therapeutic or prophylactic agent for a steroid sulfatase-related disease, comprising at least one of the aryl sulfamate derivatives or the pharmaceutically acceptable salts thereof according to any one of (1) to (25).
(30) A therapeutic or prophylactic agent for a steroid hormone dependent disease, comprising at least one of the aryl sulfamate derivatives or the pharmaceutically acceptable salts thereof according to any one of (1) to (25).
(31) A pharmaceutical composition comprising at least one of the aryl sulfamate derivatives or the pharmaceutically acceptable salts thereof according to any one of (1) to (25).
(32) A method for treating or preventing a malignant tumor, comprising a step of administering a therapeutically effective amount of the aryl sulfamate derivative or the pharmaceutically acceptable salt thereof according to any one of (1) to (25).
(33) A method for treating or preventing a disease selected from the group consisting of breast cancer, endometrial cancer, ovarian cancer, and prostate cancer, comprising a step of administering a therapeutically effective amount of the aryl sulfamate derivative or the pharmaceutically acceptable salt thereof according to any one of (1) to (25).
(34) A method for treating or preventing a steroid hormone dependent disease, comprising a step of administering a therapeutically effective amount of the aryl sulfamate derivative or the pharmaceutically acceptable salt thereof according to any one of (1) to (25).
(35) A method for treating or preventing a steroid sulfatase-related disease, comprising a step of administering a therapeutically effective amount of the aryl sulfamate derivative or the pharmaceutically acceptable salt thereof according to any one of (1) to (25).
(36) Use of the aryl sulfamate derivative or the pharmaceutically acceptable salt thereof according to any one of (1) to (25) for the manufacture of an anti-tumor agent.
(37) Use of the aryl sulfamate derivative or the pharmaceutically acceptable salt thereof according to any one of (1) to (25) for the manufacture of a therapeutic or prophylactic agent for a disease selected from the group consisting of breast cancer, endometrial cancer, ovarian cancer, and prostate cancer.
(38) Use of the aryl sulfamate derivative or the pharmaceutically acceptable salt thereof according to any one of (1) to (25) for the manufacture of a therapeutic or prophylactic agent for a steroid hormone dependent disease.
(39) Use of the aryl sulfamate derivative or the pharmaceutically acceptable salt thereof according to any one of (1) to (25) for the manufacture of a therapeutic or prophylactic agent for a steroid sulfatase-related disease.
(40) Use of the aryl sulfamate derivative or the pharmaceutically acceptable salt thereof according to any one of (1) to (25) for the manufacture of a steroid sulfatase inhibitor.

Hereinafter, the compound represented by Formula (I) will be referred to as Compound (I). Compounds with other formula numbers are referred to in the same manner.

In the definitions of the groups in Formula (I), examples of the lower alkyl moiety of the lower alkyl and the lower alkoxy include, for example, straight chain or branched alkyl having 1 to 8 carbon atoms, more specifically, methyl, ethyl, propyl, isopropyl, butyl, isobutyl, sec-butyl, tert-butyl, pentyl, isopentyl, neopentyl, hexyl, heptyl, octyl, isooctyl, and the like.

Examples of the alkyl moiety of the alkyl and the alkoxy include, for example, straight chain or branched alkyl having 1 to 20 carbon atoms, more specifically, methyl, ethyl, propyl, isopropyl, butyl, isobutyl, sec-butyl, tert-butyl, pentyl, isopentyl, neopentyl, hexyl, heptyl, octyl, isooctyl, nonyl, decyl, undecyl, dodecyl, tridecyl, tetradecyl, pentadecyl, hexadecyl, heptadecyl, octadecyl, nonadecyl, icosanyl, and the like.

Examples of the lower alkynyl include, for example, straight chain or branched alkynyl having 2 to 8 carbon atoms, more specifically, ethynyl, 1-propynyl, 1-butynyl, 1-pentynyl, 1-hexynyl, 1-heptynyl, 1-octynyl, and the like.

Examples of the lower alkenyl include, for example, straight chain or branched alkenyl having 2 to 8 carbon atoms, more specifically, vinyl, allyl, 1-propenyl, methacryl, crotyl, 1-butenyl, 2-butenyl, 3-butenyl, 1-pentenyl, 2-pentenyl, 4-pentenyl, 2-hexenyl, 5-hexenyl, 1-heptenyl, 1-octenyl, and the like.

Examples of the lower alkanoyl include, for example, straight chain or branched alkanoyl having 1 to 8 carbon atoms, more specifically, formyl, acetyl, propanoyl, butanoyl, isobutanoyl, valeryl, isovaleryl, pivaloyl, caproyl, heptanoyl, octanoyl, and the like.

Examples of the lower cycloalkyl include, for example, cycloalkyl having 3 to 8 carbon atoms, more specifically, cyclopropyl, cyclobutyl, cyclopentyl, cyclohexyl, cycloheptyl, cyclooctyl, and the like.

Examples of the aryl include, for example, aryl having 6 to 14 carbon atoms, more specifically, phenyl, naphthyl, anthranyl, and the like.

The heterocyclic group includes alicyclic heterocyclic groups and aromatic heterocyclic groups. Examples of the alicyclic heterocyclic group include 3- to 8-membered monocyclic alicyclic heterocyclic groups containing at least one atom selected from the group consisting of a nitrogen atom, an oxygen atom, and a sulfur atom, and bicyclic or tricyclic condensed alicyclic heterocyclic groups in which 3- to 8-membered rings are condensed and which contain at least one atom selected from the group consisting of a nitrogen atom, an oxygen atom, and a sulfur atom, more specifically, tetrahydropyranyl, tetrahydrofuranyl, pyrrolidinyl, piperidino, piperidinyl, perhydroazepinyl, perhydroazocinyl, morpholino, morpholinyl, thiomorpholino, thiomorpholinyl, piperazinyl, homopiperazinyl, dioxolanyl, imidazolidinyl, imidazolinyl, pyrazolidinyl, indolinyl, isoindolinyl, 2-pyrrolinyl, 2-pyrrolidonyl, 3-pyrrolidonyl, 2-piperidonyl, 3-piperidonyl, 4-piperidonyl, perhydro-2-azepinonyl, perhydro-3-azepinonyl, perhydro-4-azepinonyl, 2-thiazolidonyl, 4-thiazolidonyl, 2-oxazolidonyl, 4-oxazolidonyl, succinimido, phthalimido, glutarimido, maleimido, hydantoinyl, thiazolidinedionyl, oxazolidinedionyl, and the like. The aromatic heterocyclic group includes 3- to 8- membered monocyclic aromatic heterocyclic groups containing at least one atom selected from the group consisting of a nitrogen atom, an oxygen atom, and a sulfur atom, and bicyclic or tricyclic condensed aromatic heterocyclic groups in which 3- to 8-membered rings are condensed and which contain at least one atom selected from the group consisting of a nitrogen atom, an oxygen atom, and a sulfur atom, more specifically, furyl, thienyl, pyrrolyl, imidazolyl, pyrazolyl, triazolyl, tetrazolyl, oxazolyl, isoxazolyl, thiazolyl, isothiazolyl, 1,2,3-oxadiazolyl, 1,2,4-oxadiazolyl, 1,3,4-oxadiazolyl, 1,2,3-thiadiazolyl, 1,2,4-thiadiazolyl, 1,3,4-thiadiazolyl, furazanyl, pyridyl, pyrimidinyl, pyrazinyl, pyridazinyl, quinolyl, isoquinolyl, quinazolinyl, phthalazinyl, purinyl, indolyl, isoindolyl, 2-pyridonyl, 4-pyridonyl, uracilyl and the like.

The halogen atom means a fluorine, a chlorine, a bromine and an iodine atom.

The substituents in the substituted alkyl, the substituted alkoxy, the substituted lower alkynyl and the substituted lower alkenyl include 1 to 3 substituents which are independently selected from the group consisting of lower alkenyl, lower alkadienyl, lower alkatrienyl, lower alkynyl, tri(lower alkyl)silyloxy, (lower alkoxy)lower alkoxy, azido, cyano, isocyano, nitro, a halogen atom, substituted or unsubstituted lower cycloalkyl, substituted or unsubstituted aryl, a substituted or unsubstituted heterocyclic group, $X^{B1}NR^{B1}R^{B2}$ {wherein $X^{B1}$ represents C=O or C=S, $R^{B1}$ represents a hydrogen atom, alkyl, substituted or unsubstituted lower cycloalkyl, substituted or unsubstituted aryl or a substituted or unsubstituted heterocyclic group, and $R^{B2}$ represents a hydrogen atom, alkyl, substituted or unsubstituted lower cycloalkyl, substituted or unsubstituted aryl, a substituted or unsubstituted heterocyclic group, $OR^{B3}$ (wherein $R^{B3}$ has the same meaning as the previously described $R^{B1}$) or $NR^{B4}R^{B5}$ [wherein $R^{B4}$ has the same meaning as the previously described $R^{B1}$, and $R^{B5}$ represents a hydrogen atom, alkyl, substituted or unsubstituted lower cycloalkyl, substituted or unsubstituted aryl, a substituted or unsubstituted heterocyclic group, $COR^{B6}$ (wherein $R^{B6}$ represents a hydrogen atom, lower alkyl, lower alkenyl, lower alkadienyl, lower alkatrienyl, lower alkynyl, substituted or unsubstituted lower cycloalkyl, substituted or unsubstituted aryl or a substituted or unsubstituted heterocyclic group) or $SO_2R^{B7}$ (wherein $R^{B7}$ represents lower alkyl, lower alkenyl, substituted or unsubstituted lower cycloalkyl, substituted or unsubstituted aryl, or a substituted or unsubstituted heterocyclic group)]}, $(OCH_2CH_2)_nOCH_3$ (wherein n represents an integer of from 1 to 10), $NR^{B8}R^{B9}$ {wherein $R^{B8}$ has the same meaning as the previously described $R^{B1}$, and $R^{B9}$ represents a hydrogen atom, lower alkyl, substituted or unsubstituted lower cycloalkyl, substituted or unsubstituted aryl, a substituted or unsubstituted heterocyclic group, $X^{B2}R^{B10}$ [wherein $X^{B2}$ has the same meaning as the previously described $X^{B1}$, and $R^{B10}$ represents a hydrogen atom, lower alkyl, lower alkenyl, lower alkadienyl, lower alkatrienyl, lower alkynyl, substituted or unsubstitued lower cycloalkyl, substituted or unsubstituted aryl, a substituted or unsubstituted heterocyclic group, $NR^{B11}R^{B12}$ (wherein $R^{B11}$ and $R^{B12}$, which may be the same or different, each represent a hydrogen atom, lower alkyl, substituted or unsubstituted lower cycloalkyl, substituted or unsubstituted aryl, or a substituted or unsubstituted heterocyclic group) or $OR^{B13}$ (wherein $R^{B13}$ represents lower alkyl, substituted or unsubstituted lower

cycloalkyl, substituted or unsubstituted aryl, or a substituted or unsubstituted heterocyclic group)] or $SO_2R^{B14}$ [wherein $R^{B14}$ represents lower alkyl, lower alkenyl, substituted or unsubstituted lower cycloalkyl, substituted or unsubstituted aryl, a substituted or unsubstituted heterocyclic group, $NR^{B15}R^{B16}$ (wherein $R^{B15}$ and $R^{B16}$ have the same meanings as the previously described $R^{B11}$ and $R^{B12}$, respectively) or $OR^{B17}$ (wherein $R^{B17}$ has the same meaning as the previously described $RB^{13}$)]}, $SO_2R^{B18}$ [wherein $R^{B18}$ represents lower alkyl, lower alkenyl, substituted or unsubstituted lower cycloalkyl, substituted or unsubstituted aryl, a substituted or unsubstituted-heterocyclic group, $NR^{B19}R^{B20}$ (wherein $R^{B19}$ and $R^{B20}$ have the same meanings as the previously described $R^{B11}$ and $R^{B12}$, respectively) or $OR^{B21}$ (wherein $R^{B21}$ has the same meaning as the previously described $R^{B1}$)], $X^{B3}R^{B22}$ [wherein $X^{B3}$ has the same meaning as the previously described $X^{B1}$, and $R^{B22}$ represents a hydrogen atom, lower alkyl, lower alkenyl, lower alkadienyl, lower alkatrienyl, lower alkynyl, substituted or unsubstituted lower cycloalkyl, substituted or unsubstituted aryl, a substituted or unsubstituted heterocyclic group or $Y^{B1}R^{B23}$ (wherein $Y^{B1}$ represents an oxygen atom or a sulfur atom, and $R^{B23}$ has the same meaning as the previously described $R^{B1}$)], $Y^{B2}R^{B24}$ [wherein $Y^{B2}$ has the same meaning as the previously described $Y^{B1}$, and $R^{B24}$ represents a hydrogen atom, lower alkyl, substituted or unsubstituted lower cycloalkyl, substituted or unsubstituted aryl, a substituted or unsubstituted heterocyclic group, $X^{B4}R^{B25}$ (wherein $X^{B4}$ and $R^{B25}$ have the same meanings as the previously described $X^{B1}$ and $R^{B10}$, respectively) or $SO_2R^{B26}$ (wherein $R^{B26}$ has the same meaning as the previously described $R^{B14}$]), $S(O)R^{B27}$ (wherein $R^{B27}$ has the same meaning as the previously described $R^{B7}$), and the like.

The substitutents for the substituted lower cycloalkyl, the substituted aryl and the substituted heterocyclic group include 1 to 3 substituents which are independently selected from the group consisting of lower alkyl, lower alkenyl, lower alkadienyl, lower alkatrienyl, lower alkynyl, tri(lower alkyl)silyloxy, (lower alkoxy)lower alkoxy, azido, cyano, isocyano, nitro, a halogen atom, lower cycloalkyl, aryl, a heterocyclic group, $X^{C1}NR^{C1}R^{C2}$ {wherein $X^{C1}$ represents C=O or C=S, $R^{C1}$ represents a hydrogen atom, alkyl, lower cycloalkyl, aryl or a heterocyclic group, and $R^{C2}$ represents a hydrogen atom, alkyl, lower cycloalkyl, aryl, a heterocyclic group, $OR^{C3}$ (wherein $R^{C3}$ has the same meaning as the previously described $R^{C1}$) or $NR^{C4}CR^{C5}$ [wherein $R^{C4}$ has the same meaning as the previously described $R^{C1}$, and $R^{C5}$ represents a hydrogen atom, alkyl, lower cycloalkyl, aryl, a heterocyclic. group, $COR^{C6}$ (wherein $R^{C6}$ represents a hydrogen atom, lower alkyl, lower alkenyl, lower alkadienyl, lower alkatrienyl, lower alkynyl, lower cycloalkyl, aryl or a heterocyclic group) or $SO_2R^{C7}$ (wherein $R^{C7}$ represents lower alkyl, lower alkenyl, lower cycloalkyl, aryl or a heterocyclic group)]}, $(OCH_2CH_2)_{na}OCH_3$ (wherein na represents an integer of from 1 to 10), $NR^{C8}R^{C9}$ {wherein $R^{C8}$ has the same meaning as the previously described $R^{C1}$, and $R^{C9}$ represents a hydrogen atom, lower alkyl, lower cycloalkyl, aryl, a heterocyclic group, $X^{C2}R^{C10}$ [wherein $X^{C2}$ has the same meaning as the previously described $X^{C1}$, and $R^{C10}$ represents a hydrogen atom, lower alkyl, lower alkenyl, lower alkadienyl, lower alkatrienyl, lower alkynyl, lower cycloalkyl, aryl, a heterocyclic group, $NR^{C11}R^{C12}$ (wherein $R^{C11}$ and $R^{C12}$, which may be the same or different, each represent a hydrogen atom, lower alkyl, lower cycloalkyl, aryl or a heterocyclic group) or $OR^{C13}$ (wherein $R^{C13}$ represents lower alkyl, lower cycloalkyl, aryl or a heterocyclic group)] or $SO_2R^{C14}$ [wherein $R^{C14}$ represents lower alkyl, lower alkenyl, lower cycloalkyl, aryl, a heterocyclic group, $NR^{C15}R^{C16}$ (wherein $R^{C15}$ and $R^{C16}$ have the same meanings as the previously described $R^{C11}$ and $R^{C12}$, respectively) or $OR^{C17}$ (wherein $R^{C17}$ has the same meaning as the previously described $R^{C13}$)]}, $SO_2R^{C18}$ [wherein $R^{C18}$ represents lower alkyl, lower alkenyl, lower cycloalkyl, aryl, a heterocyclic group, $NR^{C19}R^{C20}$ (wherein $R^{C19}$ and $R^{C20}$ have the same meanings as the previously described $R^{C11}$ and $R^{C12}$, respectively) or $OR^{C21}$ (wherein $R^{C21}$ has the same meaning as the previously described $R^{C1}$)], $X^{C3}R^{C22}$ [wherein $X^{C3}$ has the same meaning as the previously described $X^{C1}$, and $R^{C22}$ represents a hydrogen atom, lower alkyl, lower alkenyl, lower alkadienyl, lower alkatrienyl, lower alkynyl, lower cycloalkyl, aryl, a heterocyclic group or $Y^{C1}R^{C23}$ (wherein $Y^{C1}$ represents an oxygen atom or a sulfur atom, and $R^{C23}$ has the same meaning as the previously described $R^{C1}$)], $Y^{C2}R^{C24}$ [wherein $Y^{C2}$ has the same meaning as the previously described $Y^{C1}$, and $R^{C24}$ represents a hydrogen atom, lower alkyl, lower cycloalkyl, aryl, a heterocyclic group, $X^{C4}R^{C25}$ (wherein $X^{C4}$ and $R^{C25}$ have the same meanings as the previously described $X^{C1}$ and $R^{C10}$, respectively) or $SO_2R^{C26}$ (wherein $R^{C26}$ has the same meaning as the previously described $R^{C14}$)], $S(O)R^{C27}$ (wherein $R^{C27}$ has the same meaning as the previously described $R^{C7}$), and the like.

In the definition of the substituents for the substituted alkyl, substituted alkoxy, substituted lower alkynyl, substituted lower alkenyl, substituted lower cycloalkyl, substituted aryl and a substituted heterocyclic group, the lower alkyl, the alkyl, the lower alkynyl, the lower alkenyl, the lower cycloalkyl, the aryl, the heterocyclic group and the halogen atom have the same meanings as previously described, respsctively. The lower alkyl moiety of the (lower alkoxy)lower alkoxy and tri(lower alkyl)silyloxy have the same meanings as the previously described lower alkyl. Examples of the lower alkadienyl include, for example, straight chain or branched alkadienyl having 4 to 8 carbon atoms, more specifically, 1,3-butadienyl, 1,3-pentadienyl, 1,3-hexadienyl, 2,4-hexadienyl, 1,3-heptadienyl, 1,3-octadienyl, and the like. Examples of the lower alkatrienyl include, for example, straight chain or branched alkatrienyl having 6 to 8 carbon atoms, more specifically, 1,3,5-hexatrienyl, 1,3,5-heptatrienyl, 1,3,5-octatrienyl, and the like. The substituents in the substituted lower cycloalkyl, the substituted aryl and the substituted heterocyclic group have the same meanings as the previously described substituents in the substituted lower cycloalkyl, the substituted aryl and the substituted heterocyclic group.

As compound (I), a compound having an anti-estrogenic activity, such as an inhibitory activity against the action of estrogen or an inhibitory activity against estrogen biosynthesis, a compound that generates a phenol derivative (a desulfamoylated form) by hydrolysis of sulfamate in a living body, and the like are preferable.

Examples of pharmaceutically acceptable salts of Compound (I) include acid addition salts, metal salts, ammonium salts, organic amine addition salts, amino acid addition salts, and the like. Examples of acid addition salts include inorganic acid salts, such as hydrochloride, hydrobromide, sulfate, phosphate, and the like, and organic acid salts such as formate, acetate, oxalate, benzoate, methanesulfonate, p-toluenesulfonate, maleate, fumarate, tartrate, citrate, succinate, lactate, and the like. Examples of metal salts include alkali metal salts such as lithium salt, sodium salt, potassium salt, and the like, alkali earth metal salts such as magnesium salt, calcium salt, and the like, aluminum salt, and zinc salt, and the like. Examples of ammonium salts include salts of ammonium, tetramethylammonium, and the like. Examples of organic amine addition salts include addition salts of morpholine, piperidine, and the like. Examples of amino acid addition salts include addition salts of glycine, phenylalanine, aspartic acid, lysine and the like.

The aryl sulfamate derivative or pharmaceutically acceptable salt thereof of the present invention is produced with a starting compound of a benzene derivative such as various phenols, benzaldehydes, acetophenones, and the like.

Next, the method for the production of Compound (I) is described.

The process for the production of Compound (I) comprises mainly each of the following reaction steps. Depending on the target substance, the reaction steps can be combined to produce this target compound.

In the production method described below, when the defined groups are changed under the conditions of the method to be conducted or are unsuitable for the conducting the method, the methods ordinally used in synthetic organic chemistry for the introduction and removal of protective groups (for example, Protective groups in organic synthesis, written by T.W. Green, John & Wiley and Sons Inc., 1981) can be used to obtain the target compound. In addition, the order of reaction steps can be altered if necessary.

Production method 1

According to the reaction steps described below, Compound (I) is obtained from Compound (B), which is obtained from a benzene derivative by known methods [Synthesis, p. 56 (1978); Tetrahedron, vol. 28, p. 663 (1972); Synthesis, vol. 3, p. 217 (1989), and the like].

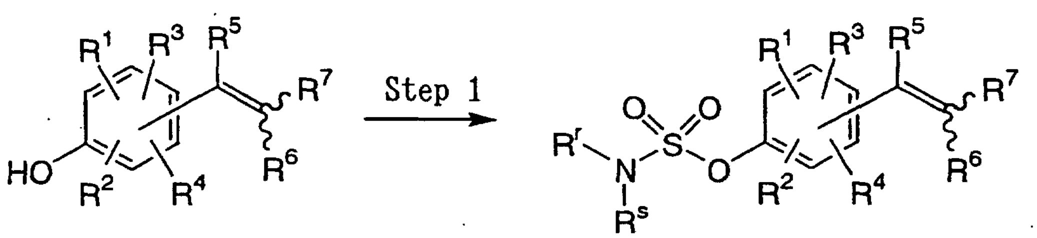


(B) (I)

(wherein $R^r$, $R^s$, $R^1$, $R^2$, $R^3$, $R^4$, $R^5$, $R^6$ and $R^7$ are the same as previously described, respectively)

Step 1

Compound (I) is obtained by reacting Compound (B) with Compound (II), which is represented by $R^rR^sNSO_2Cl$ (wherein $R^r$ and $R^s$ are the same as previously described, respectively) in the presence of a base or not, and in a solvent.

For the solvent, the following can be used individually or as mixtures: tetrahydrofuran (THF), acetonitrile, N, N-dimethylformamide (DMF), N,N-di-methylacetamide (DMAC), 1,3-dimethyl-2-imidazolidinone, toluene, dichloromethane, chloroform, 1,2-dichloroethane, 1-methyl-2-piperidone, and the like.

For the base, sodium hydride, potassium carbonate, sodium carbonate, sodium hydrogencarbonate, cesium carbonate, lithium hydroxide, potassium tert-butoxide, 2,6-di-tert-butyl-4-methylpyridine, pyridine, 2,6-di-tert-butylpyridine, 2,6-lutidine, triethylamine, diisopropylethylamine, and 4-dimethylaminopyridine (DMAP), and the like are used. Preferably, sodium hydride or 2,6-di-tert-butyl-4-methylpyridine is used. If the base is liquid, it can also be used as a solvent. More preferably, DMAC is used as the solvent without a base.

Each of the base and Compound (II) are used at 0.1 equivalent or above based on Compound (B), respectively. Preferably, 1 to 20 equivalents are used. The reaction is conducted at a temperature between -78 and 120 °C.

Preferably, the reaction is conducted at a temperature between -20 and 60 °C for 5 minutes to 72 hours.

Production method 2

Compound (Ia) is obtained from Compound (C) by the following reaction process.

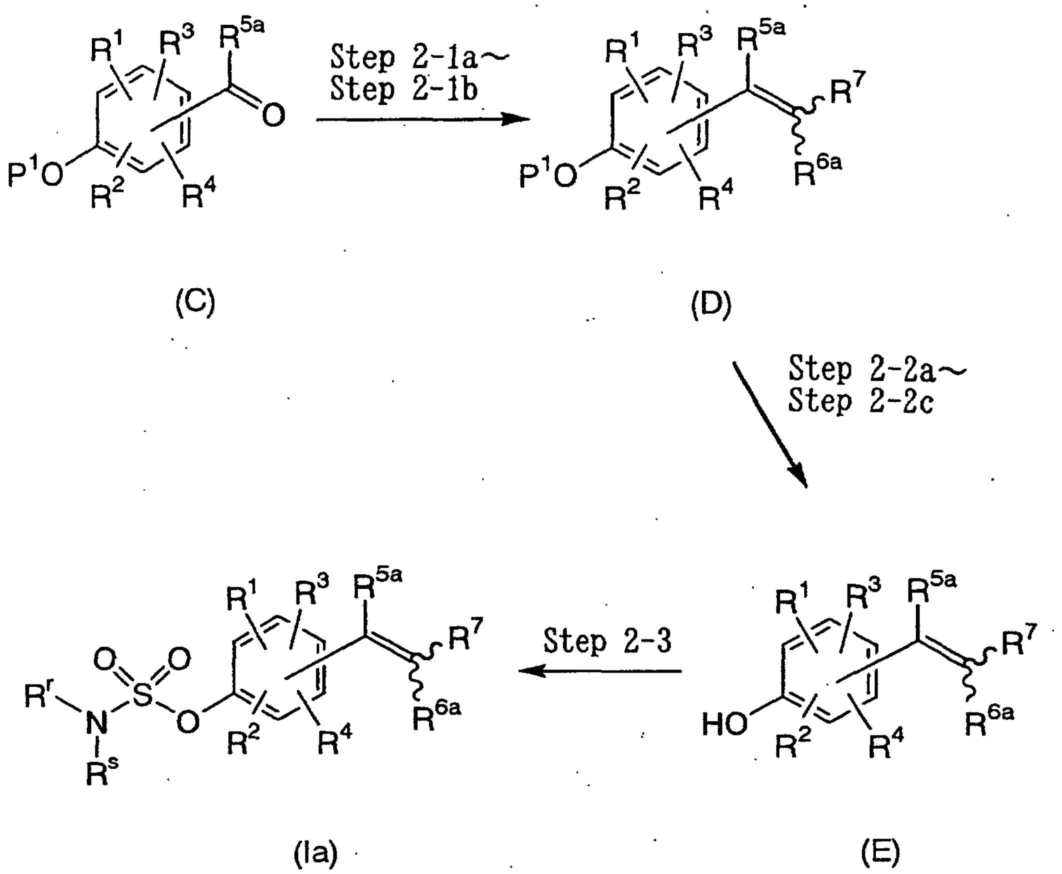


[wherein $R^r$, $R^s$, $R^1$, $R^2$, $R^3$, $R^4$ and $R^7$ are the same as previously described, recpectively, $R^{5a}$ represents a hydrogen atom or substituted or unsubstituted alkyl within the definition of $R^5$, $R^{6a}$ represents a hydrogen atom, substituted or unsubstituted alkyl, substituted or unsubstituted lower cycloalkyl, a halogen atom, nitro, cyano, substituted or unsubstituted lower alkynyl, substituted or unsubstituted lower alkenyl, substituted or unsubstituted aryl, a substituted or unsubstituted heterocyclic group, $COR^{14x}$ (wherein $R^{14x}$ is the same as previously described) or $SO_2R^{22x}$ (wherein $R^{22x}$ is the same as previously described) within the definition of $R^6$, and $P^1$ represents methyl, methoxymethyl or benzyl]

Compound (C) is obtained from a benzene derivative by known methods [Protective groups in organic synthesis, written by T. W. Greene, John Wiley & Sons Inc. (1981); Jikken kagaku kouza 19 4th ed. Yuuki Gousei I - Jikken kagaku kouza 26 Yuuki gousei VIII (Maruzen Co., Ltd, Published 1992), and the like].

Step 2-1a

Compound (Da) is Compound (D) wherein $R^{6a}$ is $R^{6aa}$ [wherein $R^{6aa}$ represents nitro, cyano, $COR^{14x}$ (wherein $R^{14x}$ is the same as previously described) or $SO_2R^{22x}$ (wherein $R^{22x}$ is the same as previously described) within the definition of $R^6$]. Compound (Da) is obtained by reacting Compound (C) with Compound (III) represented by $R^{6aa}CH_2R^7$ (wherein $R^{6aa}$ and $R^7$ are the same as previously described, respectively), in the presence of acetic acid and a base, and in a solvent.

For the solvent, toluene, benzene, THF, 1,4-dioxane and the like are used individually or as a mixture.

For the base, ammonium acetate, piperidine, piperdine acetate, pyridine acetate, triethylamine acetate, acetamide, sodium acetate, and the like are used.

Each of the acetic acid and base is used at 0.1 equivalent or above based on Compound (C), respectively.

Preferably, 0.1 to 50 equivalents are used. Acetic acid can also be used as the solvent.

Compound (III) is used at 0.1 equivalent or above based on Compound (C). Preferably 1 to 10 equivalent are used. The reaction is carried out at a temperatures between -20 and 160 °C, preferably between 20 and 140 °C, for 5 minutes to 72 hours. In addition, the reaction can also be carried out while removing water from the reaction system by using a Dean-Stark device.

Step 2-1b

Compound (Db) is Compound (D) wherein $R^{6a}$ is $R^{6ab}$ (wherein $R^{6ab}$ represents a hydrogen atom, substituted or unsubstituted alkyl, substituted or unsubstituted lower cycloalkyl, a halogen atom, substituted or unsubstituted lower alkynyl, substituted or unsubstituted lower alkenyl, substituted or unsubstituted aryl or a substituted or unsubstituted heterocyclic group within the definition of $R^6$). Compound (Db) is obtained by reacting Compound (C) with Compound (IV) represented by $(C_6H_5)_3PCHR^{6ab}R^7$ (wherein $R^{6ab}$ and $R^7$ are the same as previously described, respectively), or Compound (V) represented by $(R^yO)_2POCHR^{6ab}R^7$ (wherein $R^{6ab}$ and $R^7$ are the same as previously described, respectively, and $R^y$ represents methyl, ethyl, propyl, tert-butyl or 2,2,2-trifluoroethyl), in the presence of a base, and in a solvent.

For the solvent, toluene, benzene, THF, diethylether, dimethoxyethane, DMF, dimethylsulfoxide (DMSO), chloroform, dichloromethane, 1,2-dichloroethane, ethanol, methanol, n-hexane and the like are used individually or as a mixture.

For the base, sodium hydride, potassium carbonate, sodium carbonate, n-butyl lithium, potassium bis(trimethylsilyl)amide, tert-butoxy potassium, sodium methoxide, sodium ethoxide, sodium hydroxide, potassium hydroxide, triethylamine, pyridine, and the like are used. In addition, when potassium reagent is used as a base, the reaction can be carried out with the addition of 18-crown-6, preferably an acetonitrile adduct of 18-crown-6, at 0.1 to 10 equivalent based on Compound (C).

The base is used at 0.1 equivalent or above based on Compound (C), preferably at 0.5 to 20 equivalent.

Compound (IV) or Compound (V) is used at 0.1 equivalent or above based on Compound (C), preferably at 1 to 10 equivalent, respectively. The reaction is carried out at a temperature between -78 and 160 °C, preferably between -20 and 100 °C for 5 minutes to 72 hours.

Step 2-2a

Compound (E) is obtained by treating Compound (Dc), which is Compound (D) wherein $P^1$ is methyl, with a deprotecting agent in a solvent.

For the solvent, dichloromethane, chloroform, 1,2-dichloroethane, DMF, and the like can be used individually or as a mixture. In addition, if the deprotecting agent is liquid, the solvent can be omitted.

For the deprotecting agent, boron tribromide, iodotrimethylsilane, sodium ethanethiolate, hydrobromic acid/acetic acid, and the like are used.

The deprotecting agent is used at 0.1 equivalent or above based on Compound (Dc), preferably at 1 to 20 equivalent. If the deprotecting agent is liquid, it can also be used as the solvent. The reaction is carried out at a temperature between -78 and 180 °C, preferably between -20 and 120 °C for 5 minutes to 48 hours.

Step 2-2b

Compound (E) is also obtained by treating Compound (Dd), which is Compound (D) wherein $P^1$ is methoxymethyl, with an acid in a solvent.

For the solvent, dichloromethane, chloroform, 1,2-dichloroethane, THF, 1,4-dioxane, DMSO, DMF, toluene, methanol, ethanol, isopropyl alcohol, water, and the like can be used individually or as a mixture. In addition, if the acid is liquid, the solvent can be omitted.

For the acid, hydrochloric acid, sulfuric acid, acetic acid, trifluoroacetic acid, methanesulfonic acid, hydrobromic add, hydrobromic acid/acetic acid, and the like are used.

The acid is used at 0.1 equivalent or above based on Compound (Dd), preferably at 1 to 100 equivalent. In addition, if the acid is liquid, it can also be used as the solvent. The reaction is carried out at a temperature between -78 and 180 °C, preferably between -20 and 120 °C for 5 minutes to 48 hours.

Step 2-2c

Compound (E) is also obtained by treating Compound (De), which is Compound (D) wherein $P^1$ is benzyl, with dimethyl sulfide and boron trifluoride-diethyl ether complex in a solvent.

For the solvent, dichloromethane, chloroform, 1,2-dichloroethane, toluene, and the like are used individually or as a mixture.

The dimethyl sulfide and boron trifluoride-diethyl ether complex are used at 0.1 equivalent or above based on Compound (De), preferably at 1 to 100 equivalent. The reaction is carried out at a temperature between -78 and 120 °C, preferably between -20 and 60 °C for 5 minutes to 48 hours.

Step 2-3

According to the method in Step 1 of Production method 1, Compound (Ia) is obtained by reacting Compound (E) with Compound (II) in the presence of base or not, and in a solvent.
For the solvent and base, the same as those described in Step 1 are used.

Production method 3

Compound (Ib) is obtained from Compound (F), Compound (H) or Compound (G) by the following reaction steps.

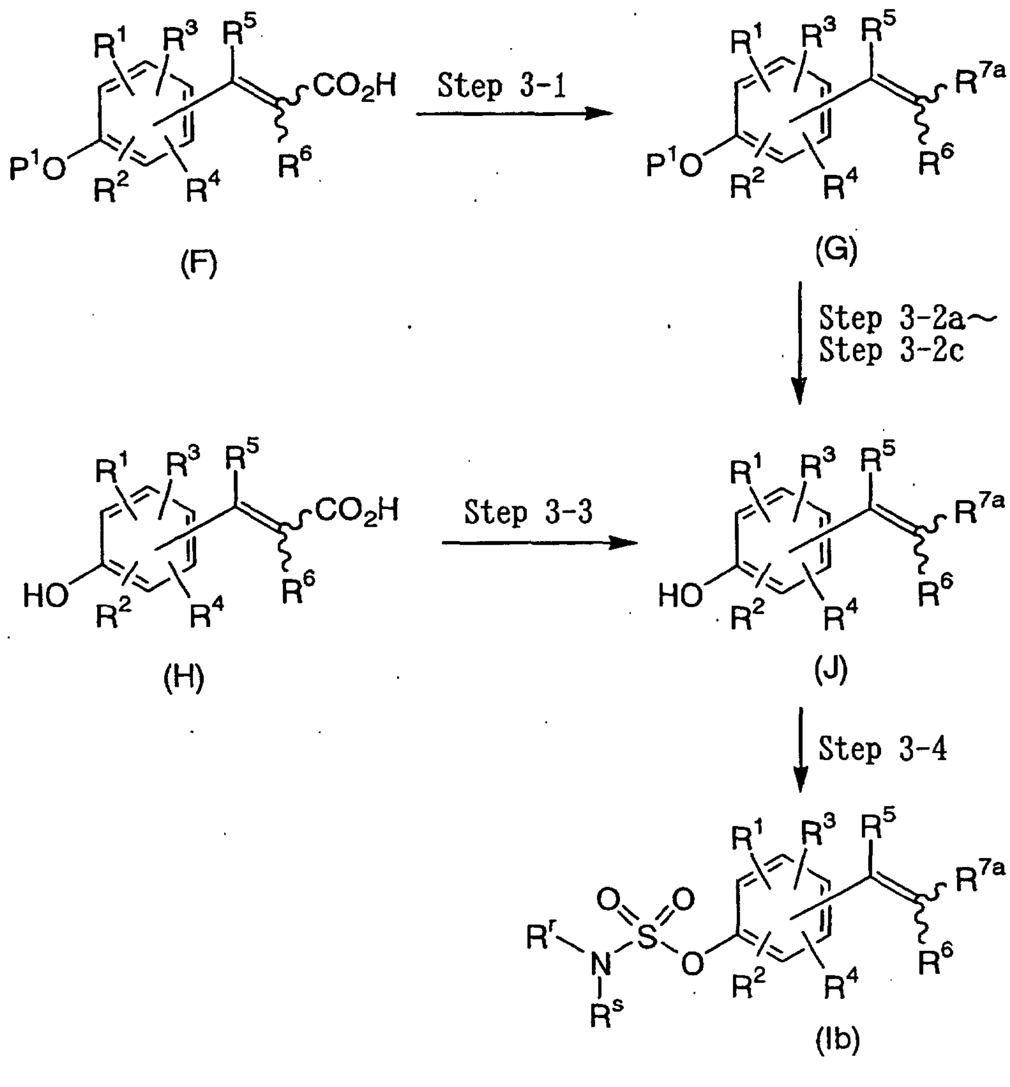

(F) (G) (H) (J) (Ib)

[wherein $R^r$, $R^s$, $R^1$, $R^2$, $R^3$, $R^4$, $R^5$, $R^6$ and $P^1$ are the same as previously described, respectively, and $R^{7a}$ represents $CONR^{23}R^{24}$ (wherein $R^{23}$ and $R^{24}$ are the same as previously described, respectively) or $CO_2R^{27}$ (wherein $R^{27}$ is the same as previously described)]
Compounds (F), Compound (H) or Compound (G) are obtained from a benzene derivative or from Compound (Ba) or Compound (Df), which are Compound (B) or Compound (D) wherein $R^7$ is $CO_2R^{27a}$ (wherein $R^{27a}$ represents alkyl within the definition of $R^{27}$) according to the known methods [Protective groups in organic synthesis, written by T. W. Greene, John Wiley & Sons Inc. (1981); Journal of the American Chemical Society, vol. 80, p.4949 (1958); The

Journal of Organic Chemistry, vol. 60, p. 4635 (1995); Synthesis, p. 56 (1978); Synthesis, p. 790 (1986); Journal of the Chemical Society Perkin Transactions I, p. 1379 (1974); Tetrahedron Letters, vol. 41 p. 3199 (2000); The Journal of Organic Chemistry, vol. 64, p. 5984 (1999); Tetrahedron, vol. 44, p. 5467 (1988); Chemistry Letters, p. 1427 (1979), and the like].

Step 3-1

Other than the method described above, Compound (G) is also obtained by reacting, in the presence of a condensing agent, and in a solvent, Compound (F) with Compound (VI) represented by $HNR^{23}R^{24}$ (wherein $R^{23}$ and $R^{24}$ are the same as previously described, respectively) or an acid addition salt thereof, or Compound (VII) represented by $HOR^{27}$ (wherein $R^{27}$ is the same as previously described).

As the solvent, THF, toluene, dichloromethane, DMF, and the like are used individually or as a mixture.

As the condensing agent,
1-(3-dimethylaminopropyl)-3-ethylcarbodiimide,
1-(3-dimethylaminopropyl)-3-ethylcarbodiimide hydrochloride (EDCI),
N,N-dicyclohexylcarbodiimide, 1,1'-carbonyldiimidazole, and the like are used. Furthermore, additives such as N-hydroxy succinimide, DMAP, 1-hydroxy benzotriazole hydrate (HOBt), and the like can be added at 0.1 to 10 equivalent based on Compound (F) in order to accelerate the reaction.

When an acid addition salt of Compound (VI) is used, the reaction can be carried out in the presence of a base at 0.1 equivalent or above, preferably 1 to 20 equivalent based on Compound (F). Examples of the base include amines such as pyridine, triethylamine, diisopropylethylamine, N,N-diethylaniline, and the like. Preferably, triethylamine is used. Compound (VI) or an acid addition salt thereof, or Compound (VII) and the condensing agent are used at 0.1 equivalents or above, preferably at 1 to 10 equivalent based on Compound (F) , respectively The reaction is carried out at a temperature between -20 and 80 °C, preferably between 0 and 60 °C, 5 minutes to 48 hours.

Step 3-2a

According to the method in Step 2-2a of Production method 2, Compound (J) is obtained by treating Compound (Ga), which is Compound (G) wherein $P^1$ is methyl, with a deprotecting agent in a solvent.

The same solvent and deprotecting agent as the examples given in Step 2-2a are used.

Step 3-2b

According to the method in Step 2-2b of Production method 2, Compound (J) is also obtained by treating Compound (Gb), which is Compound (G) wherein $P^1$ is methoxymethyl, with an acid in a solvent.

The same solvent and acid as the examples given in Step 2-2b are used.

Step 3-2c

According to the method in Step 2-2c of Production method 2, Compound (J) is obtained by treating Compound (Gc), which is Compound (G) wherein $P^1$ is benzyl, with dimethyl sulfide and boron trifluoride-diethyl ether complex in a solvent.

The same solvent as the examples given in Step 2-2c is used.

Step 3-3

According to the method in Step 3-1, Compound (J) is obtained by reacting Compound (H) with Compound (VI) or an acid addition salt thereof, or Compound (VII) in the presence of a condensing agent, and in a solvent.

The same solvent and condensing agent as the examples given in Step 3-1 are used.

Step 3-4

According to the method in Step 1 of Production method 1, Compound (Ib) is obtained by reacting Compound (J) with Compound (II) in the presence of a base or not, and in a solvent.

The same solvent and base as the examples given in Step 1 are used.

Production method 4

Compound (Ic) is obtained from Compound (K) by the following reaction steps.

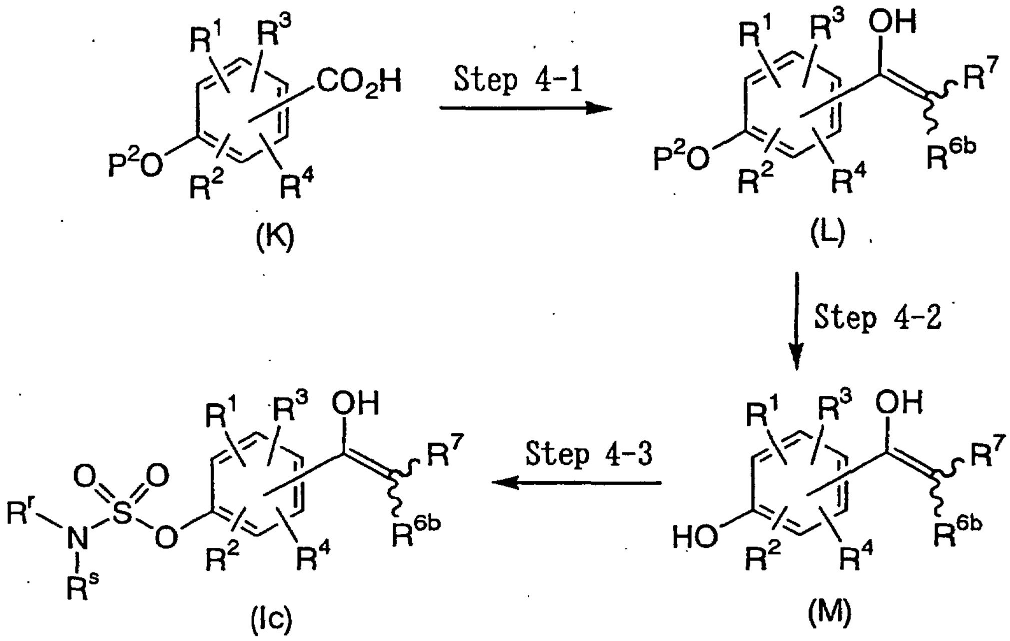


(wherein $R^r$, $R^s$, $R^1$, $R^2$, $R^3$, $R^4$ and $R^7$ are the same as previously described, respectively, $R^{6b}$ has the same meaning as the previously described $R^{6aa}$, and $P^2$ represents methoxymethyl)

Step 4-1

Compound (L) is obtained by reacting Compound (K) with Compound (III) in the presence of a base and diethylcyanophosphonate (DEPC), and in a solvent.
For the solvent, DMF, THF, toluene, dichloromethane and the like are used individually or as a mixture.
For the base, triethylamine, pyridine, diisopropylethylamine, and the like are used.
Compound (III), DEPC and a base are used at 0.1 equivalent or above based on Compound (K), preferably at 1 to 10 equivalent, respectively. The reaction is carried out at a temperature between -78 and 100 °C, preferably between -20 and 60 °C for 5 minutes to 48 hours.

Step 4-2

According to the method in Step 2-2b of Production method 2, Compound (M) is obtained by treating Compound (L) with an acid in a solvent.
The same solvent and acid as the examples given in Step 2-2b are used.

Step 4-3

According to the method in Step 1 of Production method 1, Compound (Ic) is obtained by reacting Compound (NO with Compound (II) in the presence of a base or not, and in a solvent.
The same solvent and base as the examples given in Step 1 are used.

Production method 5

Compound (Id) and Compound (Ie) are obtained from Compound (N) by the following reaction steps.

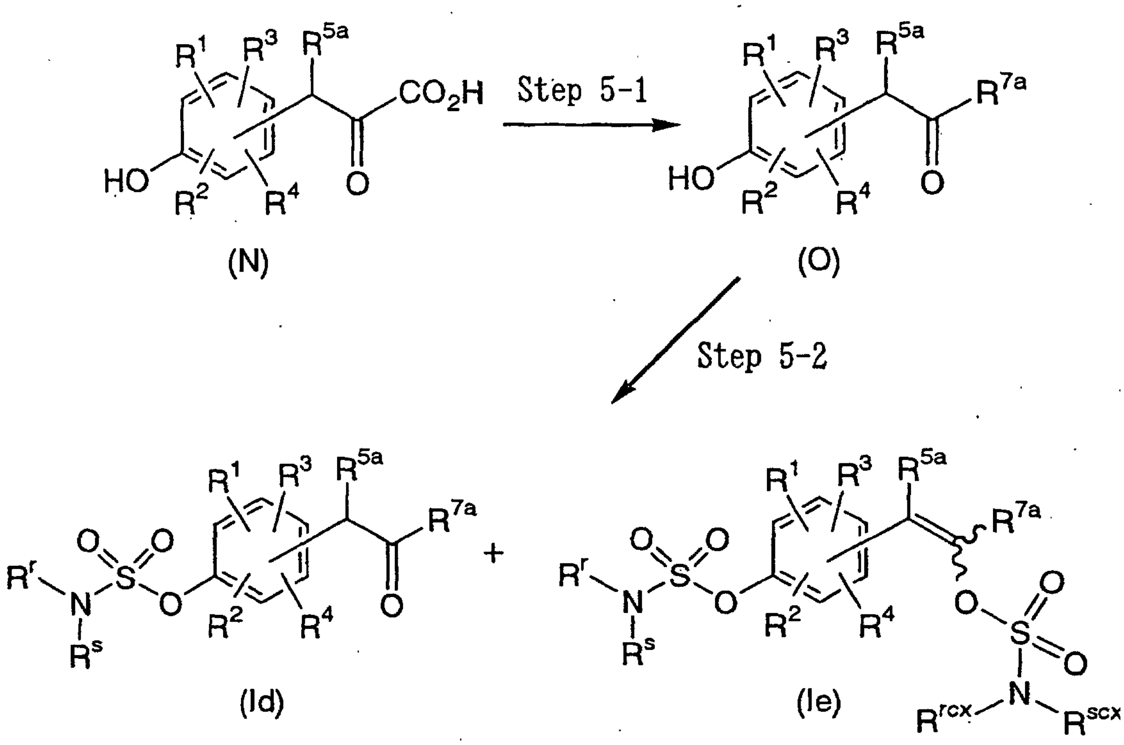


(N) (O) (Id) (Ie)

(wherein $R^r$, $R^s$, $R^{rcx}$, $R^{scx}$, $R^1$, $R^2$, $R^3$, $R^4$, $R^{5a}$ and $R^{7a}$ are the same as previously described, respectively)

Step 5-1

According to the method in Step 3-1 of Production method 3, Compound (O) is obtained by reacting Compound (N) with Compound (VI) or an acid addition salt thereof, or Compound (VII) in the presence of a condensing agent, and in a solvent.

The same solvent and condensing agent as the examples given in Step 3-1 are used.

Step 5-2

According to the method in Step 1 of Production method 1, Compound (Id) and Compound (Ie) are obtained by reacting Compound (O) with Compound (II) in the presence of a base or not, and in a solvent.

The same solvent and base as the examples given in Step 1 are used.

Production method 6

Compound (If) and Compound (Ig) are obtained from Compound (P) by the following reaction steps.

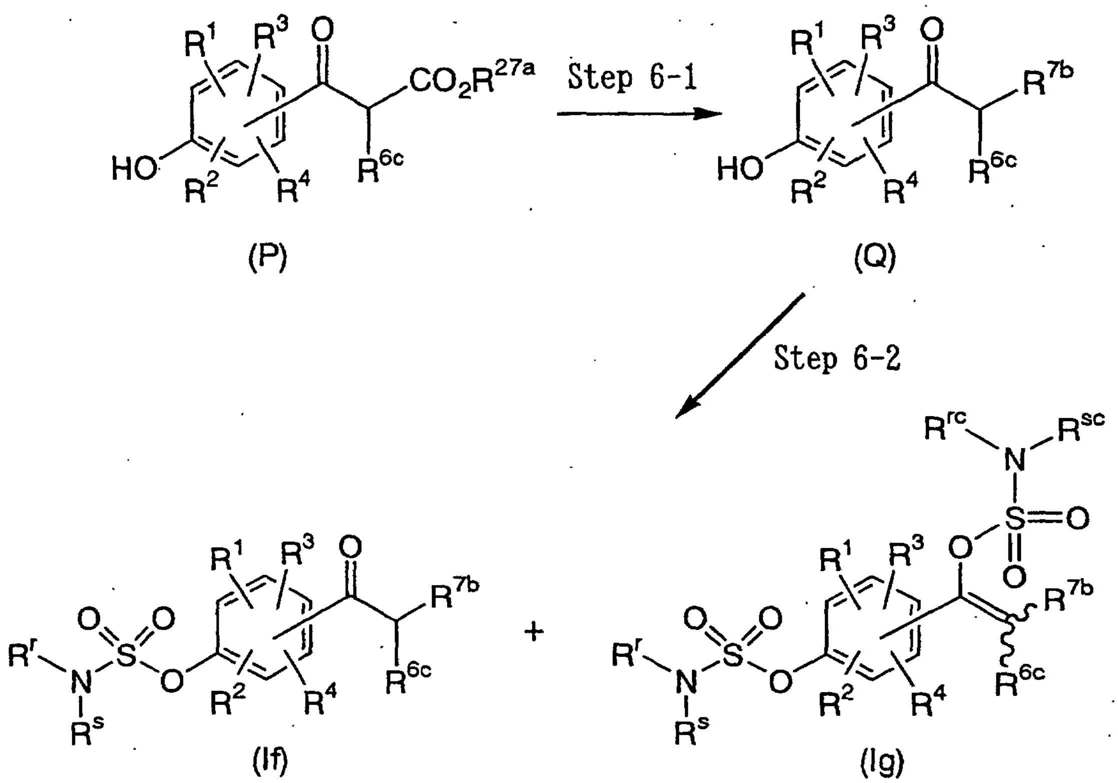


(P) (Q)

(If) + (Ig)

[wherein $R^r$, $R^s$, $R^{rc}$, $R^{sc}$, $R^1$, $R^2$, $R^3$ and $R^4$ are the same as previously described, respectively, $R^{6c}$ represents a hydrogen atom or substituted or unsubstituted alkyl within the definition of $R^6$, $R^{7b}$ represents $CONR^{23}R^{24}$ (wherein $R^{23}$ and $R^{24}$ are the same as previously described, respectively) within the definition of $R^7$, and $R^{27a}$ represents lower alkyl]

In the definition of $R^{27a}$, lower alkyl is the same as the previously described lower alkyl.

Step 6-1

Compound (Q) is obtained by reacting Compound (P) with Compound (VI) or an acid addition salt thereof in the presence of a base, and in a solvent.

For the solvent, THF, 1,4-dioxane, 1,2-dichloroethane, toluene, dichloromethane, DMF, DMAC, and the like are used individually or as a mixture.

For the base, DMAP, diisopropylethylamine, triethylamine, pyridine, and the like are used,preferably DMAP is used.

Compound (VI) or an acid addition salt thereof and base are used at 0.1 equivalent or above based on Compound (P), preferably at 0.2 to 20 equivalent, respectively. The reaction is carried out at a temperature between -20 and 180 °C, preferably between 20 and 140 °C for 5 minutes to 48 hours.

Step 6-2

According to the method in Step 1 of Production method 1, Compound (If) and Compound (Ig) are obtained by reacting Compound (Q) with Compound (II) in the presence of a base or not, and in a solvent.

The same solvent and base are used as the examples given in Step 1.

Production method 7

Compound (Ih) is obtained from Compound (R) by the following reaction steps.

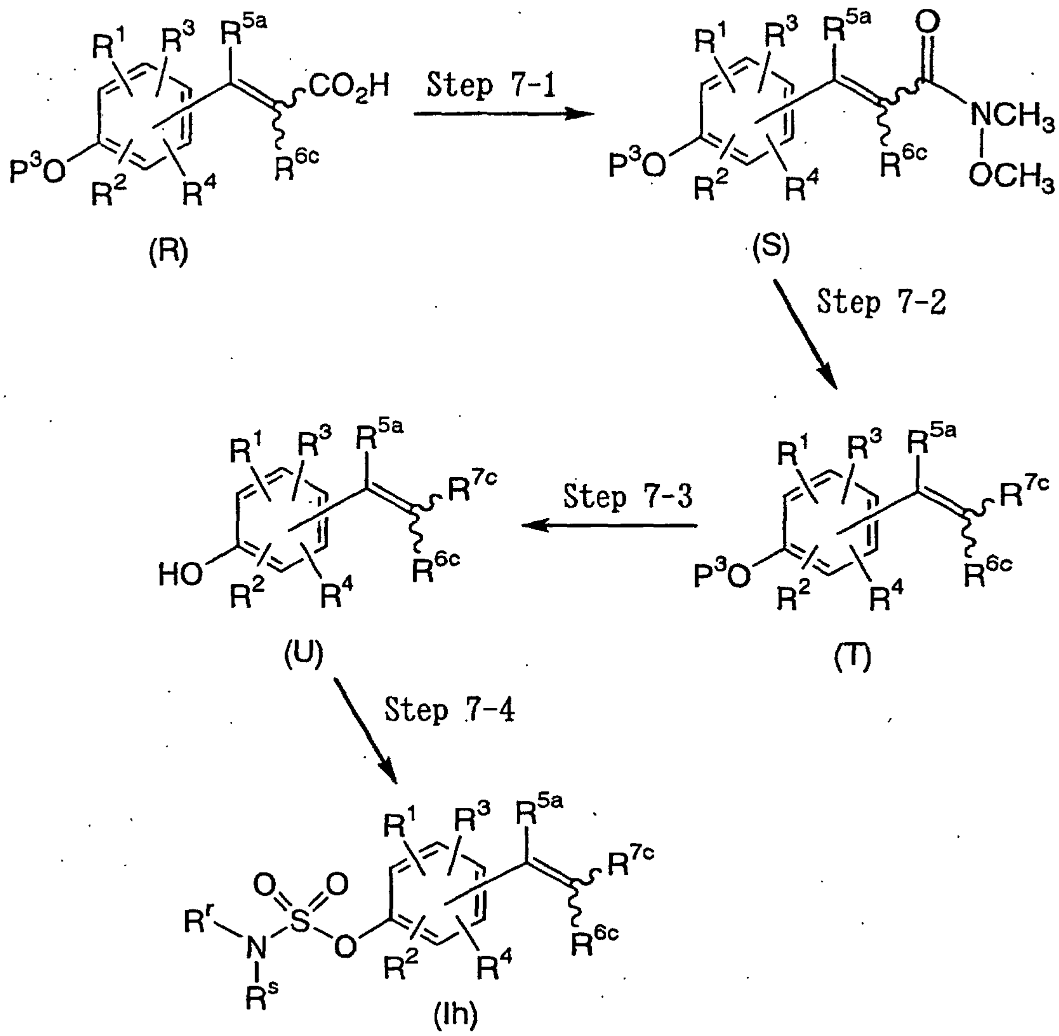


(R) (S) (T) (U) (Ih)

[wherein $R^r$, $R^s$, $R^1$, $R^2$, $R^3$, $R^4$, $R^{5a}$ and $R^{6c}$ are the same as previously described, respectively, $R^{7c}$ represents $COR^{26a}$ (wherein $R^{26a}$ represents substituted or unsubstituted alkyl, substituted or unsubstituted lower cycloalkyl, substituted or unsubstituted aryl or a substituted or unsubstituted heterocyclic group within the definition of $R^{26}$), and $P^3$ represents methyl]

Step 7-1

According to the method in Step 3-1 of Production method 3, Compound (S) is obtained by reacting Compound (R) with N,O-dimethylhydroxylamine hydrochloride in the presence of a condensing agent and a base, and in a solvent.
The same solvent, condensing agent and base as the examples given in Step 3-1 are used.

Step 7-2

Compound (T) is obtained by reacting Compound (S) with Compound (VIII) represented by $R^{26a}MgX^a$ (wherein $R^{26a}$ is the same as previously described, and $X^a$ represents a chlorine atom, a bromine atom or an iodine atom) in a solvent.
For the solvent, THF, toluene, diethylether, n-hexane, and the like are used individually or as a mixture.
Compound (VIII) is used at 0.1 equivalent or above, preferably at 1 to 10 equivalent based on Compound (S). The reaction is carried out at a temperature between -78 and 120 °C, preferably -78 to 60 °C for 5 minutes to 48 hours.

Step 7-3

According to the method in Step 2-2a of Production method 2, Compound (U) is obtained by treating Compound (T) with a deprotecting agent in a solvent.
The same solvent and deprotecting agent as the examples given in Step 2-2a are used.

Step 7-4

According to the method in Step 1 of Production method 1, Compound (Ih) is obtained by reacting Compound (U) with Compound (II) in the presence of a base or not, and in a solvent.
The same solvent and base as the examples given in Step 1 are used.

Production method 8

Compound (Ii) is obtained from Compound (W) by the following reaction steps.

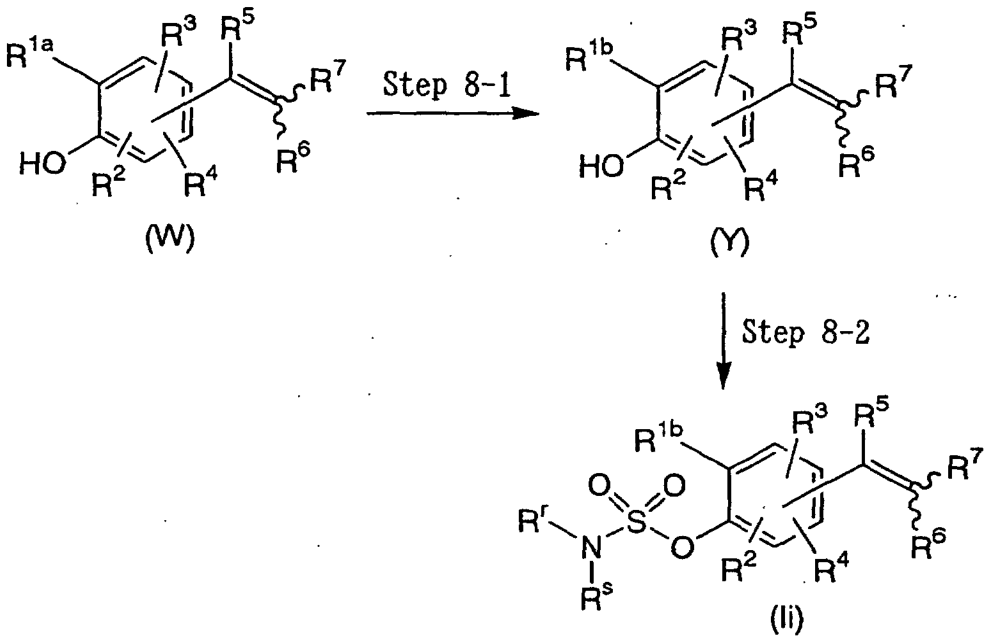


(wherein $R^r$, $R^s$, $R^2$, $R^3$, $R^4$, $R^5$, $R^6$ and $R^7$ are the same as previously described, respectively, $R^{1a}$ represents a hydrogen atom, and $R^{1b}$ represents a chlorine atom, a bromine atom or an iodine atom)

Step 8-1

Compound (Y) is obtained by treating Compound (W) with a halogenating agent in a solvent.
For the solvent, THF, toluene, dichloromethane, 1,2-dichloroethane, chloroform, diethylether, 1,4-dioxane, DMF, and the like are used individually or as a mixture.
For the halogenating agent, sulfuryl chloride, tetrabutylammonium tribromide, N-chlorosuccinimide (NCS), N-bromosuccinimide (NBS), N-iodosuccinimide (NIS), bromine, and the like are used.
The halogenating agent is used at 0.1 equivalent or above based on Compound (W), preferably at 1 to 10 equivalent. The reaction is carried out at a temperature between -78 and 120 °C, preferably -20 and 60 °C for 5 minutes to 48 hours.

Step 8-2

According to the method of Step 1 of Production method 1, Compound (Ii) is obtained by reacting Compound (Y) with Compound (II) in the presence of a base or not, and in a solvent.
The same solvent and base as the examples given in Step 1 are used.

In the production of Compound (I), the transformations of the functional groups of $R^r$, $R^s$, $R^1$, $R^2$, $R^3$, $R^4$, $R^5$, $R^6$ and $R^7$ can be carried out by known methods [for example, Comprehensive organic transformations, written by R. C. Larock (1989)].

In the above production methods, the isolation and purification of the intermediate and target compounds are conducted by methods ordinally used in organic synthesis, for example, filtration, extraction, washing, drying, concentration, crystallization, various chromatographies, combination of these methods and the like. In addition, it is also possible to use the intermediate in the following reaction without purification.

If it is desired to obtain a salt of Compound (I), when a salt of Compound (I) is obtained, then this can be purified, or when a free form of Compound (I) is obtained, then this can be dissolved or suspended in a suitable solvent, and by adding an acid or base to form a salt.

There may be various stereoisomers, geometrical isomers, regioisomers, tautomers, and the like in Compound (I) or the pharmaceutically acceptable salt thereof. For example, with Compound (I), in a compound in which one of $R^5$ or $R^6$ is hydroxy, $-CR^5=CR^6R^7$ may become tautomers of $-C(=O)-CHR^6R^7$ or $-CHR^5-C(=O)R^7$. The present invention includes all of these possible isomers and mixtures thereof. With mixtures, the ratio can be any ratio.

In addition, Compound (I) or the pharmaceutically acceptable salts thereof can exist in the form of an adduct with water or various solvents. These adducts are also included in the present invention.

Examples of Compound (I) are shown in Tables 1-4.

Table 1

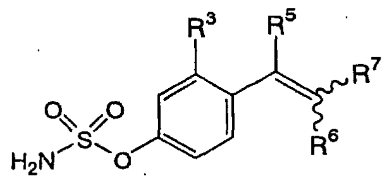

| Compound No. | $R^3$ | $R^5$ | $R^6$ | $R^7$ |
|---|---|---|---|---|
| 1 (E form) | H | H | H | $CONH(CH_2)_8CH_3$ |
| 2 (E form) | $OCH_3$ | H | H | $CONH(CH_2)_8CH_3$ |
| 3 (E form) | H | H | $CH_3$ | $CONH(CH_2)_8CH_3$ |
| 4 (E form) | H | $CH_3$ | H | $CONH(CH_2)_8CH_3$ |
| 5 (E form) | H | H | CN | $CONH(CH_2)_8CH_3$ |
| 6 (E form) | H | H | CN | $CONH(CH_2)_6CH_3$ |
| 7 (E form) | H | H | CN | $CONH(CH_2)_4CH_3$ |
| 8 (E form) | H | H | CN | $CONH(CH_2)_2CH_3$ |
| 9 (E form) | H | H | CN | $CON(CH_2CH_2CH_3)_2$ |
| 10 (E form) | H | H | CN | $CONH(CH_2CH_2O)_2(CH_2)_3CH_3$ |
| 11 (E form) | H | H | CN | $CONHCH_3$ |
| 12 (E form) | H | $CH_3$ | CN | $CONH(CH_2)_9CH_3$ |
| 13 (E form) | H | $CH_3$ | CN | $CONH(CH_2)_8CH_3$ |
| 14 (Z form) | H | $CH_3$ | CN | $CONH(CH_2)_8CH_3$ |
| 15 (E form) | H | $CH_3$ | CN | $CONH(CH_2)_7CH_3$ |
| 16 (E form) | H | $CH_3$ | CN | $CONH(CH_2)_6CH_3$ |
| 17 (E form) | H | $CH_2CH_3$ | CN | $CONH(CH_2)_8CH_3$ |
| 18 (E form) | H | $CH(CH_3)_2$ | CN | $CONH(CH_2)_8CH_3$ |
| 19 (Z form) | H | $CH_2F$ | CN | $CONH(CH_2)_8CH_3$ |
| 20 (Z form) | H | $CF_3$ | CN | $CONH(CH_2)_8CH_3$ |
| 21 (E form) | H | $CF_3$ | CN | $CONH(CH_2)_8CH_3$ |
| 22 (Z form) | H | OH | CN | $CONH(CH_2)_8CH_3$ |
| 23 | H | $OCH_3$ | CN | $CONH(CH_2)_8CH_3$ |
| 24 (E form) | H | $CH_3$ | CN | $CO_2C(CH_3)_3$ |
| 25 | H | $CH_3$ | CN | $CSNH(CH_2)_8CH_3$ |
| 26 (E form) | H | H | $CH_2CH_3$ | $CONH(CH_2)_8CH_3$ |
| 27 | H | H | $OCH_3$ | $CONH(CH_2)_8CH_3$ |
| 28 (E form) | H | H | $CH_2OCH_3$ | $CONH(CH_2)_8CH_3$ |
| 29 (E form) | $CH_3$ | H | H | $CONH(CH_2)_8CH_3$ |

Table 2

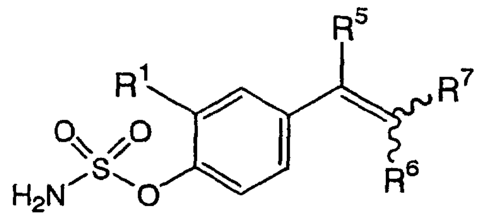

| Compound No. | $R^1$ | $R^5$ | $R^6$ | $R^7$ |
|---|---|---|---|---|
| 30 (E form) | H | H | $CH_2CONH_2$ | $CONH(CH_2)_8CH_3$ |
| 31 (E form) | H | H | $CH_2CN$ | $CONH(CH_2)_8CH_3$ |
| 32 (Z form) | H | H | $COCH_3$ | $CONH(CH_2)_8CH_3$ |
| 33 (E form) | H | H | $COCH_3$ | $CONH(CH_2)_8CH_3$ |
| 34 (E form) | H | H | $SO_2CH_3$ | $CONH(CH_2)_8CH_3$ |
| 35 | H | H | $CO_2CH_3$ | $CONH(CH_2)_8CH_3$ |
| 36 (Z form) | H | H | $N_3$ | $CONH(CH_2)_8CH_3$ |
| 37 (Z form) | H | H | $OSO_2NH_2$ | $CONH(CH_2)_8CH_3$ |
| 38 (E form) | H | H | $CH_2CON(CH_3)_2$ | $CONH(CH_2)_8CH_3$ |
| 39 (E form) | H | H | $CH_2CONH(CH_2)_8CH_3$ | $CON(CH_3)_2$ |
| 40 (Z form) | H | H | F | $CONH(CH_2)_8CH_3$ |
| 41 (Z form) | H | H | Cl | $CONH(CH_2)_8CH_3$ |
| 42 (Z form) | H | H | Br | $CONH(CH_2)_8CH_3$ |
| 43 (Z form) | H | H | I | $CONH(CH_2)_8CH_3$ |
| 44 (Z form) | H | $CH_3$ | F | $CONH(CH_2)_8CH_3$ |
| 45 (Z form) | H | $CH_3$ | Cl | $CONH(CH_2)_8CH_3$ |
| 46 (E form) | H | $CH_3$ | $CH_3$ | $CONH(CH_2)_8CH_3$ |
| 47 (Z form) | H | CN | H | $CONH(CH_2)_8CH_3$ |
| 48 (E form) | H | CN | $CH_3$ | $CONH(CH_2)_8CH_3$ |
| 49 (Z form) | H | CN | $CH_3$ | $CONH(CH_2)_8CH_3$ |
| 50 (Z form) | H | Cl | H | $CONH(CH_2)_8CH_3$ |
| 51 (Z form) | H | $OSO_2NH_2$ | H | $CONCH_3(CH_2)_7CH_3$ |
| 52 (E form) | H | H | H | $CO(CH_2)_7CH_3$ |
| 53 (E form) | H | H | $CH_2CONH(CH_2)_8CH_3$ | $CO_2CH_3$ |
| 54 | H | H | NHCHO | $CONH(CH_2)_8CH_3$ |
| 55 (E form) | Cl | $CH_3$ | CN | $CONH(CH_2)_8CH_3$ |
| 56 (E form) | Br | $CH_3$ | CN | $CONH(CH_2)_8CH_3$ |
| 57 | H | – (bond) | | $CONH(CH_2)_8CH_3$ |

Table 3

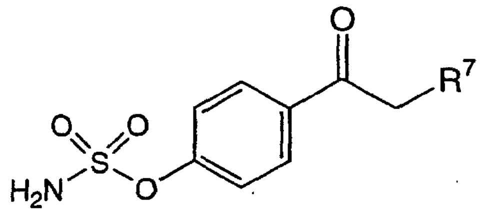

| Compound No. | $R^7$ |
|---|---|
| 58 | $CONH(CH_2)_8CH_3$ |
| 59 | $CONCH_3(CH_2)_7CH_3$ |

Table 4

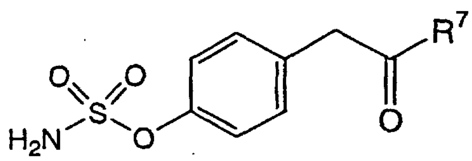

| Compound No. | $R^7$ |
|---|---|
| 60 | $CONH(CH_2)_8CH_3$ |

The pharmacological activity of representative Compound (I) is described with test examples.

Test Example 1 : Test for inhibition of estrone sulfatase (steroid sulfatase)

4-Methylumbelliferyl sulfate (final concentration 0.3 mmol/L), recombinant human estrone sulfatase (7.8 ng/well), and each solution of the test compound having each test concentrations (test compound was dissolved in 0.001 mL of DMSO) were added to a phosphate buffer (pH 7.5) (final volume 0.1 mL) containing 0.28 mol/L sucrose and 0.04 mol/L nicotinamide. An enzyme reaction was conducted at 37 °C for 1 hour. The human estrone sulfatase gene was introduced into Chinese Hamster Ovary (CHO) cells, and the recombinant human estrone sulfatase was expressed, then it was used after partial purification. After completing the enzyme reaction, 2 mol/L glycine-sodium hydroxide solution (pH 10.3) (0.13 mL) was added and stirred. Using a fluorescent plate reader (Cyto Fluor II), the fluorescent strength of the generated 4-methylumbelliferone was measured. The measurements were conducted in duplicate at the same time. The fluctuations within the assay were within 10%. An enzyme reaction without the presence of a test compound was conducted at the same time. The inhibitory activity against estrone sulfatase for each concentration of the test compounds was calculated using the following equation.

A: Amount of 4-methylumbelliferone when test compound is present
B: Amount of 4-methylumbelliferone when test compound is not present

$$\text{Inhibitory activity against estrone sulfatase} = 100 - \left(\frac{A}{B} \times 100\right)$$

The inhibitory activity against estrone sulfatase of the aryl sulfamate derivative can be expressed as the

concentration ($IC_{50}$) of aryl sulfamate derivative at which the estrone sulfatase activity in the presence of the aryl sulfamate derivative is half of that when no test compound is added.

The results of Test Example 1 are shown in Table 5.

Table 5 :

| Inhibitory activity against steroid sulfatase | |
|---|---|
| Compound No. | $IC_{50}$ (nmol/L) |
| 1 | 73 |
| 3 | 61 |
| 5 | 14 |
| 6 | 12 |
| 13 | 12 |
| 15 | <12 |
| 16 | <12 |
| 17 | 17 |
| 19 | 35 |
| 22 | <12 |
| 42 | 54 |
| 43 | 59 |
| 47 | 44 |
| 55 | 38 |
| 56 | 41 |
| 57 | 99 |
| 58 | 31 |

As shown in Table 5, Compound (I) shows inhibitory activity against steroid sulfatase and is useful as a therapeutic or prophylactic agent for steroid hormone dependent diseases.

Compound (I) or the pharmaceutically acceptable salt thereof is administered orally or parenterally as it is or in various pharmaceutical formulations. Examples of dosing forms for these pharmaceutical formulations include tablet, powder, granule, injection, and the like.

Known methods are used for formulating the dosing forms described above. For example, the resulting formulations can include various fillers, lubricants, binding agents, disintegrants, suspending agents, isotonizing agents, emulsifiers, or absorption accelerators, and the like.

Examples of carriers used in pharmaceutical formulations include water, distilled water for injection, saline, glucose, fructose, sucrose, mannitol, lactose, starch, cornstarch, cellulose, methylcellulose, carboxymethylcellulose, hydroxypropylcellulose, alginic acid, talc, sodium citrate, calcium carbonate, calcium hydrogenphosphate, magnesium stearate; urea, silicone resin, sorbitan fatty acid ester, or glycerine fatty acid ester, and the like. These are selected according to the type of formulation.

Although the dose and dosage frequency of Compound (I) used for the objective described above will depend on the target therapeutic or prophylactic effect, method of drug administration, treatment period, age, weight, and the like, the dose for a normal adult by oral or parenteral (such as injection and the like) administration is 0.01 to 20 mg/kg once or several times daily.

Preferred embodiments of the invention

Embodiments are described below. The $^1$H-NMR used in the Examples was measured at 270 MHz. The observed multiplicity, coupling constant (unit, Hz), and proton number are shown in order in the parentheses following the delta value of each signal.

Example 1 (Compound 1)

Step 1-1

4-Hydroxycinnamic acid (0.500 g, 3.05 mmol) was dissolved in THF (15 mL). While cooling on ice, EDCI (1.46 g, 7.63 mmol) and HOBt (0.467 g, 3.05 mmol) were added, and the mixture was stirred for 10 minutes at the same

temperature. Then, n-nonylamine (1.68 mL, 9.15 mmol) was added, and the mixture was stirred for 1.5 hours at room temperature. The reaction solution was added with water and 1 mol/L hydrochloric acid, and extracted with ethyl acetate. After the organic layer was washed with saturated aqueous sodium hydrogencarbonate solution and then saturated brine, the organic layer was dried over anhydrous sodium sulfate and concentrated under reduced pressure. The obtained residue was purified by silica gel column chromatography (n-hexane/ethyl acetate = 2/1～1/1), and (E)-3-(4-hydroxyphenyl)-N-nonylacrylamide (0.801 g, 91% yield) was obtained. APCI-MS m/z: $[M+H]^+$ 290. $^1$H-NMR (DMSO-$d_6$) δ (ppm): 0.85 (t, J = 6.6 Hz, 3H), 1.25 (m, 12H), 1.43 (m, 2H), 3.13 (m, 2H), 6.39 (d, J = 15.7 Hz, 1H), 6.78 (d, J = 8.6 Hz, 2H), 7.29 (d, J = 15.7 Hz, 1H), 7.37 (d, J = 8.6 Hz, 2H), 7.92 (t, J = 5.6 Hz, 1H), 9.80 (brs, 1H).

Step 1-2

(E)-3-(4-Hydroxyphenyl)-N-nonylacrylamide (50 mg, 0.17 mmol) was dissolved in DMAC (1.5 mL). Sulfamoyl chloride (40 mg, 0.35 mmol) was added, and the mixture was stirred for 1 hour at room temperature. The reaction solution was added with water, and extracted with ethyl acetate. After the organic layer was washed with saturated brine, the organic layer was dried over anhydrous sodium sulfate and concentrated under reduced pressure. The obtained residue was purified by preparative thin layer chromatography (chloroform/methanol= 15/1), and Compound 1 (57 mg, 89% yield) was obtained.
FAB-MS m/z: $[M+H]^+$ 369.
$^1$H-NMR (DMSO-$d_6$) δ (ppm): 0.85 (t, J = 6.6 Hz, 3H), 1.25 (m, 12H), 1.44 (m, 2H), 3.15 (m, 2H), 6.60 (d, J = 15.7 Hz, 1H), 7.30 (d, J = 8.8 Hz, 2H), 7.42 (d, J = 15.7 Hz, 1H), 7.64 (d, J = 8.8 Hz, 2H), 8.05 (s, 2H), 8.09 Gm, 1H).

Example 2 (Compound 2)

Step 2-1

4-Hydroxy-2-methoxybenzaldehyde (500 mg, 3.29 mmol) was dissolved in pyridine (8mL). Malonic acid (685 mg, 6.58 mmol) and piperidine (1.63 mL, 16.5 mmol) were added, and the mixture was stirred for 1.5 hours at room temperature and then for 1.2 hours at 60 °C. The reaction solution was poured into ice water and made acidic with 6 mol/L hydrochloric acid. The precipitate that was deposited was collected by filtration and washed with water. The product was dried in vacuo at 50 °C, and (E)-3-(4-hydroxy-2-methoxyphenyl) acrylic acid (353 mg, 55% yield) was obtained.
APCI-MS m/z: $[M-H]^-$ 193.
$^1$H-NMR ($CDCl_3$ + $CD_3OD$) δ (ppm): 3.85 (s, 3H), 6.36 (d, J = 16.0 Hz, 1H), 6.43 (d, J = 1.9 Hz, 1H), 6.44 (dd, J = 1.9, 7.5 Hz, 1H), 7.37 (d, J = 7.5 Hz, 1H), 7.92 (d, J = 16.0 Hz, 1H).

Step 2-2

According to Step 1-1 of Example 1, (E)-3-(4-hydroxy-2-methoxyphenyl) acrylic acid (100 mg, 0.515 mmol) was treated with EDCI (247 mg, 1.29 mmol), HOBt (79 mg, 0.52 mmol), and n-nonylamine (0.28 mL, 1.5 mmol) to thereby obtain (E)-3-(4-hydroxy-2-methoxyphenyl)-N-nonylacrylamide (127 mg, 77% yield).
APCI-MS m/z: $[M+H]^+$ 320.

Step 2-3

According to Step 1-2 of Example 1, (E)-3-(4-hydroxy-2-methoxyphenyl)-N-nonylacrylamide (60 mg, 0.19 mmol) was treated with sulfamoyl chloride (43 mg, 0.38 mmol) to thereby obtain Compound 2 (63 mg, 84% yield).
APCI-MS m/z: $[M+H]^+$ 399.
$^1$H-NMR (DMSO-$d_6$) δ (ppm): 0.85 (t, J = 6.6 Hz, 3H), 1.25 (m, 12H), 1.44 (m, 2H), 3.15 (m, 2H), 3.87 (s, 3H), 6.63 (d, J = 16.0 Hz, 1H), 6.91 (dd, J = 2.1, 8.5 Hz, 1H), 6.97 (d, J = 2.1 Hz, 1H), 7.57 (d, J = 8.5 Hz, 1H), 7.59 (d, J = 16.0 Hz, 1H), 8.05 (brs, 3H).

Example 3 (Compound 3)

Step 3-1

According to Step 1-1 of Example 1, (E)-3-(4-hydroxyphenyl)-2-methylacrylic acid (50 mg, 0.28 mmol) was treated with EDCI (84 mg, 0.44 mmol), HOBt (69 mg, 0.45 mmol), and n-nonylamine (0.077 mL, 0.42 mmol) to thereby obtain (E)-3-(4-hydroxyphenyl)-2-methyl-N-nonylacrylamide (57 mg, 66% yield).

APCI-MS m/z: [M-H]- 302.

Step 3-2

According to Step 1-2 of Example 1, (E)-3-(4-hydroxyphenyl)-2-methyl-N-nonylacrylamide (52 mg, 0.17 mmol) was treated with sulfamoyl chloride (50 mg, 0.44 mmol) to thereby obtain Compound 3 (65 mg, 100% yield)..
APCI-MS m/z: [M-H]- 381.
$^1$H-NMR (DMSO-$d_6$) δ (ppm): 0.86 (t, J = 6.7 Hz, 3H), 1.15-1.37 (m, 12H), 1.47 (m, 2H), 2.00 (d, J = 1.3 Hz, 3H), 3.14 (dt, J = 6.0, 7.0 Hz, 2H), 7.18 (s, 1H), 7.30 (d, J = 8.7 Hz, 2H), 7.46 (d, J = 8.7 Hz, 2H), 8.00 (t, J = 6.0 Hz, 1H), 8.03 (s, 2H).

Example 4 (Compound 4)

Step 4-1

According to 1-1 of Example 1, (E)-3-(4-methoxyphenyl)-2-butenoic acid (27 mg, 0.14 mmol) was treated with EDCI (43 mg, 0.23 mmol), HOBt (34 mg, 0.22 mmol), and n-nonylamine (0.038 mL, 0.21 mmol) to thereby obtain (E)-3-(4-methoxyphenyl)-N-nonyl-2-butenamide (39 mg, 89% yield).
APCI-MS m/z: $[M+H]^+$ 318.

Step 4-2

(E)-3-(4-methoxyphenyl)-N-nonyl-2-butenamide (24 mg, 0.076 mmol) was dissolved in dichloromethane (1.2 mL). Boron tribromide (0.30 mL, 0.30 mmol, 1.0 mol/L dichloromethane solution) was added at -18 °C, and the mixture was stirred for 2 hours at room temperature. The reaction solution was added with methanol and water, and extracted with chloroform. After the organic layer was washed with saturated aqueous sodium hydrogen carbonate solution and then saturated brine, the organic layer was dried over anhydrous sodium sulfate and concentrated under reduced pressure. The obtained residue was purified by preparative thin layer chromatography (chloroform/acetone = 4/1), and (E)-3-(4-hydroxyphenyl)-N-nonyl-2-butenamide (13 mg, 57 % yield) was obtained.
APCI-MS m/z: $[M+H]^+$ 304.

Step 4-3

According to Step 1-2 of Example 1, (E)-3-(4-hydroxyphenyl)-N-nonyl-2-butenamide (17 mg, 0.054 mmol) was treated with sulfamoyl chloride (13 mg, 0.11 mmol) to thereby obtain Compound 4 (18 mg, 87% yield).
APCI-MS m/z: $[M+H]^+$ 383.
$^1$H-NMR (DMSO-$d_6$) δ (ppm): 0.86 (m, 3H), 1.10-1.50 (m, 14H), 2.46 (s, 3H), 3.11 (dt, J = 6.1, 6.8 Hz, 2H), 6.19 (s, 1H), 7.29 (d, J = 8.6 Hz, 2H), 7.56 (d, J = 8.6 Hz, 2H), 7.96 (brt, 1H), 8.03 (s, 1H).

Example 5 (Compound 5 to Compound 10)

According to Steps 1-1 and 1-2 of Example 1, after condensing α -cyano-4-hydroxycinnamic acid with the corresponding amine, the product was sulfamoylated to thereby obtain each of Compound 5 to Compound 10.

Compound 5

APCI-MS m/z: $[M+H]^+$ 394.
$^1$H-NMR (DMSO-$d_6$) δ (ppm): 0.83 (m, 3H), 1.15-1.58 (m, 14H), 3.19 (m, 2H), 7.46 (d, J = 8.7 Hz, 2H), 8.02 (d, J = 8.7 Hz, 2H), 8.17 (s, 1H), 8.21 (s, 1H), 8.45 (m, 1H).

Compound 6

APCI-MS m/z: $[M+H]^+$ 366.
$^1$H-NMR (DMSO-$d_6$) δ (ppm): 0.87 (t, J = 6.6 Hz, 3H), 1.16-1.38 (m, 8H), 1.50 (m, 2H), 3.20 (m, 2H), 7.46 (d, J = 8.8 Hz, 2H), 8.02 (d, J = 8.8 Hz, 2H), 8.17 (s, 1H), 8.20 (s, 1H), 8.45 (t, J = 5.3 Hz, 1H).

Compound 7

APCI-MS m/z: $[M+H]^+$ 338.

$^1$H-NMR (DMSO-$d_6$) δ (ppm): 0.87 (t, J = 6.8 Hz, 3H), 1.19-1.40 (m, 4H), 1.50 (m, 2H), 3.20 (m, 2H), 7.45 (d, J = 8.7 Hz, 2H), 8.02 (d, J = 8.7 Hz, 2H), 8.16 (s, 1H), 8.20 (s, 1H), 8.45 (t, J = 4.8 Hz, 1H).

Compound 8

APCI-MS m/z: $[M+H]^+$ 310.
$^1$H-NMR (DMSO-$d_6$) δ (ppm): 0.87 (t, J = 7.4 Hz, 3H), 1.51 (m, 2H), 3.17 (m, 2H), 7.45 (d, J = 8.7 Hz, 2H), 8.02 (d, J = 8.7 Hz, 2H), 8.16 (s, 1H), 8.20 (s, 1H), 8.46 (t, J = 4.9 Hz, 1H).

Compound 9

APCI-MS m/z: $[M+H]^+$ 352.
$^1$H-NMR (DMSO-$d_6$) δ (ppm): 0.85 (m, 6H), 1.59 (m, 4H), 3.30 (m, 4H), 7.43 (d, J = 8.6 Hz, 2H), 7.75 (s, 1H), 7.98 (d, J = 8.6 Hz, 2H), 8.17 (s, 1H).

Compound 10

APCI-MS m/z: $[M+H]^+$ 412.
$^1$H-NMR (DMSO-$d_6$) δ (ppm): 0.84 (t, J = 7.3 Hz, 3H), 1.29 (m, 2H), 1.45 (m, 2H), 3.20-3.65 (m, 8H), 3.37 (t, J = 6.5 Hz, 2H), 7.45 (d, J = 8.9 Hz, 2H), 8.02 (d, J = 8.9 Hz, 2H), 8.19 (s, 2H), 8.44 (t, J = 5.6 Hz, 1H).

Example 6 (Compound 11)

Step 6-1

According to Step 1-1 of Example 1, α-cyano-4-hydroxycinnamic acid (51 mg, 0.27 mmol) was treated with 1-(3-dimethylaminopropyl)-3-ethylcarbodiimide (0.077 mL, 0.40 mmol), HOBt (63 mg, 0.41 mmol), and methylamine hydrochloride (31 mg, 0.46 mmol) to thereby obtain (E)-2-cyano-3-(4-hydroxyphenyl)-N-methylacrylamide (33 mg, 62% yield).
APCI-MS m/z: $[M-H]^-$ 201.

Step 6-2

According to Step 1-2 of Example 1, (E)-2-cyano-3-(4-hydroxyphenyl)-N-methylacrylamide (31 mg, 0.16 mmol) was treated with sulfamoyl chloride (43 mg, 0.38 mmol) to thereby obtain Compound 11 (39 mg, 89% yield):
APCI-MS m/z:$[M+H]^+$ 282.
$^1$H-NMR (DMSO-$d_6$) δ (ppm): 2.75 (d, J = 4.6 Hz, 3H), 7.45 (d, J = 8.8 Hz, 2H), 8.02 (d, J = 8.8 Hz, 2H), 8.18 (s, 1H), 8.19 (s, 1H), 8.40 (m, 1H).

Example 7 (Compound 12)

Step 7-1

p-Acetylphenol (20.0 g, 147 mmol) was dissolved in DMF (200 mL). While cooling on ice, sodium hydride (6.46 g, 0.16 mol, 60% in oil) and then chloromethyl methylether (16.7 mL, 220 mmol) were added to the solution, and the mixture was stirred for 2 hours at the same temperature. The reaction solution was added with ice and saturated aqueous sodium hydrogencarbonate, and extracted with ethyl acetate. After the organic layer was washed with saturated brine, the organic layer was dried over anhydrous sodium sulfate and concentrated under reduced pressure. The obtained residue was,purified by silica gel column chromatography (n-hexane/ethyl acetate = 9/1), and 4-(methoxymethoxy)acetophenone (23.7 g, 89% yield) was obtained.
FAB -MS m/z: $[M+H]^+$ 181.
$^1$H-NMR ($CDCl_3$) δ (ppm): 2.56 (s, 3H), 3.48 (s, 3H), 5.23 (s, 2H), 7.07 (d, J = 9.1 Hz, 2H), 7.93 (d, J = 9.1 Hz, 2H).

Step 7-2

4-(Methoxymethoxy)acetophenone (150 mg, 0.831 mmol) and 2-cyano-N-decylacetamide (187 mg, 0.834 mmol) obtained in Reference Example 2 were dissolved in toluene (3 mL). Ammonium acetate (500 mg, 6.49 mmol) and acetic acid (1.17 mL, 20.4 mmol) were added to the solution. Using a Dean-Stark device in which the receiver was

filled with toluene, the mixture was heated under reflux for 7 hours. After cooling the reaction solution, the reaction solution was added with water, and extracted with ethyl acetate. After the organic layer was washed with saturated brine, the organic layer was dried over anhydrous sodium sulfate and concentrated under reduced pressure. The obtained residue was purified by silica gel column chromatography (n-hexane/ethyl acetate = 9/1), and (E)-2-cyano-N-decyl-3-(4-(methoxymethoxy)phenyl]-2-butenamide (120 mg, 37% yield) and (Z)-2-cyano-N-decyl-3-[4-(methoxymethoxy)phenyl]-2-butenamide (820 mg, 26% yield) were obtained.

E-form

ESI-MS m/z: $[M-H]^-$ 385.
$^1$H-NMR ($CDCl_3$) δ (ppm): 0.88 (t, J = 6.6 Hz, 3H), 1.18-1.44 (m, 14H), 1.58 (m, 2H), 2.65 (s, 3H), 3.35 (dt, J = 6.1, 7.0 Hz, 2H), 3.49 (s, 3H), 5.21 (s, 2H), 6.31 (brt, 1H), 7.09 (d, J = 8.9 Hz, 2H), 7.41 (d, J = 8.9 Hz, 2H).

Z-form

ESI-MS m/z: $[M+H]^+$ 387.
$^1$H-NMR ($CDCl_3$) δ (ppm): 0.88 (t, J = 6.8 Hz, 3H), 1.02-1.39 (m, 16H), 2.48 (s, 3H), 3.14 (dt, J = 5.9, 6.9 Hz, 2H), 3.47 (s, 3H), 5.19 (s, 2H), 5.49 (brt, 1H), 7.04 (d, J = 8.8 Hz, 2H), 7.23 (d, J = 8.8 Hz, 2H).

Step 7-3

(E)-2-Cyano-N-decyl-3-[4-(methoxymethoxy)phenyl]-2-butenamide (120 mg, 0.310 mmol) was dissolved in a mixed solvent of THF (4 mL) and isopropyl alcohol (4 mL). 4 mol/L hydrochloric acid (4 mL) was added to the solution, and the mixture was stirred for 1 hour at 40°C. The reaction solution was added with ice and saturated aqueous sodiumhydrogen carbonate solution, and extracted with ethyl acetate. After the organic layer was washed with saturated brine, the organic layer was dried over anhydrous sodium sulfate and concentrated under reduced pressure. The obtained residue was triturated with a mixed solvent of diisopropyl ether and n-hexane, and (E)-2-cyano-N-decyl-3-(4-hydroxyphenyl)-2-butenamide (91 mg, 86% yield) was obtained.
ESI-MS m/z: [M-H]- 341.
$^1$H-NMR ($CDCl_3$) δ (ppm): 0.88 (t, J = 6.6 Hz, 3H), 1.10-1.40 (m, 14H), 1.56 (m, 2H), 2.65 (s, 3H), 3.36 (dt, J = 6.1, 7.0 Hz, 2H), 5.32 (brs, 1H), 6.27 (brt, 1H), 6.88 (d, J = 8.8 Hz, 2H), 7.38 (d, J = 8.8 Hz, 2H).

Step 7-4

According to Step 1-2 of Example 1, (E)-2-cyano-N-decyl-3-(4-hydroxyphenyl)-2-butenamide (86 mg, 0.25 mmol) was treated with sulfamoyl chloride (70 mg, 0.61 mmol) to thereby obtain Compound 12 (83 mg, 79% yield).
ESI-MS m/z: $[M-H]^-$ 420.
$^1$H-NMR (DMSO-$d_6$) δ (ppm): 0.85 (t, J = 6.6 Hz, 3H), 1.12-1.38 (m, 14H), 1.48 (m, 2H), 2.27 (s, 3H), 3.17 (m, 2H), 7.38 (d, J = 8.6 Hz, 2H), 7.61 (d, J = 8.6 Hz, 2H), 8.14 (s, 2H), 8.65 (brt, J = 5.5 Hz, 1H).

Example 8 (Compound 13 to Compound 16)

According to Step 7-2 of Example 7, 4-(methoxymethoxy)acetophenone was condensed with the corresponding N-alkyl-2-cyanoacetamide (following Reference Example 2, this compound was obtained from cyanoacetic acid and the corresponding amine). Then, according to Steps 7-3 and 7-4 of Example 7, demethoxymethylation and sulfamoylation were conducted to thereby obtain Compound 13 to Compound 16.

Compound 13

FAB-MS m/z: [M+H]+ 408.
$^1$H-NMR CDMSO-$d_6$) δ (ppm): 0.85 (t, J = 6.7 Hz, 3H), 1.20-1.40 (m, 12H), 1.48 (m, 2H), 2.27 (s, 3H), 3.18 (dt, J = 5.8, 6.8 Hz, 2H), 7.38 (d, J = 8.8 Hz, 2H), 7.60 (d, J = 8.8 Hz, 2H), 8.13 (brs, 2H), 8.65 (t, J = 5.8 Hz, 1H).

Compound 14

FAB-MS m/z: $[M+H]^+$ 408.
$^1$H-NMR (DMSO-$d_6$) δ (ppm): 0.85 (m, 3H), 0.90-1.35 (m, 14H), 2.39 (s, 3H), 2.91 (m, 2H), 7.26 (d, J = 8.3 Hz, 2H), 7.41 (d, J = 8.3 Hz, 2H), 8.07 (brs, 2H), 8.28 (t, J = 5.5 Hz, 1H).

Compound 15

APCI-MS m/z: $[M-H]^-$ 392.
$^1H$-NMR (DMSO-$d_6$) δ (ppm): 0.85 (t, J = 6.8 Hz, 3H), 1.15-1.35 (m, 10H), 1.48 (m, 2H), 2.27 (s, 3H), 3.18 (dt, J = 5.8, 6.7 Hz, 2H), 7.38 (d, J = 8.8 Hz, 2H), 7.61 (d, J = 8.8 Hz, 2H), 8.14 (s, 2H), 8.66 (t, J = 5.8 Hz, 1H).

Compound 16

APCI-MS m/z: $[M-H]^-$ 378.
$^1H$-NMR (DMSO-$d_6$) δ (ppm): 0.87 (t, J = 6.8 Hz, 3H), 1.16-1.39 (m, 8H), 1.48 (m, 2H), 2.49 (s, 3H), 3.18 (dt, J = 5.9, 6.5 Hz, 2H), 7.38 (d, J = 8.5 Hz, 2H), 7.61 (d, J = 8.5 Hz, 2H), 8.15 (s, 2H), 8.67 (t, J = 5.9 Hz, 1H).

Example 9 (Compound 17)

Step 9-1

According to Step 7-1 of Example 7, p-hydroxypropiophenone (1.00 g, 6.66 mmol) was treated with chloromethyl methylether (0.76 mL, 1.0 mmol) and sodium hydride (293 mg, 7.3 mmol, 60% in oil) to thereby obtain 4-(methoxymethoxy)propiophenone (1.30 g 100% yield).
FAB-MS m/z: $[M+H]^+$ 195.
$^1H$-NMR ($CDCl_3$) δ (ppm): 1.21 (t, J = 7.3 Hz, 3H), 2.96 (q, J = 7.3 Hz, 2H), 3.48 (s, 3H), 5.23 (s, 2H), 7.07 (d, J = 8.9 Hz, 2H), 7.94 (d, J = 8.9 Hz, 2H).

Step 9-2

According to Step 7-2 of Example 7, 4-(methoxymethoxy)propiophenone was condensed with 2-cyano-N-nonylacetamide, which was obtained in Reference Example 3. Then, according to Steps 7-3 and 7-4 of Example 7, demethoxymethylation and sulfamoylation were conducted to thereby obtain Compound 17.
APCI-MS m/z: [M-H]- 341.
$^1H$-NMR ($CDCl_3$) δ (ppm): 0.88 (t, J = 6.8 Hz, 3H), 1.04 (t, J = 7.4 Hz, 3H), 1.11-1.41 (m, 12H), 1.58 (m, 2H), 3.06 (q, J = 7.4 Hz, 2H), 3.36 (dt, J = 5.9, 7.1 Hz, 2H), 6.27 (brt, 1H), 6.88 (d, J = 8.8 Hz, 2H), 7.31 (d, J = 8.8 Hz, 2H).

Example 10 (Compound 18)

Step 10-1

DMSO (70 mL) was added to potassium hydroxide (31.51 g, 561.6 mmol). While stirring at 60 °C, 4-(methoxymethoxy)acetophenone (4.93 g, 27.4 mmol) and methyl iodide (13.6 mL, 218 mmol) dissolved in DMSO (25 mL) were added to the solution over 1 hour. Then, the mixture was further stirred for 2 hours at the same temperature. After cooling the reaction solutionl, the reaction solution was added with ice water, and extracted with ethyl acetate. After the organic layer was washed with water and then saturated brine, the organic layer was dried over anhydrous sodium sulfate and concentrated under reduced pressure. The obtained residue was purified by silica gel column chromatography (n-hexane/ethyl acetate = 20/1), and 1-[4-(methoxymethoxy)phenyl)-2-methylpropane-1-one (1.40 g, 25% yield) was obtained.
$^1H$-NMR ($CDCl_3$) δ (ppm): 1.21 (d, J = 6.8 Hz, 6H), 3.50 (s, 3H), 3.51 (m, 1H), 5.23 (s, 2H), 7.07 (d, J = 9.1 Hz, 2H), 7.94 (d,J= 9.1 Hz, 2H).

Step 10-2

According to Step 7-2 of Example 7, 1-[4-(methoxymethoxy)phenyl]-2-methylpropane-1-one (1.17 g, 5.62 mmol) was treated with ethyl cyanoacetate (1.23 mL, 11.2 mmol), ammonium acetate (1.57 g, 20.4 mmol), and acetic acid (4.70 mL, 82.1 mmol) to thereby obtain ethyl (E)-2-cyano-3-[4-(methoxymethoxy)phenyl]-4-methyl-2-pentenoate (0.87 g, 51% yield).
APCI-MS m/z: $[M-H]^-$ 302.
$^1H$-NMR ($CDCl_3$) δ (ppm): 1.09 (t, J = 7.2 Hz, 3H), 1.10 (d, J = 7.0 Hz, 6H), 3.46 (q, J = 7.0 Hz, 1H), 3.49 (s, 3H), 4.04 (q, J = 7.2 Hz, 2H) , 5.19 (s, 2H), 6.92 (d, J = 8.9 Hz, 2H), 7.04 (d, J = 8.9 Hz, 2H).

Step 10-3

Ethyl (E)-2-cyano-3-[4-(methoxymethoxy)phenyl]-4-methyl-2-pentenoate (120 mg, 0.396 mmol) was dissolved in a mixted solvent of methanol (2.4 mL) and water (0.8 mL). Lithium hydroxide (47 mg, 2.0 mmol) was added to the solution, and the mixture was stirred for 25 minutes at 40 °C. Methanol was concentrated under reduced pressure, and the reaction solution was added with 1.0 mol/L hydrochloric acid (2 mL), and extracted with ethyl acetate. After the organic layer was washed with saturated brine, the organic layer was dried over anhydrous sodium sulfate. The obtained organic layer was concentrated under reduced pressure, and (E)-2-cyano-3-[4-(methoxymethoxy)phenyl]-4-methyl-2-pentenoic acid (111 mg) was obtained.
APCI-MS m/z: $[M-H]^-$ 274.

Step 10-4

According to Step 1-1 of Example 1, (E)-2-cyano-3-[4-(methoxymethoxy)phenyl]-4-methyl-2-pentenoic acid (111 mg) was treated with EDCI (114 mg, 0.594 mmol), HOBt (91 mg, 0.59 mmol) and n-nonylamine (0.108 mL, 0.594 mmol), to thereby obtain (E)-2-cyano-3-[4-(methoxymethoxy)phenyl]-4-methyl-N-nonyl-2-pentenamide (20 mg, 12 % yield).
APCI-MS m/z: $[M+H]^+$ 401.
$^1$H-NMR ($CDCl_3$) δ (ppm): 0.88 (t, J = 6.7 Hz, 3H), 1.04 (d, J = 6.8 Hz, 6H), 1.16-1.41 (m, 12H), 1.55 (m, 2H), 3.35 (dt, J = 5.9, 7.0 Hz, 2H), 3.50 (s, 3H), 4.14 (m, 1H), 5.20 (s, 2H), 6.19 (m, 1H), 7.08 (m, 4H).

Step 10-5

According to Steps 7-3 and 7-4 of Example 7, demethoxymethylation and sulfamoylation of (E)-2-cyano-3-[4-(methoxymethoxy) phenyl]-4-methyl-N-nonyl-2-pentenamide was conducted, and Compound 18 was obtained.
APCI-MS m/z: $[M-H]^-$ 355.
$^1$H-NMR ($CDCl_3$) δ (ppm): 0.88 (t, J = 6.7 Hz, 3H), 1.03 (d, J = 6.9 Hz, 6H), 1.14-1.44 (m, 12H), 1.58 (m, 2H), 3.35 (dt, J = 6.1, 7.0 Hz, 2H), 4.07 (m, 1H), 5.33 (s, 2H), 6.28 (brt, 1H), 7.19 (d, J = 8.4 Hz, 2H), 7.38 (d, J = 8.4 Hz, 2H).

Example 11 (Compound 19)

Step 11-1

According to Step 7-1 of Example 7, p-bromophenol (4.00 g, 23.1 mmol) was treated with sodium hydride (1.02 g, 25 mmol, 60% in oil), and chloromethyl methylether (2.63 mL, 34.7 mmol) to thereby obtain 1-bromo-4-(methoxymethoxy)benzene (5.18 g, 100% yield).
$^1$H-NMR ($CDCl_3$) δ (ppm): 3.46 (s; 3H), 5.14 (s, 2H), 6.92 (d, J = 9.1 Hz, 2H), 7.38 (d, J = 9.1 Hz, 2H).

Step 11-2

1-Bromo-4-(methoxymethoxy)benzene (270 mg, 1.24 mmol) was dissolved in THF (2 mL), and n-buytl lithium (0.86 mL, 2.2 mmol, 2.5 mol/L n-hexane solution) was gradually added to the solution at -78 °C. After stirring for 5 minutes, ethyl fluoroacetate (0.156 mL, 1.62 mmol) was gradually added at the same temperature, and the mixture was stirred for 15 minutes. The reaction solution was added water, and extracted with ethyl acetate. After the organic layer was washed with saturated brine, the organic layer was dried over anhydrous sodium sulfate and concentrated under reduced pressure. The obtained residue was purified by silica gel column chromatography (n-hexane/ethyl acetate = 10/1), to thereby obtain 2-fluoro-1-[4-(methoxymethoxy)phenyl] ethanone (138 mg, 56% yield).
FAB-MS m/z: [M+H]+ 199.
$^1$H-NMR ($CDCl_3$) δ (ppm): 3.48 (s, 3H), 5.24 (s, 2H), 5.48 (d, J = 47.2 Hz, 2H), 7.10 (d, J = 8.9 Hz, 2H), 7.88 (d, J = 8.9 Hz, 2H).

Step 11-3

According to Step 7-2 of Example 7, 2-fluoro-1-[4-(methoxymethoxy)phenyl]ethanone was condensed with 2-cyano-N-nonylacetamide obtained in Reference Example 3. Then, following Steps 7-3 and 7-4 of Example 7, demethoxymethylation and sulfamoylation were conducted to thereby obtain Compound 19.
APCI-MS m/z: $[M-H]^-$ 424.
$^1$H-NMR ($CDCl_3$) δ (ppm): 0.88 (t, J = 6.7 Hz, 3H), 1.19-1.40 (m, 12H), 1.57 (m, 2H), 3.35 (dt, J = 6.4, 7.2 Hz, 2H),

5.15 (s, 2H), 5.89 (d, J = 48.6 Hz, 2H), 6.39 (brt, 1H), 7.40-7.53 (m, 4H).

Example 12 (Compound 20 and Compound 21)

Step 12-1

According to Step 11-2 of Example 11, 1-bromo-4-(methoxymethoxy)benzene (270 mg, 1.24 mmol) was treated with n-butyl lithium (0.60 mL, 1.5 mmol, 2.5 mol/L n-hexane solution) and ethyl trifluoroacetate (0.223 mL, 1.87 mmol) to thereby obtain 2,2,2-trifluoro-1-[4-(methoxymethoxy)phenyl]ethanone (132 mg, 47% yield).
FAB-MS m/z: $[M+H]^+$ 235.
$^1$H-NMR ($CDCl_3$) δ (ppm): 3.49 (s; 3H), 5.27 (s, 2H), 7.14 (d, J = 9.1 Hz, 2H), 8.05 (d, J = 9.1 Hz, 2H).

Step 12-2

According to Step 7-2 of Example 7, 2,2,2-trifluoro-1-[4-(methoxymethoxy)phenyl]ethanone was condensed with 2-cyano-N-nonylacetamide obtained in Reference Example 3. Next, according to Steps 7-3 and 7-4 of Example 7, demethoxymethylation and sulfamoylation were conducted to thereby obtain Compound 20 and Compound 21.

Compound 20

APCI-MS m/z: $[M-H]^-$ 460.
$^1$H-NMR ($CDCl_3$) δ (ppm): 0.88 (t, J = 6.6 Hz, 3H), 1.17-1.43 (m, 12H), 1.58 (m, 2H), 3.40 (dt, J = 6.6, 6.9 Hz, 2H), 5.16 (s, 2H), 6.04 (brt, 1H), 7.45 (d, J = 9.1 Hz, 2H), 7.51 (d, J = 9.1 Hz, 2H).

Compound 21

APCI-MS m/z: [M-H]- 460.
$^1$H-NMR ($CD_3OD$) δ (ppm): 0.80 (t, J = 6.8 Hz, 3H), 0.92 (m, 2H), 0.99-1.32 (m, 12H), 2.91 (t, J = 6.8 Hz, 2H), 7.25-7.42 (m, 4H).

Example 13 (Compound 22)

Step 13-1

According to Step 7-1 of Example 7, methyl p-hydroxybenzoate (3.01 g, 19.8 mmol) was treated with sodium hydride (0.95 g, 24 mmol, 60% in oil) and chloromethyl methylether (2.25 mL, 29.6 mmol) to thereby obtain methyl 4-(methoxymethoxy)benzoate (3.88 g, 100% yield).
FAB-MS m/z: $[M+H]^+$ 197.
$^1$H-NMR ($CDCl_3$) δ (ppm): 3.48 (s, 3H), 3.89 (s, 3H), 5.22 (s, 2H), 7.05 (d, J = 9.1 Hz, 2H), 7.99 (d, J = 9.1 Hz, 2H).

Step 13-2

According to Step 10-3 of Example 10, methyl 4-(methoxymethoxy)benzoate (1.68 g, 8.58 mmol) was treated with lithium hydroxide (1.03 g, 43.0 mmol) to thereby obtain 4-(methoxymethoxy)benzoic acid (1.37 g, 88% yield).
FAB-MS m/z: $[M+H]^+$ 183.
$^1$H-NMR ($CDCl_3$) δ (ppm): 3.49 (s, 3H), 5.25 (s, 2H), 7.09 (d, J = 8.9 Hz, 2H), 8.07 (d, J = 8.9 Hz, 2H).

Step 13-3

4-(Methoxymethoxy)benzoic acid (500 mg, 2.74 mmol) was dissolved in DMF (5 mL). While cooling on ice, 2-cyano-N-nonylacetamide (576 mg, 2.74 mmol) obtained in Reference Example 3, DEPC (0.499 mL, 3.29 mmol), and triethylamine (1.22 mL, 8.77 mmol) were added to the solution. The mixture was stirred for 2 hours at the same temperature and then for 2 hours at room temperature. Ethyl acetate was added to the reaction solution. The obtained mixture was washed with 10% sulfuric acid, water, saturated aqueous sodium hydrogencarbonate solution, and saturated brine, and then the organic layer was dried over anhydrous sodium sulfate and concentrated under reduced pressure. The obtained residue was purified by silica gel column chromatography (n-hexane/ethyl acetate = 17/3), and 2-cyano-3-hydroxy-3-[4-(methoxymethoxy)phenyl]-N-nonylacrylamide (486 mg, 47% yield) was obtained.
APCI-MS m/z: [M-H]- 373.

$^1$H-NMR ($CDCl_3$) δ (ppm): 0.88 (t, J = 6.8 Hz, 3H), 1.20-1.46 (m, 12H), 1.58 (m, 2H), 3.37 (m, 2H), 3.48 (s, 3H), 5.23 (s, 2H), 6.22 (brs, 1H), 7.10 (d, J = 8.9 Hz, 2H), 7.95 (d, J = 8.9 Hz, 2H).

Step 13-4

According to Steps 7-3 and 7-4 of Example 7, demethoxymethykation and then sulfamoylation of 2-cyano-3-hydroxy-3-[4-(methoxymethoxy)phenyl]-N-nonylacrylamide were conducted to thereby obtain Compound 22.
APCI-MS m/z: $[M-H]^-$ 408.
$^1$H-NMR ($CDCl_3$) δ (ppm): 0.89 (t, J = 7.3 Hz, 3H), 0.99-1.50 (m, 12H), 1.62 (m, 2H), 3.37 (m, 2H), 6.25 (m, 2H), 7.45 (m, 2H), 8.01 (m, 2H).

Example 14 (Compound 23)

Step 14-1

2-Cyano-3-hydroxy-3-[4-(methoxymethoxy)phenyl]-N-nonylacrylamide (31 mg, 0.082 mmol) was dissolved in dichloromethane (0.5 mL). Trimethylsilyldiazomethane (0.165 mL, 0.30 mmol, 2.0 mol/L n-hexane solution) was added to the solution, and the mixture was stirred for 10 minutes at room temperature. The reaction solution was concentrated under reduced pressure, and the obtained residue was purified by preparative thin layer chromatography (n-hexane/ethyl acetate = 2/1), and 2-cyano-3-methoxy-3-[4-(methoxymethoxy)phenyl]-N-nonylacrylamide (24 mg, 74 % yield) was obtained.
APCI-MS m/z: $[M+H]^+$ 389.
$^1$H-NMR ($CDCl_3$) δ (ppm): 0.88 (t, J = 7. Hz, 3H), 1.18-1.37 (m, 12H), 1.57 (m, 2H), 3.37 (m, 2H), 3.50 (s, 3H), 3.72 (s, 3H), 5.22 (s, 2H), 6.92 (brs, 1H), 7.15 (d, J = 8.7 Hz, 2H), 7.42 (d, J = 8.7 Hz, 2H).

Step 14-2

According to Steps 7-3 and 7-4 of Example 7, demethoxymethylation and then sulfamoylation were conducted on 2-cyano-3-methoxy-3-[4-(methoxymethoxy)phenyl]-N-nonylacrylamide to thereby obtain compound 23.
APCI-MS m/z: $[M-H]^-$ 422.
$^1$H-NMR ($CDCl_3$) δ (ppm): 0.88 (t, J = 6.6 Hz, 3H), 1.18-1.45 (m, 12H), 1.62 (m, 2H), 3.42 (dt, J = 5:8, 7.1 Hz, 2H), 4.33 (s, 3H), 5.22 (s, 2H), 7.35 (d, J = 8.9 Hz, 2H), 7.81 (d, J = 8.9 Hz, 2H), 11.43 (brt, 1H).

Example 15 (Compound 24)

According to Step 7-2 of Example 7, 4-(methoxymethoxy)acetophenone was condensed with tert-butyl 2-cyanoacetate. Then, following Steps 7-3 and 7-4 of Example 7, demethoxymethylation and sulfamoylation were conducted to thereby obtain Compound 24.
APCI-MS m/z: $[M-H]^-$ 337.
$^1$H-NMR ($CDCl_3$) δ (ppm): 1.57 (s, 9H), 2.63 (s, 3H), 5.35 (brs, 2H), 7.40 (d, J = 8.7 Hz, 2H), 7.47 (d, J = 8.7 Hz, 2H).

Example 16 (Compound 25)

Step 16-1

2-Cyano-N-nonylacetamide (500 mg, 2.38 mmol) obtained in Reference Example 3 was dissolved in THF (10 mL), 2,4-bis(p-tolylthio)-1,3-dithia-2,4-diphosphetane-2,4-disulfide (634 mg, 1.45 mmol) was added to the solution, and the mixture was stirred for 1.5 hours at room temperature. 2,4-bis(p-tolylthio)-1,3-dithia-2,4-diphosphetane-2,4-disulfide (307 mg, 0.703 mmol) was further added thereto. After stirring at room temperature for 1.5 hours, the insoluble portion was separated by filtration, and filtrate was concentrated under reduced pressure. The obtained residue was purified by silica gel column chromatography (n-hexane/ethyl acetate = 4/1), and 2-cyano-N-nonylthioacetamide (498 mg, 92% yield) was obtained.
APCI-MS m/z: $[M-H]^-$ 225.
$^1$H-NMR ($CDCl_3$) δ (ppm): 0.89 (t, J = 6.4 Hz, 3H), 1.18-1.45 (m, 12H), 1.70 (m, 2H), 3.68 (dt, J = 6.3, 6.9 Hz, 2H), 3.93 (s, 2H), 7.67 (brs, 1H).

Step 16-2

According to Step 7-2 of Example 7, 4-(methoxymethoxy)acetophenone (100 mg, 0.555 mmol) was condensed with 2-cyano-N-nonylthioacetamide (153 mg, 0.676 mmol). Then, following Steps 7-3 and 7-4 of Example 7, demethoxymethylation and sulfamoylation were conducted to thereby obtain Compound 25 (E/Z = 77/23).
APCI-MS m/z: $[M-H]^-$ 422.
E form $^1$H-NMR ($CD_3OD$) δ (ppm): 1.10 (t, J = 6.3 Hz, 3H), 1.28-1.72 (m, 12H), 1.93 (m, 2H), 2.41 (s, 3H), 3.89 (t, J = 7.2 Hz, 2H), 7.60 (d, J = 8.9 Hz, 2H), 7.79 (d, J = 8.9 Hz, 2H).
Z form $^1$H-NMR ($CD_3OD$) δ (ppm): 1.09 (t, J = 6.8 Hz, 3H), 1.16 (m, 2H), 1.29-1.67 (m, 12H), 2.63 (s, 3H), 3.56 (t, J = 6.9 Hz, 2H), 7.49 (d, J = 8.9 Hz, 2H), 7.69 (d, J = 8.9 Hz, 2H).

Example 17 (Compound 26)

Step 17-1

According to Step 7-2 of Example 7, p-anisaldehyde (527 mg, 3.87 mmol) was treated with ethylmalonic acid (516 mg, 3.91 mmol), ammonium acetate (2.24 g, 29.1 mmol), and acetic acid (10.6 mL, 185 mmol) to thereby obtain (E)-2-ethyl-3-(4-methoxyphenyl)acrylic acid (78 mg, 10% yield).
FAB-MS m/z: $[M+H]^+$ 206.
$^1$H-NMR ($CDCl_3$) δ (ppm): 1.22 (t, J = 7.4 Hz, 3H), 2.60 (q, J = 7.4 Hz, 2H), 3.84 (s, 3H), 6.94 (d, J = 8.9 Hz, 2H), 7.40 (d, J = 8.9 Hz, 2H), 7.74 (s, 1H).

Step 17-2

According to Step 4-1 to Step 4-3 of Example 4, amidation and demethylation followed by sulfamoylation of (E)-2-ethyl-3-(4-methoxyphenyl)acrylic acid were conducted to thereby obtain Compound 26.
APCI-MS m/z: $[M+H]^+$ 397.
$^1$H-NMR ($CDCl_3$) δ (ppm): 0.88 (t, J = 6.7 Hz, 3H), 1.13 (t, J = 7.6 Hz, 3H), 1.18-1.68 (m, 14H), 2.50 (q, J = 7.6 Hz, 2H), 3.37 (dt, J = 5.8, 7.2 Hz, 2H), 5.05 (brs, 2H), 5.85 (brt, 1H), 7.07 (s, 1H), 7.20-7.37 (m, 4H).

Example 18 (Compound 27)

Step 18-1

According to Step 1-1 of Example 1, 4-hydroxyphenylpyruvic acid (100 mg, 0.560 mmol) was treated with EDCI (268 mg, 1.40 mmol), HOBt (86 mg, 0.56 mmol), and n-nonylamine (0.31 mL, 1.7 mmol) to thereby obtain 3-(4-hydroxyphenyl)-N-nonyl-2-oxopropionamide (47 mg, 28% yield).
APCI-MS m/z: $[M-H]^-$ 304.
$^1$H-NMR ($CDCl_3$) δ (ppm): 0.87 (t, J = 6.6 Hz, 3H), 1.25 (m, 12H), 1.52 (m, 2H), 3.28 (m, 2H), 4.12 (s, 2H), 6.43 (brs, 1H), 6.77 (d, J = 8.4 Hz, 2H), 7.04 (m, 1H), 7.08 (d, J = 8.4 Hz, 2H).

Step 18-2

3-(4-Hydroxyphenyl)-N-nonyl-2-oxopropionamide (121 mg, 0.396 mmol) was dissolved in methanol (2 mL). Trimethoxymethane (0.860 mL, 7.86 mmol) and camphor sulfonic acid (10 mg, 0.043 mmol) were added to the solution. While heating under reflux, the mixture was stirred for 7 hours. After cooling the reaction solution, the reaction solution was added by saturated aqueous sodium hydrogencarbonate solution, and then extracted with ethyl acetate. After the organic layer was washed with saturated brine, the organic layer was dried over anhydrous sodium sulfate and concentrated under reduced pressure. The obtained residue was dissolved in toluene (3 mL), and camphor sulfonic acid (11 mg, 0.047 mmol) was added to the solution. While heating under reflux, the mixture was stirred for 1.5 hours. After cooling the reaction solution, the reaction solution was added by saturated aqueous sodium hydrogencarbonate solution, and then extracted with ethyl acetate. After the organic layer was washed with saturated brine, the organic layer was dried over anhydrous sodium sulfate and concentrated under reduced pressure. The obtained residue was purified by preparative thin layer chromatography (n-hexane/ethyl acetate = 2/1), and 3-(4-hydroxyphenyl)-2-methoxy-N-nonylacrylamide (27 mg, 21% yield) was obtained.
APCI-MS m/z: $[M-H]^-$ 318.
$^1$H-NMR ($CDCl_3$) δ (ppm): 0.87 (t, J = 6.8 Hz, 3H), 1.14-1.46 (m, 12H), 1.58 (m, 2H), 3.38 (dt, J = 6.6, 6.9 Hz, 2H), 3.62 (s, 3H), 6.73 (brt, 1H), 6.88 (d, J = 8.6 Hz, 2H), 6.93 (s, 1H), 7.02 (s, 1H), 7.53 (d, J = 8.6 Hz, 2H).

Step 18-3

According to Step 1-2 of Example 1, 3-(4-hydroxyphenyl)-2-methoxy-N-nonylacrylamide (27 mg, 0.085 mmol) was treated with sulfamoyl chloride (40 mg, 0.35 mmol) to thereby obtain Compound 27 (24 mg, 71% yield).
APCI-MS m/z: $[M-H]^-$ 397.
$^1$H-NMR (DMSO-$d_6$) δ (ppm): 0.85 (t, J = 6.6 Hz, 3H), 1.11-1.34 (m, 12H), 1.47 (m, 2H), 3.17 (dt, J = 5.8, 6.4 Hz, 2H), 3.60 (s, 3H), 6.65 (s, 1H), 7.2 7 (d, J = 8.7 Hz, 2H), 7.74 (d, J = 8.7 Hz, 2H), 8.01 (brs, 2H), 8.24 (brt, 1H).

Example 19 (Compound 28)

Step 19-1

According to Step 7-1 of Example 7, p-hydroxybenzaldehyde (2.00 g, 16.4 mmol) was treated with sodium hydride (0.72 g, 18 mmol, 60% in oil) and chloromethyl methylether (1.87 mL, 24.6 mmol) to thereby obtain 4-(methoxymethoxy)benzaldehyde (2.53 g, 93% yield).
FAB-MS m/z: $[M+H]^+$ 167.
$^1$H-NMR ($CDCl_3$) δ (ppm): 3.49 (s, 3H), 5.26 (s, 2H), 7.15 (d, J = 8.8 Hz, 2H), 7.84 (d, J = 8.8 Hz, 2H), 9.90 (s, 1H).

Step 19-2

While cooling on ice, sodium hydride (19 mg, 0.079 mmol, 60% in oil) and methanol (0.016 mL, 0.40 mmol) dissolved in THF (0.2 mL) were added to THF (0.5 mL). After stirring at room temperature for 30 minutes, 4-(methoxymethoxy)benzaldehyde (40 mg, 0.24 mmol) dissolved in THF (0.5 mL), and methyl acrylate (0.030 mL, 0.33 mmol) were added thereto at the same temperature, and the mixture was stirred at room temperature for 1 hour. Water (1 mL) was added to the reaction solution. After evaporating THF under reduced pressure, 15% aqueous sodium hydroxide solution (4 mL) was added, and the mixture was heated under reflux for 30 minutes. After cooling the reaction solution, the aqueous layer was washed with toluene, and 2 mol/L hydrochloric acid was added thereto to adjust the pH to approximately 3. After the reaction solution was extracted with chloroform, the organic layer was washed with saturated brine, dried over anhydrous sodium sulfate and concentrated under reduced pressure. The obtained residue was purified by preparative thin layer chromatography (chloroform/methanol = 9/1), and (E)-3-[4-(methoxymethoxy) phenyl]-2-(methoxymethyl)acrylic acid (28 mg, 47% yield) was obtained.
APCI-MS m/z: $[M-H]^-$ 251.
$^1$H-NMR ($CDCl_3$) δ (ppm): 3.46 (s, 3H), 3.49 (s, 3H), 4.29 (s, 2H), 5.22 (s, 2H), 7.08 (d, J = 8.6 Hz, 2H), 7.53 (d, J = 8.6 Hz, 2H), 8.02 (s, 1H).

Step 19-3

According to Step 1-1 of Example 1, (E)-3-[4-(methoxymethoxy)phenyl]-2-(methoxymethy])acrylic acid was condensed with n-nonylamine. Then, according to Steps 7-3 and 7-4 of Example 7, demethoxymethylation and sulfamoylation were conducted to thereby obtain Compound 28.
APCI-MS m/z: $[M-H]^-$ 411.
$^1$H-NMR ($CDCl_3$) δ (ppm): 0.88 (t, J = 6.8 Hz, 3H), 1.10-1.42 (m, 12H), 1.56 (m, 2H), 3.33 (m, 2H), 3.36 (s, 3H), 4.20 (s, 2H), 5.64 (brs, 2H), 7.03 (brt, 1H), 7.21-7.37 (m, 4H), 7.64 (s, 1H).

Example 20 (Compound 29)

Step 20-1

According to Step 2-1 of Example 2, 4-benzyloxy-2-methylbenzaldehyde was treated with malonic acid and piperidine. Then, according to Step 1-1 of Example 1, the obtained product was condensed with n-nonylamine to thereby obtain (E)-3-(4-benzyloxy-2-methylphenyl)-N-nonylacrylamide (37 mg, 77% yield).
APCI-MS m/z: $[M+H]^+$ 394.
$^1$H-NMR ($CDCl_3$) δ (ppm): 0.88 (t, J = 6.6 Hz, 3H), 1.26 (m, 12H), 1.55 (m, 2H), 2.39 (s, 3H), 3.36 (m, 2H), 5.04 (s, 2H), 5.81 (brs, 1H), 6.21 (d, J = 15.4 Hz, 1H), 6.75 (dd, J = 2.6, 8.6 Hz, 1H), 6.79 (d, J = 2.5 Hz, 1H), 7.29-7.39 (m, 5H), 7.44 (d, J = 8.6 Hz, 1H), 7.84 (d, J = 15.4 Hz, 1H).

Step 20-2

(E)-3-(4-Benzyloxy-2-methylphenyl)-N-nonylacrylamide (33 mg, 0.084 mmol) was dissolved in dichloromethane (1 mL). Dimethylsulfide (0.373 mL, 5.05 mmol) and boron trifluoride-diethyl ether complex (0.160 mL, 1.26 mmol) were added to the solution, and the mixture was stirred at room temperature for 16 hours. The reaction solution was added by water, and then extracted with ethyl acetate. After the organic layer was washed with saturated brine, the organic layer was dried over anhydrous sodium sulfate and concentrated under reduced pressure. The obtained residue was purified by preparative thin layer chromatography (chloroform/methanol = 20/1), and (E)-3-(4-hydroxy-2-methylphenyl)-N-nonylacrylamide (13 mg, 50% yield) was obtained.
APCI-MS m/z: $[M-H]^-$ 302.
$^1$H-NMR ($CDCl_3$) δ (ppm): 0.87 (t, J = 6.6 Hz, 3H), 1.25 (m, 12H), 1.56 (m, 2H), 2.22 (s, 3H), 3.38 (m, 2H), 5.88 (m, 1H), 6.18 (d, J = 15.5 Hz, 1H), 6.67 (brd, J = 7.6 Hz, 1H), 6.69 (brs, 1H), 7.35 (d, J = 8.6 Hz, 1H), 7.80 (brs, 1H), 7.83 (d, J = 15.5 Hz, 1H).

Step 20-3

According to Step 1-2 of Example 1, (E)-3-(4-hydroxy-2-methylphenyl)-N-nonylacrylamide (10 mg, 0.034 mmol) was treated with sulfamoyl chloride (7.8 mg, 0.068 mmol) to thereby obtain Compound 29 (11 mg, 83% yield).
APCI-MS m/z: $[M-H]^-$ 381.
$^1$H-NMR (DMSO-$d_6$) δ (ppm): 0.86 (t, J = 6.4 Hz, 3H), 1.26 (m, 12H), 1.45 (m, 2H), 2.38 (s, 3H), 3.16 (m, 2H), 6.51 (d, J = 15.5 Hz, 1H), 7.15 (m, 1H), 7.16 (s, 1H), 7.58 (m, 1H), 7.59 (d, J = 15.5 Hz, 1H), 8.02 (brs, 2H), 8.12 (t, J = 5.3 Hz, 1H).

Example 21 (Compound 30 and Compound 31)

Step 21-1

2-(4-Methoxybenzylidene)succinic acid (1.002 g, 4.240 mmol) was dissolved in DMF (10 mL). While cooling on ice, an ammonia addition product of HOBt (0.774 g, 5.09 mmol) and EDCI (0.788 g, 5.98 mmol) were added to the solution, and the mixture was stirred at room temperature for 23 hours. Water was added to the reaction solution, and the reaction solution was extracted with ethyl acetate. The organic layer was washed with 1 mol/L hydrochloric acid and then with saturated brine, dried over anhydrous sodium sulfate and concentrated under reduced pressure. The obtained residue was triturated with chloroform, and crude (E)-2-carbamoyl methyl-3-(4-methoxyphenyl) acrylic acid (0.543 g) was obtained.

Step 21-2

According to Step 1-1 of Example 1, crude (E)-2-carbamoyl methyl-3-(4-methoxyphenyl) acrylic acid was condensed with n-nonylamine. Next, following Step 4-2 of Example 4, methyl removal was conducted to thereby obtain (E)-2-carbamoyl methyl-3-(4-hydroxyphenyl)-N-nonylacrylamide.
APCI-MS m/z: $[M-H]^-$ 345.
$^1$H-NMR ($CD_3OD$) δ (ppm): 0.89 (t, J = 6.7 Hz, 3H), 1.23-1.42 (m, 12H), 1.57 (m, 2H), 3.26 (t, J = 7.0 Hz, 2H), 3.43 (s, 2H), 6.80 (d, J = 8.6 Hz, 2H), 7.27 (s, 1H), 7.29 (d, J = 8.6 Hz, 2H).

Step 21-3

According to Step 1-2 of Example 1, (E)-2-carbamoylmethyl -3-(4-hydroxyphenyl)-N-nonylacrylamide (23 mg, 0.065 mmol) was treated with sulfamoyl chloride (15 mg, 0.13 mmol) to thereby obtain Compound 30 (8.2 mg, 30% yield) and Compound 31 (5.3 mg, 20% yield).

Compound 30

APCI-MS m/z: $[M-H]^-$ 424.
$^1$H-NMR (DMSO-$d_6$) δ (ppm): 0.85 (t, J = 6.8 Hz, 3H), 1.12-1.37 (m, 12H), 1.46 (m, 2H), 3.13 (dt, J = 5.4, 6.2 Hz, 2H), 3.24 (s, 2H), 6.94 (brs, 1H), 7.25 (s, 1H), 7.29 (d, J = 8.6 Hz, 2H), 7.31 (brs, 1H), 7.51 (d, J = 8.6 Hz, 2H), 8.03 (s, 2H), 8.14 (brt, J = 5.4 Hz, 1H).

Compound 31

APCI-MS m/z: $[M-H]^-$ 406.
$^1$H-NMR ($CDCl_3$) δ (ppm): 0.88 (t, J = 6.7 Hz, 3H), 1.01-1.47 (m, 12H), 1.61 (m, 2H), 3.38 (dt, J = 5.6, 7.1 Hz, 2H), 3.52 (s, 2H), 5.26 (s, 2H), 6.10 (brt, J = 5.6 Hz, 1H), 7.24 (s, 1H), 7.38 (m, 4H).

Example 22 (Compound 32)

Step 22-1

Diketene (0.46 mL, 6.0 mmol) was dissolved in THF (6 mL). To the solution, n-nonylamine (1.00 mL, 5.52 mmol) dissolved in THF (20 mL) was added over 5 minutes. The mixture was stirred for 2.5 hours at room temperature. Water was added to the reaction solution, and the reaction solution was extractied with ethyl acetate. The organic layer was washed with 0.5 mol/L hydrochloric acid and then with saturated brine, dried over anhydrous sodium sulfate and concentrated under reduced pressure. The obtained residue was triturated with diisopropyl ether (10 mL), and N-nonyl-3-oxobutylamide (982 mg, 78% yield) was obtained.
APCI-MS m/z: $[M+H]^+$ 228.
$^1$H-NMR ($CDCl_3$) δ (ppm): 0.88 (t, J = 7.1 Hz, 3H), 1.17-1.43 (m, 12H), 1.52 (m, 2H), 2.27 (s, 3H), 3.26 (q, J = 6.6 Hz, 2H), 3.41 (s, 2H), 6.92 (brs, 1H).

Step 22-2

4-(Methoxymethoxy)benzaldehyde (70 mg, 0.42 mol) was dissolved in toluene (2 mL). N-nonyl-3-oxobutylamide (190 mg, 0.836 mmol), piperidine (0.167 mL, 1.69 mmol) and acetic acid (0.145 mL, 2.53 mmol) were added thereto, and the mixture was stirred for 8.5 hours at room temperature. Water was added to the reaction solution, and the reaction solution was extracted with ethyl acetate. The organic layer was washed with 0.5 mol/L hydrochloric acid, saturated aqueous sodium hydrogencarbonate solution, and then with saturated brine, dried over anhydrous sodium sulfate and concentrated under reduced pressure. The obtained residue was purified by silica gel column chromatography (n-hexane/ethyl acetate = 4/1) followed by preparative thin layer chromatography (n-hexane/ethyl acetate = 4/1), and (Z)-2-acetyl-3-[4-(methoxymethoxy) phenyl]-N-nonylacrylamide (101 mg, 64% yield) was obtained.
APCI-MS m/z: $[M+H]^+$ 376.
$^1$H-NMR ($CDCl_3$) δ (ppm): 0.88 (t, J = 6.7 Hz, 3H), 1.11-1.35 (m, 12H), 1.52 (m, 2H), 2.40 (s, 3H), 3.36 (m, 2H), 3.47 (s, 3H), 5.19 (s, 2H), 5.83 (brt, J = 5.1 Hz, 1H), 7.02 (d, J = 8.8 Hz, 2H), 7.47 (s, 1H), 7.50 (d, J = 8.8 Hz, 2H).

Step 22-3

According to Steps 7-3 and 7-4 of Example 7, demethoxymethylation followed by sulfamolylation of (Z)-2-acetyl-3-[4-(methoxymethoxy)phenyl]-N-nonylacrylamide was conducted to thereby obtain Compound 32.
FAB-MS m/z: $[M+H]^+$ 411.
$^1$H-NMR (DMSO-$d_6$) δ (ppm): 0.85 (t, J = 6.7 Hz, 3H), 1.11-1.35 (m, 12H),1.42 (m, 2H), 2.34 (s, 3H), 3.14 (dt, J = 5.9, 6.8 Hz, 2H), 7.30 (d, J = 8.7 Hz, 2H), 7.52 (s, 1H), 7.68 (d, J = 8.7 Hz, 2H), 8.10 (brs, 2H), 8.34 (t, J = 5.9 Hz, 1H).

Example 23 (Compound 33)

Compound 32 (21 mg, 0.052 mmol) was dissolved in acetonitrile (2 mL). Using a UV lamp, the solution was exposed to UV (254 nm) for 2.5 hours. The reaction solution was concentrated under reduced pressure; and the obtained residue was purified by preparative thin layer chromatography (n-hexane/ethyl acetate = 1/1), and Compound 33 (8.6 mg, 40% yield) was obtained.
ESI-MS m/z: $[M-H]^-$ 409.
$^1$H-NMR ($CD_3OD$) δ (ppm): 0.80 (t, J = 6.8 Hz, 3H), 1.12-1.33 (m, 12H), 1.47 (m, 2H), 2.13 (s, 3H), 3.21 (m, 2H), 7.25 (d, J = 8.8 Hz, 2H), 7.33 (d, J = 8.8 Hz, 2H), 7.42 (s, 1H).

Example 24 (Compound 34)

Step 24-1

Methyl methane sulfonyl acetate (1.005 g, 6.604 mmol) was dissovlved in toluene (15 mL). To the solution, n-nonylamine (2.42 mL, 13.2 mmol) and DMAP (397 mg, 3,25 mmol) were added. While heating under reflux, the

mixture was stirred for 6.5 hours. After cooling the reaction solution, water was added, and the reaction solution was extracted with ethyl acetate. The organic layer was washed with 1 mol/L hydrochloric acid and then with saturated brine, dried over anhydrous sodium sulfate and concentrated under reduced pressure. The obtained residue was triturated with diisopropyl ether (10 mL), and 2-methane sulfonyl-N-nonylacetamide (762 mg, 44% yield) was obtained.
APCI-MS m/z: $[M-H]^-$ 262.
$^1$H-NMR ($CDCl_3$) δ (ppm): 0.88 (t, J = 6.6 Hz, 3H), 1.17-1.47 (m, 12H), 1.54 (m, 2H), 3.08 (s, 3H), 3.31 (dt, J = 5.9, 7.1 Hz, 2H), 3.87 (s, 2H), 6.45 (brs, 1H).

Step 24-2

According to Step 7-2 of Example 7, 4-(methoxymethoxy)benzaldehyde was condensed with 2-methane sulfonyl-N-nonylacetamide. Next, according to Steps 7-3 and 7-4 of Example 7, methoxymethyl removal and sulfamoylation were conducted to thereby obtain Compound 34.
FAB-MS m/z: $[M+H]^+$ 447.
$^1$H-NMR (DMSO-$d_6$) δ (ppm): 0.85 (t, J = 6.5 Hz, 3H), 1.10-1.32 (m, 12H), 1.40 (m, 2H), 3.14 (dt, J = 6.1, 6.6 Hz, 2H), 3.18 (s, 3H), 7.33 (d, J = 8.8 Hz, 2H), 7.48 (s, 1H), 7.64 (d, J = 8.8 Hz, 2H), 8.14 (brs, 2H), 8.75 (t, J = 5.5 Hz, 1H).

Example 25 (Compound 35)

Step 25-1

n-Nonylamine (0.300 mL, 1.63 mmol) and pyridine (0.660 mL, 8.24 mmol) were dissolved in dichloromethane (10 mL). At -78 °C, methyl malonyl chloride (0.210 mL, 1.96 mmol) dissolved in dichloromethane (10 mL) was added thereto. While raising the temperature to 0 °C, the mixture was stirred for 2 hours. Water and 1 mol/L hydrochloric acid were added to the reaction solution, and the reaction solution was extracted with chloroform. The organic layer was washed with 0.5 mol/L hydrochloric acid and saturated aqueous sodium hydrogencarbonate solution, followed by saturated brine Next, the organic layer was dried over anhydrous sodium sulfate and concentrated under reduced pressure. The obtained residue was purified by silica gel column chromatography (n·hexane/ethyl acetate = 1/3), and methyl N-nonyl malonamide (188 mg, 47% yield) was obtained.
APCI-MS m/z: $[M-H]^-$ 242.
$^1$H-NMR ($CDCl_3$) δ (ppm): 0.88 (t, J = 6.1 Hz, 3H), 1.16-1.40 (m, 12H), 1.52 (m, 2H), 3.26 (dt, J = 5.9, 7.3 Hz, 2H), 3.31 (s, 2H), 3.75 (s, 3H), 7.08 (brs, 1H).

Step 25-2

4-(methoxymethoxy)benzaldehyde (26 mg, 0.16 mol) was dissolved in toluene (1 mL). Methyl N-nonyl malonamide (188 mg, 0.773 mmol), piperidine (0.068 mL, 0.69 mmol) and acetic acid (0.059 mL, 1.0 mmol) were added thereto, and the mixture was stirred for 1 hour at 40 °C followed by 0.5 hours at 90 °C. Water was added to the reaction solution, and the reaction solution was extracted with ethyl acetate. The organic layer was washed with 1 mol/L hydrochloric acid and saturated aqueous sodium hydrogencarbonate solution, followed by saturated brine. Next, the organic layer was dried over anhydrous sodium sulfate and concentrated under reduced pressure. The obtained residue was purified by preparative thin layer chromatography (chloroform/acetone = 9/1), and methyl 3-[4-(methoxymethoxy) phenyl]-2-(nonylcarbamoyl)acrylate (23 mg, 38% yield) was obtained.
APCI-MS m/z: $[M+H]^+$ 392.
$^1$H-NMR ($CDCl_3$) δ (ppm): 0.88 (t, J = 6.6 Hz, 3H), 1.10-1.67 (m, 14H), 3.37 (dt, J = 5.9, 7.1 Hz, 2H), 3.47 (s, 3H), 3.82 (s, 3H), 5.19 (s, 2H), 5.75 (brt, 1H), 7.01 (d, J = 9.0 Hz, 2H), 7.51 (d, J = 9.0 Hz, 2H), 7.66 (s, 1H).

Step 25-3

According to Steps 7-3 and 7-4 of Example 7, demethoxymethylation followed by sulfamoylation of methyl 3-[4-(methoxymethoxy) phenyl-2-(nonylcarbamoyl) acrylate were conducted to thereby obtain Compound 35.
$^1$H-NMR (DMSO-$d_6$) δ (ppm): 0.85 (t, J = 6.1 Hz, 3H), 1.15-1.35 (m, 12H), 1.41 (m, 2H), 3.14 (dt, J = 5.6, 6.3 Hz, 2H), 3.73 (s, 3H), 7.29 (d, J = 8.9 Hz, 2H), 7.56 (s, 1H), 7.67 (d, J = 8.9 Hz, 2H), 8.10 (s, 2H), 8.42 (t, J = 5.6 Hz, 1H).

Example 26 (Compound 36)

Step 26-1

4-(Methoxymethoxy)benzaldehyde (512 mg, 3.08 mmol) was dissolved in THF (15 mL). Diethylphosphonoacetate (781 mg, 3.98 mmol) and sodium hydride (321 mg, 8.0 mmol, 60% in oil) were added thereto. While heating under reflux, the mixture was stirred for 4 hours. Ice and 1 mol/L hydrochloric acid were added to the reaction solution, and the reaction solution was extracted with ethyl acetate. After the organic layer was washed with saturated brine, the organic layer was dried over anhydrous sodium sulfate and concentrated under reduced pressure. The obtained residue was triturated with a mixed solvent of ethyl acetate and n-hexane, and (E)-3-[4-(methoxymethoxy)phenyl] acrylic acid (515 mg, 80% yield) was obtained.
FAB-MS m/z: $[M+H]^+$ 209.

Step 26-2

According to Step 1-1 of Example 1, (E)-3-[4-(methoxymethoxy)phenyl]acrylic acid (201 mg, 0.904 mmol) was treated with EDCI (211 mg, 1.10 mmol), HOBt (167 mg, 1.09 mmol) and n-nonylamine (0.200 mL, 1.09 mmol) to thereby obtain (E)-3-[4-(methoxymethoxy) phenyl]-N-nonylacrylamide (295 mg, 94% yield).
APCI-MS m/z: $[M+H]^+$ 334.
$^1$H-NMR ($CDCl_3$) $\delta$ (ppm): 0.88 (t, J = 6.7 Hz, 3H), 1.16-1.41 (m, 12H), 1.56 (m, 2H), 3.37 (q, J = 7.0 Hz, 2H), 3.48 (s, 3H), 5.19 (s, 2H), 5.55 (brt, 1H), 6.26 (d, J = 15.6 Hz, 1H), 7.02 (d; J = 8.8 Hz, 2H), 7.44 (d, J = 8.8 Hz, 2H), 7.58 (d, J = 15.6 Hz, 1H).

Step 26-3

Under an argon atmosphere, (E)-3-[4-(methoxymethoxy) phenyl]-N-nonylacrylamide (104 mg, 0.313 mmol) and sodium azide (30 mg, 0.47 mmol) were suspended in degased acetonitrile (4 mL). While cooling on ice, diammonium cerium (IV) nitrate (CAN) (431 mg, 0.786 mmol) dissolved in degased acetronitrile (6 mL) was added to the suspension, and the mixture was stirred for 2 hours at room temperature. Water was added to the reaction solution, and the reaction solution was extracted with chloroform. After the organic layer was washed with saturated brine, the organic layer was dried over anhydrous sodium sulfate and concentrated under reduced pressure. The obtained residue was dissolved in THF (3 mL), and sodium hydride (38 mg, 0.95 mmol, 60% in oil) was added thereto. The mixture was stirred at room temperature for 1.5 hours. Water was added to the reaction solution, and the reaction solution was extracted with ethyl acetate. After the organic layer was washed with saturated brine, the organic layer was dried over anhydrous sodium sulfate and concentrated under reduced pressure. The obtained residue was purified by silica gel column chromatography (n-hexane/ethyl acetate = 4/1), and (Z)-2-azido-3-[4-(methoxymethoxy) phenyl]-N-nonylacrylamide (86 mg, 74% yield) was obtained.
$^1$H-NMR ($CDCl_3$) $\delta$ (ppm): 0.88 (t, J = 6.7 Hz, 3H), 1.18-1.42 (m, 12H), 1.59 (m, 2H), 3.37 (dt, J = 6.0, 7.1 Hz, 2H), 3.48 (s, 3H), 5.20 (s, 2H), 6.26 (brt, 1H), 6.70 (s, 1H), 7.05 (d, J = 8.9 Hz, 2H), 7.57 (d, J = 8.9 Hz, 2H).

Step 26-4

According to Steps 7-3 and 7-4 of Example 7, demethoxymethylation followed by sulfamoylation of (Z)-2-azido-3-[4-(methoxymethoxy) phenyl]-N-nonylacrylamide were conducted to thereby obtain Compound 36.
ESI-MS m/z: $[M-H]^-$ 408.
$^1$H-NMR (DMSO-$d_6$) $\delta$ (ppm): 0.85 (t, J = 6.6 Hz, 3H), 1.10-1.36 (m, 12H), 1.49 (m, 2H), 3.18 (dt, J = 5.7, 6.5 Hz, 2H), 6.51 (s, 1H), 7.29 (d, J = 8.8 Hz, 2H), 7.81 (d, J = 8.8 Hz, 2H), 8.05 (s, 2H), 8.57 (brt, J = 5.7 Hz, 1H).

Example 27 (Compound 37 and Compound 60)

According to Step 1-2 of Example 1, 3-(4-hydroxyphenyl)-N-nonyl-2-oxopropionamide (150 mg, 0.491 mmol) obtained in Step 18-1 of Example 18 was treated with sulfamoyl chloride (113 mg, 0.978 mmol) to thereby obtain Compound 37 (15 mg, 7% yield) and Compound 60 (128 mg, 68% yield).

Compound 37

APCI-MS m/z: $[M-H]^-$ 462.
$^1$H-NMR (DMSO-$d_6$) $\delta$ (ppm): 0.85 (t, J = 6.6 Hz, 3H), 1.09-1.38 (m, 12H), 1.47 (m, 2H), 3.17 (m, 2H), 7.12 (s, 1H),

7.29 (d, J = 8.8 Hz, 2H), 7.83 (d, J = 8.8 Hz, 2H), 7.96 (m, 1H), 8.07 (brs, 4H).

Compound 60

FAB-MS m/z: $[M+H]^+$ 385.
$^1$H-NMR (DMSO-$d_6$) δ (ppm): 0.86 (t, J = 6.6 Hz, 3H), 1.24 (m, 12H), 1.44 (m, 2H), 3.11 (m, 2H), 4.20 (s, 1.72H), 7.21 (d, J = 8.6 Hz, 2H), 7.29 (d, J = 8.6 Hz, 2H), 7.71 (s, 0.07H), 7.74 (s, 0.07H), 7.98 (brs, 2H), 8.40 (m, 0.14H), 8.62 (t, J = 5.6 Hz, 0.86H).

Example 28 (Compound 38)

Step 28-1

According to Step 1-1 of Example 1, (E)-2-(4-methoxybenzylidene) succinic acid (503 mg, 2.13 mmol) was treated with 1-(3-dimethylaminopropyl)-3-ethylcarbodiimide (0.408 mL, 2.13 mmol), HOBt (328 mg, 2.14 mmol), and dimethylamine hydrochloride (174 mg, 2.13 mmol) to thereby obtain (E)-3-(4-methoxyphenyl)-2-[(dimethylcarbamoyl) methyl] acrylic acid (190 mg, 34 % yield).
APCI-MS m/z: $[M-H]^-$ 262.
$^1$H-NMR ($CDCl_3$) δ (ppm): 3.02 (s, 3H), 3.06 (s, 3H), 3.54 (s, 2H), 3.82 (s, 3H), 6.90 (d, J = 8.8 Hz, 2H), 7.31 (d, J = 8.8 Hz, 2H), 7.89 (s, 1H).

Step 28-2

According to Step 1-1 of Example 1, (E)-3-(4-methoxyphenyl)-2-[(dimethylcarbamoyl)methyl] acrylic acid was condensed with n-nonylamine. Then, according to Steps 4-2 and 4-3 of Example 4, de-methylation and sulfamoylation were conducted to thereby obtain Compound 38.
APCI-MS m/z: $[M-H]^-$ 452.
$^1$H-NMR (DMSO-$d_6$) δ (ppm): 0.85 (t, J = 6.6 Hz, 3H), 1.10-1.60 (m, 14H), 2.83 (s, 3H), 2.99 (s, 3H), 3.10 (m, 2H), 3.44 (s, 2H), 7.26 (s, 1H), 7.28 (d, J = 8.7 Hz, 2H), 7.40 (d, J = 8.7 Hz, 2H), 8.02 (brs, 2H), 8.09 (brt, 1H).

Example 29 (Compound 39)

According to Steps 28-1 and 28-2 of Example 28, (E)-2-(4-methoxybenzylidene)succinic acid was condensed with n-nonylamine and then condensed with dimethylamine hydrochloride. Next, demethylation and sulfamoylation were conducted to thereby obtain Compound 39.
APCI-MS m/z: $[M+H]^+$ 389.
$^1$H-NMR ($CDCl_3$) δ (ppm): 0.87 (t, J = 6.7 Hz, 3H), 1.11-1.37 (m, 12H), 1.48 (m, 2H), 3.10 (brs, 6H), 3.23 (dt, J = 6.0, 6.9 Hz, 2H), 3.32 (s, 2H), 3.81 (s, 3H), 6.59 (s, 1H), 6.92 (d, J = 8.9 Hz, 2H), 7.37 (d, J = 8.9 Hz, 2H), 7.63 (brt, 1H).

Example 30 (Compound 40)

Step 30-1

4-(Methoxymethoxy)benzaldehyde (239 mg, 1.44 mmol) and triethyl 2-fluoro-2-phosphonoacetate (349 mg, 1.44 mmol) were dissolved in THF (5 mL). Sodium hydride (52 mg, 2.2 mmol, 60% in oil) was added thereto, and the mixture was stirred at room temperature for 40 minutes. Water was added to the reaction solution, and the reaction solution was extracted with ethyl acetate. After the organic layer was washed with saturated brine, the organic layer was dried over anhydrous sodium sulfate. The obtained organic layer was concentrated under reduced pressure, and ethyl 2-fluoro-3-[4-(methoxymethoxy)phenyl]acrylate (316 mg, 86% yield, E/Z = 69/31) was obtained.
FAF-MS m/z: $[M+H]^+$ 255.
E-form $^1$H-NMR ($CDCl_3$) δ (ppm): 1.29 (t, J = 7.2 Hz, 3H), 3.47 (s, 3H), 4.28 (q, J = 7.2 Hz, 2H), 5.19 (s, 2H), 6.85 (d, J = 23.4 Hz, 1H), 7.01 (d, J = 8.9 Hz, 2H), 7.49 (d, J = 8.9 Hz, 2H).

Step 30-2

Ethyl 2-fluoro-3-[4-(methoxymethoxy)phenyl]acrylate (E/Z = 69/31) was treated according to Steps 10-3 to 10-5 of Example 10, and Compound 40 was obtained.
APCI-MS m/z: $[M-H]^-$ 385.

$^1$H-NMR (DMSO-$d_6$) δ (ppm): 0.84 (t, J = 6.9 Hz, 3H), 1.16-1.33 (m, 12H), 1.47 (m, 2H), 3.32 (dt, J = 5.8, 6.9 Hz, 2H), 6.87 (d, J = 38.6 Hz, 1H), 7.32 (d, J = 8.6 Hz, 2H), 7.72 (d, J = 8.6 Hz, 2H), 8.05 (brs, 2H), 8.59 (brt, 1H).

Example 31 (Compound 41)

Step 31-1

(Tert-butoxycarbonylmethylene)triphenylphosphorane (453 mg, 1.20 mmol) was dissolved in dichloromethane (5 mL). NCS (176 mg, 1.32 mmol) was added to the solution at -18 °C. After stirring for 0.5 hours, potassium carbonate (415 mg, 3.01 mmol) and 4-(methoxymethoxy)benzaldehyde (200 mg, 1.20 mmol) were added thereto at the same temperature. After stirring for 15.5 hours at room temperature, the insoluble portion was separated by filtration, and concentrated under reduced pressure. The obtained residue was purified by silica gel column chromatography (n-hexane/ethyl acetate = 20/1). Tert-butyl (Z)-2-chloro-3-(4-(methoxymethoxy)phenyl] acrylate (235 mg, 66% yield) was obtained.
$^1$H-NMR ($CDCl_3$) δ (ppm): 1.57 (s, 9H), 3.49 (s, 3H), 5.21 (s, 2H), 7.07 (d, J = 8.9 Hz, 2H), 7.75 (s, 1H), 7.81 (d, J = 8.9 Hz, 2H).

Step 31-2

Tert-butyl (Z)-2-chloro-3-(4-(methoxymethoxy)phenyl] acrylate (210 mg, 0.701 mmol) was dissolved in dichloromethane (5 mL). Trifluoroacetate (5 mL) was added thereto, and the mixture was stirred at room temperature for 1 hour. After the reaction solution was concentrated under reduced pressure, the reaction solution was triturated with chloroform, and (Z)-2-chloro-3-(4-hydroxyphenyl) acrylic acid (121 mg, 87% yield) was obtained.
APCI-MS m/z: $[M-H]^-$ 197.
$^1$H-NMR (DMSO-$d_6$) δ (ppm): 6.85 (d, J = 8.7 Hz, 2H), 7.80 (d, J = 8.7 Hz, 2H), 7.82 (s, 1H), 10.17 (brs, 1H).

Step 31-3

According to Steps 1-1 and 1-2 of Example 1, (Z)-2-chloro-3-(4-hydroxyphenyl) acrylic acid was amidated and then sulfamoylated to thereby obtain Compound 41.
FAB-MS m/z: $[M+H]^+$ 403.
$^1$H-NMR (DMSO-$d_6$) δ (ppm): 0.84 (t, J = 6.6 Hz, 3H), 1.13-1.40 (m, 12H), 1.48 (m, 2H), 3.18 (dt, J = 5.7, 6.3 Hz, 2H), 7.34 (d, J = 8.8 Hz, 2H), 7.75 (s, 1H), 7.87 (d, J = 8.8 Hz, 2H), 8.09 (s, 2H), 8.44 (t, J = 5.7 Hz, 1H).

Example 32 (Compound 42)

According to Step 31-1 of Example 31, (tert-butoxycarbonylmethylene)triphenyl phosphorane was treated with NBS. In the presence of potassium carbonate, the treated product was reacted with 4-(methoxymethoxy)benzaldehyde. Then, the resulting tert-butyl (Z)-2-bromo-3-[4-(methoxymethoxy)phenyl]acrylate was treated according to Steps 31-2 and 31-3 of Example 31 to thereby obtain Compound 42.
APCI-MS m/z: $[M-H]^-$ 445, 447.
$^1$H-NMR (DMSO-$d_6$) δ (ppm): 0.84 (t, J = 6.6 Hz, 3H), 1.13-1.35 (m, 12H), 1.47 (m, 2H), 3.17 (dt, J = 5.5, 6.6 Hz, 2H), 7.34 (d, J = 8.8 Hz, 2H), 7.84 (d, J = 8.8 Hz, 2H), 7.91 (s, 1H), 8.09 (s, 2H), 8.35 (t, J = 5.5 Hz, 1H).

Example 33 (Compound 43)

According to Step 31-1 of Example 31, (tert-butoxycarbonylmethylene)triphenylphosphorane was treated with NIS. In the presence of potassium carbonate, the treaded product was reacted with 4-(methoxymethoxy)benzaldehyde. Then, the resulting tert-butyl (Z)-2-iodo-3-[4-(methoxymethoxy)phenyl]acrylate was treated according to Steps 31-2 and 31-3 of Example 31 to thereby obtain Compound 43.
APCI-MS m/z: $[M-H]^-$ 493.
$^1$H-NMR ($CDCl_3$) δ (ppm): 0.88 (t, J = 6.9 Hz, 3H), 1.16-1.47 (m, 12H), 1.59 (m, 2H), 3.37 (dt, J = 5.6, 7.3 Hz, 2H), 5.17 (s, 2H), 6.67 (brt, 1H), 7.37 (d, J = 8.6 Hz, 2H), 7.65 (d, J = 8.6 Hz, 2H), 8.20 (s, 1H).

Example 34 (Compound 44)

4-(Methoxymethoxy)acetophenone was treated according to Steps 30-1 and 30-2 of Example 30, and Compound 44 was obtained.

APCI-MS m/z: $[M-H]^-$ 399.
$^1$H-NMR ($CDCl_3$) δ (ppm): 0.88 (t, J = 6.8 Hz, 3H), 1.14-1.41 (m, 12H), 1.56 (m, 2H), 2.50 (d, J = 3.3 Hz, 3H), 3.35 (dt, J = 6.6, 6.8 Hz, 2H), 4.94 (s, 2H), 6.35 (m, 1H), 7.33 (d, J = 8.9 Hz, 2H), 7.40 (d, J = 8.9 Hz, 2H).

Example 35 (Compound 45)

Step 35-1

While cooling on ice, n-butyl lithium (24.4 mL, 39 mmol, 1.6 mol/L n-hexane solution) followed by triethyl 2-chloro-2-phosphonoacetate (8.55 mL, 40.0 mmol) was added to anhydrous THF (25 ml). After stirring for 10 minutes at room temperature, 4-(methoxymethoxy)acetophenone (7.21 g, 40.0 mmol) dissolved in anhydrous THF (25 mL) was gradually added thereto. While heating under reflux, the mixture was stirred for 8 hours. Next, after cooling, saturated aqueous ammonium chloride solution was added to the reaction solution, and the reaction solution was extracted with ethyl acetate. After the organic layer was washed with saturated brine, the organic layer was dried over anhydrous sodium sulfate and concentrated under reduced pressure. The obtained residue was purified by silica gel column chromatography (n-hexane/ethyl acetate = 49/1). Ethyl (Z)-2-chloro-3-[4-(methoxymethoxy). phenyl]-2-butenoate (0.399 g, 4% yield) was obtained.
FAB-MS m/z: $[M+H]^+$ 285.
$^1$H-NMR ($CDCl_3$) δ (ppm): 1.38 (t, J = 7.1 Hz, 3H), 2.38 (s, 3H), 3.49 (s, 3H), 4.33 (q, J = 7.1 Hz, 2H), 5.19 (s, 2H), 7.05 (d, J = 8.9 Hz, 2H), 7.21 (d, J = 8.9 Hz, 2H).

Step 35-2

Ethyl (Z)-2-chloro-3-[4-(methoxymethoxy)phenyl]-2-butenoate was treated according to Steps 10-3 to 10-5 of Example 10, and Compound 45 was obtained.
FAB-MS m/z: $[M+H]^+$ 417.
$^1$H-NMR (DMSO-$d_6$) δ (ppm): 0.85 (t, J = 6.6 Hz, 3H), 1.15-1.38 (m, 12H), 1.47 (m, 2H), 2.10 (s, 3H), 3.15 (dt, J = 5.2, 6.4 Hz, 2H), 7.30 (d, J = 8.8 Hz, 2H), 7.40 (d, J = 8.8 Hz, 2H), 8.05 (s, 2H), 8.49 (brt, J = 5.2 Hz, 1H).

Example 36 (Compound 46)

Step 36-1

According to Step 35-1 of Example 35, 4-methoxyacetophenone (600 mg, 4.00 mmol) was treated with triethyl 2-phosphonopropionate (0.78 mL, 3.7 mmol), which had been treated with n-butyl lithium (2.5 mL, 4.0 mmol, 1.6 mol/L n-hexane solution) to thereby obtain Ethyl (E)-3-(4-methoxyphenyl)-2-methyl-2-butenoate (132 mg, 14% yield).
FAB-MS m/z: $[M]^+$ 234.
$^1$H-NMR ($CDCl_3$) δ (ppm): 1.34 (t, J = 7.1 Hz, 3H), 1.78 (d, J = 1.0 Hz, 3H), 2.24 (d, J = 1.0 Hz, 3H), 3.81 (s, 3H), 4.25 (q, J = 7.1 Hz, 2H), 6.89 (d, J = 8.6 Hz, 2H), 7.09 (d, J = 8.6 Hz, 2H).

Step 36-2

After treating ethyl (E)-3-(4-methoxyphenyl)-2-methyl-2-butenoate according to Steps 10-3 and 10-4 of Example 10, the resulting (E)-3-(4-methoxyphenyl)-2-methyl-N-nonyl-2-butenamide was demethylated and then sulfamoylated according to Steps 4-2 and 4-3 of Example 4, and Compound 46 was obtained.
APCI-MS m/z: $[M+H]^+$ 397.
$^1$H-NMR (DMSO·$d_6$) δ (ppm): 0.85 (t, J = 6.8 Hz, 3H), 1.13-1.42 (m, 12H), 1.45 (m, 2H), 1.65 (d, J = 1.5 Hz, 3H), 1.93 (d, J = 1.5 Hz, 3H), 3.12 (dt, J = 5.9, 6.8 Hz, 2H), 7.26 (m, 4H), 7.96 (brt, J = 5.3 Hz, 1H), 7.99 (brs, 2H).

Example 37 (Compound 47)

Step 37-1

4-(Methoxymethoxy)benzylcyanide (1.04 g, 5.85 mmol) was obtained from 4-hydroxybenzylcyanide according to Step 7-1 of Example 7 was dissolved in methanol (15 mL). Glycolic acid monohydrate (0.81 g, 8.8 mmol) and potassium carbonate (2.02 g, 14.6 mmol) were added thereto. While heating under reflux, the mixture was stirred for 5 hours. After cooling the reaction solution, the precipitate was collected by filtration, and dissolved in water (80 mL). While cooling on ice, 1 mol/L hydrochloric acid was added thereto to adjust the pH to approximately 3. The resulting

precipitate was collected by filtration, and (Z)-3-cyano-3-[4-(methoxymethoxy)phenyl] acrylic acid (0.78 g, 57% yield) was obtained.
APCI-MS m/z: $[M-H]^-$ 232.
$^1$H-NMR ($CD_3OD$) δ (ppm): 3.45 (s, 3H), 5.24 (s, 2H), 7.00 (s, 1H), 7.14 (d, J = 9.1 Hz, 2H), 7.73 (d, J = 9.1 Hz, 2H).

Step 37-2

(Z)-3-Cyano-3-[4-(methoxymethoxy)phenyl]acrylic acid was amidated according to Step 1-1 of Example 1. Next, according to Steps 7-3 and 7-4 of Example 7, demethoxymethylation and sulfamoylation were conducted, and Compound 47 was obtained.
FAB-MS m/z: $[M+H]^+$ 394.
$^1$H-NMR (DMSO-$d_6$) δ (ppm): 0.85 (t, J = 6.4 Hz, 3H), 1.11-1.37 (m, 12H), 1.46 (m, 2H), 3.18 (q, J = 6.5 Hz, 2H), 7.26 (s, 1H), 7.42 (d, J = 8.9 Hz, 2H), 7.74 (d, J = 8.9 Hz, 2H), 8.14 (brs, 2H), 8.49 (brt, J = 5.9 Hz, 1H).

Example 38 (Compound 48)

Step 38-1

Methyl 4-hydroxybenzoylacetate (3.00 g, 15.5 mmol) was dissolved in DMF (60 mL). Imidazole (2.10 g, 30.9 mmol) and tert-butyl dimethylsilylchloride (2.79 g, 18.5 mmol) were added thereto, and the mixture was stirred at room temperature for 1.5 hours. Water was added to the reaction solution, and the reaction solution was extracted with ethyl acetate. After the organic layer was washed with saturated brine, the organic layer was dried over anhydrous sodium sulfate and concentrated under reduced pressure. The obtained residue was purified by silica gel column chromatography (n-hexane/ethyl acetate = 19/1), and methyl 4-(tert-butyl dimethylsilyloxy)benzoyl acetate (4.81 g, 100% yield) was obtained.
$^1$H-NMR ($CDCl_3$) δ (ppm): 0.24 (s, 6H), 0.99 (s, 9H), 3.75 (s, 3H), 3.95 (s, 2H), 6.88 (d, J = 8.6 Hz, 2H), 7.86 (d, J = 8.6 Hz, 2H).

Step 38-2

Methyl 4-(tert-butyldimethylsilyloxy)benzoyl acetate (4.81 g, 15.5 mmol) was dissolved in THF (60 mL). While cooling on ice, potassium tert-butoxide (2.08 g, 18.5 mmol) and then methyl iodide (1.44 mL, 23.2 mmol) were added thereto. While gradually raising the temperature to room temperature, the mixture was stirred for 5 hours. Saturated aqueous ammonium chloride solution was added to the reaction solution, and the reaction solution was extracted with ethyl acetate. After the organic layer was washed with saturated brine, the organic layer was dried over anhydrous sodium sulfate and concentrated under reduced pressure. The obtained residue was purified by silica gel column chromatography (n-hexane/ethyl acetate = 19/1), and methyl 3-[4-(tert-butyldimethylsilyloxy)phenyl]-2-methyl-3-oxopropionate (4.02 g, 80% yield) was obtained.
$^1$H-NMR ($CDCl_3$) δ (ppm): 0.24 (s, 6H), 0.99 (s, 9H), 1.48 (d, J = 7.0 Hz, 3H), 3.69 (s, 3H), 4.36 (q, J = 7.0 Hz, 1H), 6.88 (d, J = 8.7 Hz, 2H), 7.90 (d, J = 8.7 Hz, 2H).

Step 38-3

Methyl 3-[4-(tert-butyldimethylsilyloxy)phenyl]-2-methyl-3-oxopropionate (4.02 g, 12.3 mmol) was dissolved in toluene (25 mL). Trimethylsilylnitrile (8.18 mL, 61.4 mmol) and then zinc (II) iodide (1.96 g, 6.14 mmol) were added thereto, and the mixture was stirred at room temperature for 18.5 hours. Water was added to the reaction solution, and the reaction solution was extracted with ethyl acetate. The organic layer was washed with saturated aqueous sodium hydrogencarbonate solution and then with saturated brine, dried over anhydrous sodium sulfate and concentrated under reduced pressure. The obtained residue was dissolved in pyridine (30 mL), and phosphorus oxychloride (5.72 mL, 61.4 mmol) was added thereto, and the mixture was stirred for 4 hours at 100 °C. After cooling the reaction solution, ice and 1 mol/L hydrochloric acid were added, and the reaction solution was extracted with ethyl acetate. The organic layer was washed with 1 mol/L hydrochloric acid, water, saturated aqueous sodium hydrogencarbonate solution, and then with saturated brine. Next, the organic layer was dried over anhydrous sodium sulfate and concentrated under reduced pressure. The obtained residue was purified by silica gel column chromatography (n-hexane/ethyl acetate = 19/1), and methyl (E)-3-[4-(tert-butyldimethylsilyloxy) phenyl]-3-cyano-2-methyl acrylate (0.129 g, 3% yield) was obtained.
APCI-MS m/z: $[M-Si(CH_3)_2C(CH_3)_3]^-$ 216.
$^1$H-NMR ($CDCl_3$) δ (ppm): 0.23 (s, 6H), 0.99 (s, 9H), 2.12 (s, 3H), 3.92 (s, 3H), 6.89 (d, J = 8.9 Hz, 2H), 7.27 (d, J =

8.9 Hz, 2H).

Step 38-4

Methyl (E)-3-[4-(tert-butyldimethylsilyloxy)phenyl]-3-cyano-2-methylacrylate (59 mg, 0.18 mmol) was dissolved in a mixed solvent of methanol (2 mL) and water (0.5 mL). Lithium hydroxide (43 mg, 1.8 mmol) was added thereto, and the mixture was stirred for 1.5 hours at 40 °C. Further, lithium hydroxide (43 mg, 1.8 mmol) was added thereto, and after stirring at the same temperature for 4.5 hours, methanol was removed under reduced pressure. Ice water and 1 mol/L hydrochloric acid were added to the obtained residue to adjust the pH to approximately 2. The mixture was then adsorbed to an HP20 resin column (Mitsubishi Kagaku). After the column was washed with water, the column was eluted with methanol. The resulting eluent was concentrated under reduced pressure, and a crude product of 3-cyano-3-(4-hydroxyphenyl)-2-methylacrylic acid (36 mg) was obtained.
$^1$H-NMR ($CD_3OD$) δ (ppm): 2.24 (s, 3H), 6.92 (d, J = 8.9 Hz, 2H), 7.63 (d, J = 8.9 Hz, 2H).

Step 38-5

According to Steps 1-1 and 1-2 of Example 1, amidation and sulfamoylation of the crude product of 3-cyano-3-(4-hydroxyphenyl)-2-methylacrylic acid were conducted to thereby obtain Compound 48.
APCI-MS m/z: $[M-H]^-$ 406.
$^1$H-NMR ($CD_3OD$) δ (ppm): 0.89 (t, J = 6.8 Hz, 3H), 1.05 (m, 2H), 1.16-1.38 (m, 12H), 2.35 (s, 3H), 3.05 (t, J = 6.9 Hz, 2H), 7.34 (d, J = 8.8 Hz, 2H), 7.47 (d, J = 8.8 Hz, 2H).

Example 39 (Compound 49)

According to Example 23, Compound 48 was exposed to UV (254 nm) to thereby obtain Compound 49 (1.8 mg, 6% yield).
ESI-MS m/z: $[M-H]^-$ 406.
$^1$H-NMR ($CD_3OD$) δ (ppm): 0.80 (t, J = 6.9 Hz, 3H), 1.20-1.40 (m, 12H), 1.55 (m, 2H), 1.97 (s, 3H), 3.20 (m, 2H), 7.34 (d, J = 9.0 Hz, 2H), 7.40 (d, J= 9.0 Hz, 2H).

Example 40 (Compound 50)

Step 40-1

Palladium chloride (41 mg, 0.23 mmol) and copper (II) chloride (1.613 g, 12.00 mmol) were suspended in benzene (40 mL). Under a carbon monoxide flow, 1-ethynyl-4-methoxybenzene (528 mg, 4.00 mmol) dissolved in tert-butanol (2.4 mL) was added thereto, and the mixture was stirred for 2 hours at room temperature. After separating the insoluble portion by filtration, the resulting solution was concentrated under reduced pressure. The obtained residue was purified by silica gel column chromatography (n-hexane/ethyl acetate = 15/1). Tert-butyl (Z)-3-chloro-3-(4-methoxyphenyl)acrylate (76 mg, 7% yield) was obtained.
FAB-MS m/z: $[M+H]^+$ 269.
$^1$H-NMR ($CDCl_3$) δ (ppm): 1.53 (s, 9H), 3.84 (s, 3H), 6.39 (s, 1H), 6.90 (d, J = 9.0 Hz, 2H), 7.63 (d, J = 9.0 Hz, 2H).

Step 40-2

According to Step 31-2 of Example 31, tert-butyl (Z)-3-chloro-3-(4-methoxyphenyl)acrylate (72 mg, 0.27 mmol) was treated with trifluoroacetic acid (2 mL) to thereby obtain (Z)-3-chloro-3-(4-methoxyphenyl)acrylic acid (58 mg, 100% yield).
FAB-MS m/z: $[M+H]^+$ 213.
$^1$H-NMR ($CDCl_3$) δ (ppm): 3.86 (s, 3H), 6.53 (s, 1H), 6.93 (d, J = 8.9 Hz, 2H), 7.69 (d, J = 8.9 Hz, 2H).

Step 40-3

According to Steps 4-2 and 4-3 of Example 4, (Z)-3-chloro-3-(4-methoxyphenyl)acrylic acid was demethylated and sulfamoylated to thereby obtain Compound 50.
$^1$H-NMR (DMSO-$d_6$) δ (ppm): 0:85 (t, J = 6.7 Hz, 3H), 1.11-1.36 (m, 12H), 1.42 (m, 2H), 3.12 (m, 2H), 6.96 (s, 1H), 7.33 (d, J = 8.9 Hz, 2H), 7.69 (d, J = 8.9 Hz, 2H), 8.09 (brs, 2H), 8.15 (m, 1H).

Example 41 (Compounds 51 and 59)

Step 41-1

According to Step 24-1 of Example 24, methyl 4-hydroxybenzoyl acetate (300 mg, 1.54 mmol) was treated with N-methyloctylamine (0.84 mL, 4.6 mmol) and DMAP (94 mg, 0.77 mmol) to thereby obtain 3-(4-hydroxypheny) -N-methyl-N-octyl-3-oxopropionamide (320 mg, 68% yield).
APCI-MS m/z: 306 $[M+H]^+$.
$^1$H-NMR (DMSO-$d_6$) δ (ppm): 0.85 (m, 3H), 1.24 (m, 10H), 1.46 (m, 2H), 2.80 (s, 1.5H), 2.92 (s, 1.5H), 3.27 (m, 2H), 4.04 (m, 2H), 6.84 (d, J = 8.7 Hz, 2H), 7.82 (d, J = 8.7 Hz, 2H), 10.38 (brs, 1H).

Step 41-2

According to Step 1-2 of Example 1, 3-(4-hydroxyphenyl)-N-methyl-N-octyl-3-oxopropionamide (31 mg, 0.10 mmol) was treated with sulfamoyl chloride (23 mg, 0.20 mmol) to thereby obtain Compound 51 (9mg, 19% yield) and Compound 59 (21 mg, 53% yield).

Compound 51 (isomer ratio 1/2)

FAB-MS m/z: 464 $[M+H]^+$.
$^1$H-NMR (DMSO-$d_6$) δ (ppm): 0.85 (m, 3H), 1.21 (m, 10H), 1.40 (m, 2H), 2.82 (s, 1H), 3.06 (s, 2H), 3.18 (m, 1.34H), 3.37 (m, 0.66H), 6.79 (s, 0.67H), 6.85 (s, 0.33H), 7.01 (brs, 2H), 7.43 (d, J = 8.9 Hz, 2H), 8.10 (d, J = 8.6 Hz, 1.34H), 8.19 (d, J = 8.9 Hz, 0.66H), 8.26 (brs, 2H).

Compound 59

APCI-MS m/z: 385 $[M+H]^+$.
$^1$H-NMR (DMSO-$d_6$) δ (ppm): 0.86 (t, J = 6.4 Hz, 3H), 1.24 (m, 10H), 1.48 (m, 2H), 2.81 (s, 1.5H), 2.95 (s, 1.5H), 3.27 (t, J = 7.6 Hz, 2H), 4.17 (brs, 2H), 7.40 (d, J = 8.3 Hz, 2H), 8.03 (d, J = 8.6 Hz, 1H), 8.03 (d, J = 8.9 Hz, 1H), 8.21 (brs, 2H).

Example 42 (Compound 52)

Step 42-1

According to Step 6-1 of Example 6, 4-methoxycinnamic acid (1.00 g, 5.62 mmol) was treated with 1-(3-dimethylaminopropyl)-3-ethyl carbodiimide (1.30 mL, 6.79 mmol), HOBt (1.04 g, 6.77 mmol), and N,O-dimethylhydroxyamine hydrochloride (0.66 g, 6.7 mmol) to thereby obtain (E)-N-methoxy-3-(4-methoxyphenyl)-N-methylacrylamide (1.22 g, 99% yield).
APCI-MS m/z: $[M+H]^+$ 222.
$^1$H-NMR ($CDCl_3$) δ (ppm): 3.30 (s, 3H), 3.76 (s, 3H), 3.84 (s, 3H), 6.90 (d, J = 8.6 Hz, 2H), 6.91 (d, J = 15.8 Hz, 1H), 7.52 (d, J = 8.6 Hz, 2H), 7.69 (d, J = 15.8 Hz, 1H).

Step 42-2

(E)-N-methoxy-3-(4-methoxyphenyl)-N-methylacrylamide (210 mg, 0.949 mmol) was dissolved in THF (2 mL). While cooling on ice, n-octylmagnesium bromide (1.5 mL, 1 mol/L THF solution) was added thereto, and the mixture was stirred for 1.5 hours. After stirring for a further 40 minutes at 50 °C, n-octylmagnesium bromide (1.5 mL, 1mol/L THF solution) was further added, and the mixture was stirred at room temperature for 40 minutes. Saturated aqueous ammonium chloride solution was added to the reaction solution, and the reaction solution was extracted with ethyl acetate. After the organic layer was washed with saturated brine, the organic layer was dried over anhydrous sodium sulfate and concentrated under reduced pressure. The obtained residue was purified by silica gel column chromatography (n-hexane/ethyl acetate = 4/1), and 1-(4-methoxyphenyl)-undece-1-ene-3-one (95 mg, 37% yield) was obtained.
APCI-MS m/z: $[M+H]^+$ 275.
$^1$H-NMR ($CDCl_3$) δ (ppm): 0.88 (t, J = 7.3 Hz, 3H), 1.10-1.38 (m, 10H), 1.67 (m, 2H), 2.63 (t, J = 7.5 Hz, 2H), 3.84 (s, 3H), 6.63 (d, J = 16.2 Hz, 1H), 6.91 (d, J = 8.6 Hz, 2H), 7.50 (d, J = 8.6 Hz, 2H), 7.51 (d, J = 16.2 Hz, 1H).

Step 42-3

According to Steps 4-2 and 4-3 of Example 4, 1-(4-methoxyphenyl)-undece-1-ene-3-one was demethylated and then sulfamoylated to thereby obtain Compound 52.
APCI-MS m/z: $[M-H]^-$ 338.
$^1$H-NMR ($CDCl_3$) δ (ppm): 0.88 (t, J = 6.7 Hz, 3H), 1.18-1.41 (m, 10H), 1.67 (m, 2H), 2.65 (t, J = 7.4 Hz, 2H), 5.04 (s, 2H), 6.70 (d, J = 16.2 Hz, 1H), 7.35 (d, J = 8.7 Hz, 2H), 7.51 (d, J = 16.2 Hz, 1H), 7.59 (d, J = 8.7 Hz, 2H).

Eample 43 (Compound 53)

Step 43-1

(E)-3-(4-Methoxyphenyl)-2-[(nonylcarbamoyl)methyl]acrylic acid (70 mg, 0.19 mmol) was dissolved in a mixed solvent of dichloromethane (2 mL) and methanol (1 mL). Trimethylsilyldiazomethane (0.15 mL, 0.30 mmol, 2.0 mol/L n-hexane solution) was added thereto, and the mixture was stirred at room temperature for 15 minutes. Trimethylsilyldiazomethane (0.10 mL, 0.20 mmol, 2.0 mol/L n-hexane solvent) was further added to the reaction solution, and the mixture was stirred at the same temperature for 15 minutes and concentrated under reduced pressure, and methyl (E)-3-(4-methoxyphenyl)-2-[(nonylcarbamoyl)methyl]acrylate (76 mg, 100% yield) was obtained.
APCI-MS m/z: $[M+H]^+$ 376.
$^1$H-NMR ($CDCl_3$) δ (ppm): 0.88 (t, J = 6.6 Hz, 3H), 1.15-1.40 (m, 12H), 1.48 (m, 2H), 3.24 (dt, J = 6.3, 6.9 Hz, 2H), 3.44 (s, 2H), 3.83 (s, 3H), 3.84 (s, 3H), 6.35 (brt, 1H), 6.95 (d, J = 8.8 Hz, 2H), 7.78 (d, J = 8.8.Hz, 2H), 7.86 (s, 1H).

Step 43-2

According to Steps 4-2 and 4-3 of Example 4, demethylation and then sulfamoylation were conducted on methyl (E)-3-(4-methoxyphenyl)-2-[(nonylcarbamoyl)methyl]acrylate to thereby obtain Compound 53.
APCI-MS m/z: $[M+H]^+$ 441.
$^1$H-NMR (DMSO-$d_6$) δ (ppm): 0.85 (t, J = 6.4 Hz, 3H), 1.12-1.34 (m, 12H), 1.39 (m, 2H), 3.05 (dt, J = 5.6, 6.7 Hz, 2H), 3.30 (s, 2H), 3.71 (s, 3H), 7.30 (d, J = 8.8 Hz, 2H), 7.58 (d, J = 8.8 Hz, 2H), 7.75 (s, 1H), 7.94 (t, J = 5.6 Hz, 1H), 8.05 (brs, 2H).

Example 44 (Compound 54)

Step 44-1

4-(Methoxymethoxy)benzaldehyde (574 mg, 3.46 mmol) and ethyl isocyanoacetate (0.378 mL, 3.46 mmol) dissolved in THF (12 mL) were gradually added at 35°C to sodium hydride (166 mg, 4.1 mmol, 60% in oil) suspended in THF (7 mL). The mixture was stirred for 5 minutes. After cooling the reaction solution on ice, 10% aqueous acetic acid solution (4.0 mL) was added thereto, and the reaction solution was extracted with ethyl acetate. After the organic layer was washed with saturated brine, the organic layer was dried over anhydrous sodium sulfate and condensed under reduced pressure. The obtained residue was purified by silica gel column chromatography (chloroform), and ethyl 2-formylamino-3-[4-(methoxymethoxy)phenyl]acrylate (654 mg, 68% yield) was obtained.
APCI-MS m/z: $[M+H]^+$ 280.

Step 44-2

Ethyl 2-formylamino-3-[4-(methoxymethoxy)phenyl]acrylate was treated according to Steps 10-3 to 10-5 of Example 10, and Compound 54 was obtained.
APCI-MS m/z: $[M-H]^-$ 410.
$^1$H-NMR ($CDCl_3$) δ (ppm): 0.88 (t, J = 6.4 Hz, 3H), 1.13-1.42 (m, 12H), 1.56 (m, 2H), 3.32 (m, 2H), 5.50 (m, 0.5H), 5.77 (m, 0.5H), 6.43 (m, 0.5H), 6.60 (m, 0.5H), 6.92 (brs, 1H), 7.03 (brs, 1H), 7.10-7.51 (m, 5H), 8.04 (brs, 1H).

Example 45 (Compound 55)

Step 45-1

(E)-2-Cyano-3-(4-hydroxyphenyl)-N-nonyl-2-butenamide (50 mg, 0.15 mmol) was dissolved in THF (1 mL). Sulfuryl chloride (0.018 mL, 0.22 mmol) was added, and the mixture was stirred at room temperature for 1.5 hours.

Sulfuryl chloride (0.018 mL, 0.22 mmol) was further added thereto, and the mixture was stirred at the same temperature for 1 hour. Saturated aqueous sodium hydrogencarbonate solution was added to the reaction solution, and the reaction solution was extracted with ethyl acetate. After the organic layer was washed with saturated brine, the organic layer was dried over anhydrous sodium sulfate and concentrated under reduced pressure. The obtained residue was purified by preparative thin layer chromatography (chloroform/methanol = 40/1), and (E)-3-(3-chloro-4-hydroxyphenyl-2-cyano-N-nonyl-2-butenamide (40 mg, 72% yield) was obtained.
APIC-MS m/z: $[M-H]^-$ 361.
$^1$H-NMR ($CDCl_3$) $\delta$ (ppm): 0.88 (t, J = 6.6 Hz, 3H), 1.15-1.45 (m, 12H), 1.58 (m, 2H), 2.62 (s, 3H), 3.35 (dt, J = 6.3, 6.9 Hz, 2H), 6.34 (brs, 2H), 7.07 (d, J = 8.5 Hz, 1H), 7.30 (dd, J = 2.3, 8.5 Hz, 1H), 7.54 (d, J = 2.3 Hz, 1H).

Step 45-2

According to Step 1-2 of Example 1, (E)-3-(3-chloro-4-hydroxyphenyl)-2-cyano-N-nonyl-2-butenamide (38 mg, 0.10 mmol) was treated with sulfamoyl chloride (93 mg, 0.80 mmol) to thereby obtain Compound 55 (24 mg, 53% yield).
APIC-MS m/z: $[M+H]^+$ 442.
$^1$H-NMR ($CDCl_3$) $\delta$ (ppm): 0.88 (t, J = 6.6 Hz, 3H), 1.15-1.45 (m, 12H), 1.58 (m, 2H), 2.61 (m, 3H), 3.35 (dt, J = 6.4, 6.9 Hz, 2H), 5.42 (brs, 2H), 6.32 (m, 1H), 7.35 (dd, J = 2.2, 8.5 Hz, 1H), 7.51 (dd, J = 2.0, 2.2 Hz, 1H), 7.55 (dd, J = 2.0, 8.5 Hz, 1H).

Example 46 (Compound 56)

Step 46-1

(E)-2-Cyano-3-(4-hydroxyphenyl)-N-nonyl-2-butenamide (54 mg, 0.16 mmol) was dissolved in chloroform (2 mL). While cooling on ice, tetrabutylammonium tribromide (86 mg, 0.18 mmol) was added thereto, and the mixture was stirred at room temperature for 1.5 hours. Saturated aqueous sodium sulfite solution was added to the reaction solution, and the reaction solution was extracted with ethyl acetate. The organic layer was washed with 1 mol/L hydrochloric acid, saturated aqueous sodium hydrogencarbonate solution, and then saturated brine. Next, the organic layer was dried over anhydrous sodium sulfate and concentrated under reduced pressure. The obtained residue was purified by preparative thin layer chromatography (chloroform/methanol = 20/1), and (E)-3-(3-bromo-4-hydroxyphenyl)-2-cyano-N-nonyl-2-butenamide (16 mg, 24% yield) was obtained.
APIC-MS m/z: $[M-H]^-$ 405,407.
$^1$H-NMR ($CDCl_3$) $\delta$ (ppm): 0.88 (t, J = 6.7 Hz, 3H), 1.10-1.40 (m, 12H), 1.58 (m, 2H), 2.62 (s, 3H), 3.35 (dt, J = 5.9, 6.7 Hz, 2H), 5.98 (brs, 1H), 6.29 (brt, 1H), 7.07 (d, J = 8.4 Hz, 1H), 7.35 (dd, J = 2.2, 8.4 Hz, 1H), 7.58 (d, J = 2.2 Hz; 1H).

Step 46-2

According to Step 1-2 of Example 1, (E)-3-(3-bromo-4-hydroxyphenyl)-2-cyano-N-nonyl-2-butenamide (14 mg, 0.034 mmol) was treated with sulfamoyl chloride (80 mg, 0.69 mmol) to thereby obtain Compound 56 (10 mg, 62% yield).
FAB-MS m/z: $[M+H]^+$ 486, 488.
$^1$H-NMR ($CDCl_3$) $\delta$ (ppm): 0.88 (t, J = 6.4 Hz, 3H), 1.11-1.45 (m, 12H), 1.58 (m, 2H), 2.63 (s, 3H), 3.35 (q, J = 6.7 Hz, 2H), 5.36 (brs, 2H), 6.30 (brt, 1H), 7.40 (dd, J = 2.1, 8.4 Hz, 1H), 7.59 (d, J = 8.4 Hz, 1H), 7.67 (d, J = 2.1 Hz, 1H).

Example 47 (Compound 57)

Step 47-1

Triphenylphosphine oxide (430 mg, 1.55 mmol) was dissolved in 1,2-dichloroethane (3 mL). A 1,2-dichloroethane solution (3 mL) of trifluoromethanesulfonic acid anhydride (0.260 mL, 1.55 mmol) was added thereto, and the mixture was stirred at 0 °C for 15 minutes. Next, a 1,2-dichloroethane solution (2.5 mL) of methyl 4-hydroxybenzoyl acetate (200 mg, 1.03 mmol) and triethylamine (0.431 mL, 3.09 mmol) were added thereto. While heating under reflux, the mixture was stirred for 3 hours. Water was added to the reaction solution, and the reaction solution was extracted with chloroform. After the organic layer was washed with saturated brine, the organic layer was dried over anhydrous sodium sulfate and concentrated under reduced pressure. The obtained residue was purified by preparative thin layer chromatography (chloroform/methanol = 20/1, then n-hexane/ethyl acetate = 2/1), and methyl (4-hydroxyphenyl)propynoate (45 mg, 25% yield) was obtained.

ESI-MS m/z: $[M+H]^+$ 177.
$^1$H-NMR ($CDCl_3$ + $CD_3OD$) δ (ppm): 3.82 (s, 3H), 6.82 (d, J = 8.9 Hz, 2H), 7.45 (d, J = 8.6 Hz, 2H).

Step 47-2

Methyl (4-hydroxyphenyl)propynoate (44 mg, 0.25 mmol) was dissolved in methanol (2.2 mL). 3 mol/L sodium hydroxide aqueous solution (0.24 mL, 0.72 mmol) was added thereto, and the mixture was stirred at 50 °C for 2 hours. 1 mol/L Hydrochloric acid was added to the reaction solution, and the reaction solution was extracted with ethyl acetate. After the organic layer was washed with saturated brine, the organic layer was dried over anhydrous sodium sulfate. The resulting organic layer was concentrated under reduced pressure, and (4-hydroxyphenyl)propynoic acid (38 mg, 93% yield) was obtained.
APCI-MS m/z: $[M-H]^-$ 161.
$^1$H-NMR ($CDCl_3$ + $CD_3OD$) δ (ppm): 5.00 (brs, 1H), 6.81 (d, J = 8.7 Hz, 2H), 7.44 (d, J = 8.9 Hz, 2H).

Step 47-3

According to Steps 1-1 and 1-2 of Example 1, (4-hydroxyphenyl)propynoic acid was amidated and then sulfamoylated to thereby obtain Compound 57.
ESI-MS m/z: 367 $[M+H]^+$.
$^1$H-NMR (DMSO-$d_6$) δ (ppm): 0.85 (t, J = 6.7 Hz, 3H), 1.24 (m, 12H), 1.42 (m, 2H), 3.10(m, 2H), 7.34 (d, J = 8.7 Hz, 2H), 7.65 (d, J = 8.7 Hz, 2H), 8.14 (s, 2H), 8.75 (t, J = 5.8 Hz, 1H).

Example 48 (Compound 58)

According to Steps 41-1 and 41-2 of Example 41, methyl 4-hydroxybenzoylacetate was amidated and then sulfamoylated to thereby obtain Compound 58.
APCI-MS m/z: 385 $[M+H]^+$.
$^1$H-NMR (DMSO-$d_6$) δ (ppm): 0.87 (t, J = 6.5 Hz, 3H), 1.25 (m, 12H), 1.40 (m, 2H), 3.05 (m, 1.4H), 3.16 (m, 0.6H), 3.87 (m, 0.7H), 5.74 (s, 0.3H), 7.37 (d, J = 8.7 Hz, 0.6H), 7.41 (d, J = 8.7 Hz, 1.4H), 7.76 (d, J = 8.7 Hz, 0.6H), 8.06 (d, J = 8.7 Hz, 1.4H), 8.11 (m, 1H), 8.18 (brs, 2H).
The synthesis methods for compounds used in the Examples are described below.

Reference Example 1 [2-(2-butoxyethoxy) ethylamine]

Step 1-1

2-(2-Butoxyethoxy)acetate (1.02 g, 5.78 mmol) was dissolved in dichloromethane (5 mL). Oxalyl chloride (2.52 mL, 28.9 mmol) was added thereto, and the mixture was stirred at room temperature for 1 hour. The reaction solution was concentrated under reduced pressure, and the obtained residue was dissolved in 1,4-dioxane (10 mL). While cooling on ice, 0.5 mol/L ammonia-1,4-dioxane solution (60 mL) was added to the solution. The mixture was stirred at room temperature for 19 hours. Water was added to the reaction solution, and the reaction solution was extracted with chloroform. After the organic layer was washed with saturated brine, the organic layer was dried over anhydrous sodium sulfate, and the solvent was removed under reduced pressure. 2-(2-butoxyethoxy) acetamide (0.94 g, 92% yield) was obtained.
APCI-MS m/z: $[M+H]^+$ 176.

Step 1-2

2-(2-Butoxyethoxy)acetamide (922 mg, 5.26 mmol) was dissolved in THF (20 mL). While cooling on ice, lithium aluminum hydride (420 mg, 11.1 mmol) suspended in THF (20 mL) was added thereto. The mixture was stirred at the same temperature for 1 hour. After stirring for another 6 hours at room temperature, sodium sulfate hydrate (8.90 g, 27.6 mmol) was added to the reaction solution and the mixture was stirred. The insoluble portion was separated by filtration, and the solvent was removed under reduced pressure, and 2-(2-butoxyethoxy) ethylamine (779 mg, 92% yield) was obtained.
APCI-MS m/z: $[M+H]^+$ 162.
$^1$H-NMR ($CDCl_3$) δ (ppm): 0.93 (t, J = 7.2 Hz, 3H), 1.20-1.72 (m, 4H), 2.87 (t, J = 5.2 Hz, 2H), 3.40-3.78 (m, 8H).

Reference Example 2 [2-cyano-N-decylacetamide]

According to Step 1-1 of Example 1, cyanoacetic acid (505 mg, 5.93 mmol) was treated with EDCI (1.71 g, 8.90 mmol), HOBt (1.36 g, 8.90 mmol), and n-decylamine (1.78 mL, 8.91 mmol) to thereby obtain 2-cyano-N-decylacetamide (774 mg, 58% yield).
ESI-MS m/z: $[M-H]^-$ 223.
$^1$H-NMR ($CDCl_3$) δ (ppm): 0.88 (t, J =8.6 Hz, 3H), 1.15-1.38 (m, 14H), 1.54 (m, 2H), 3.30 (dt, J = 5.8, 7.1 Hz, 2H), 3.36 (s, 2H), 6.06 (brt, 1H).

Reference Example 3 [2-cyano-N-nonylacetamide]

According to Step 1-1 of Example 1, cyanoacetic acid (10.00 g, 117.6 mmol) was treated with EDCI (33.81 g, 176.4 mmol), HOBt (27.01g, 176.4 mmol), and n-nonylamine (25.5 mL, 141 mmol) to thereby obtain 2-cyano-N-nonylacetamide (13.69 g, 55% yield).
FAB-MS m/z: $[M+H]^+$ 211.
$^1$H-NMR ($CDCl_3$) δ (ppm): 0.88 (t, J = 6.7 Hz, 3H), 1.08-1.42 (m, 12H), 1.54 (m, 2H), 3.30 (m, 2H), 3.36 (s, 2H), 6.09 (brs, 1H).

Formulation Example 1 (Tablet)

A tablet having the following composition is formulated by the standard methods.

| | |
|---|---|
| Compound 1 | 5 mg |
| Lactose | 60 mg |
| Potato starch | 30 mg |
| Polyvinyl alcohol | 2 mg |
| Magnesium stearate | 1 mg |
| Tar pigment | small amount |

Formulation Example 2 (granules)

Granules having the following composition are formulated by the standard methods.

| | |
|---|---|
| Compound 2 | 5 mg |
| Lactose | 280 mg |

Industrial Applicability

According to the present invention, aryl sulfamate derivatives or pharmaceutically acceptable salts thereof which have an inhibitory activity against steroid sulfatase and are useful in treating or preventing steroid hormone dependent diseases are provided.

## Claims

**1.** An aryl sulfamate derivative represented by Formula (I)

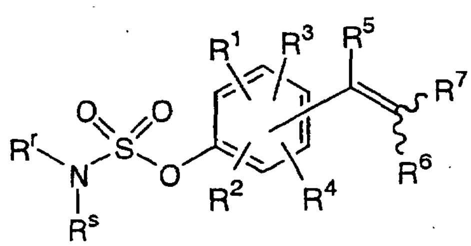

(I)

{wherein $R^r$ and $R^s$, which may be the same or different, each represent a hydrogen atom or lower alkyl, $R^1$, $R^2$, $R^3$ and $R^4$, which may be the same or different, each represent a hydrogen atom, lower alkyl, a halogen atom, nitro, cyano, azido, $OR^8$ [wherein $R^8$ represents a hydrogen atom, lower alkyl, lower alkanoyl or $SO_2NR^{ra}R^{sa}$ (wherein $R^{ra}$ and $R^{sa}$ have the same meanings as the previously described $R^r$ and $R^s$, respectively)], $COR^9$ [wherein $R^9$ represents a hydrogen atom, lower alkyl, hydroxy, lower alkoxy or $NR^{10}R^{11}$ (wherein $R^{10}$ and $R^{11}$, which may be the same or different, each represent a hydrogen atom or lower alkyl)] or $NR^{12}R^{13}$ [wherein $R^{12}$ represents a hydrogen atom or lower alkyl, and $R^{13}$ represents a hydrogen atom, lower alkyl, lower alkanoyl, or $SO_2NR^{rb}R^{sb}$ (wherein $R^{rb}$ and $R^{sb}$ have the same meanings as the previously described $R^r$ and $R^s$, respectively)], $R^5$ represents a hydrogen atom, substituted or unsubstituted alkyl, substituted or unsubstituted lower cycloalkyl, a halogen atom, nitro, cyano, azido, isocyano, substituted or unsubstituted lower alkynyl, substituted or unsubtitued lower alkenyl, hydroxy, substituted or unsubstituted alkoxy, $OSO_2NR^{rc}R^{sc}$ (wherein $R^{rc}$ and $R^{sc}$ have the same meanings as the previously described $R^r$ and $R^s$, respectively), $COR^{14}$ [wherein $R^{14}$ represents a hydrogen atom, substituted or unsubstituted alkyl, substituted or unsubstituted lower cycloalkyl, substituted or unsubstituted aryl, a substituted or unsubstituted heterocyclic group, $OR^{15}$ (wherein $R^{15}$ represents a hydrogen atom, substituted or unsubstituted alkyl, substituted or unsubstituted lower cycloalkyl, substituted or unsubstituted aryl, or a substituted or unsubstituted heterocyclic group) or $NR^{16}R^{17}$ (wherein $R^{16}$ and $R^{17}$, which may be the same or different, each represent a hydrogen atom, substituted or unsubstituted alkyl, substituted or unsubstituted lower cycloalkyl, substituted or unsubstituted aryl, or a substituted or unsubstituted heterocyclic group)], $NR^{18}R^{19}$ (wherein $R^{18}$ and $R^{19}$, which may be the same or different, each represent substituted or unsubstituted alkyl), $NR^{20}COR^{21}$ (wherein $R^{20}$ and $R^{21}$, which may be the same or different, each represent a hydrogen atom, substituted or unsubstituted alkyl, substituted or unsubstituted lower cycloalkyl, substituted or unsubstituted aryl, or a substituted or unsubstituted heterocyclic group) or $SO_2R^{22}$ (wherein $R^{22}$ represents substituted or unsubstituted alkyl), $R^6$ represents a hydrogen atom, substituted or unsubstituted alkyl, substituted or substituted lower cycloalkyl, a halogen atom, nitro, cyano, azido, isocyano, substituted or unsubstituted lower alkynyl, substituted or unsubstituted lower alkenyl, hydroxy, substituted or unsubstituted alkoxy, substituted or unsubstituted aryl, a substituted or unsubstituted heterocyclic group, $OSO_2NR^{rcx}R^{scx}$ (wherein $R^{rcx}$ and $R^{scx}$ have the same meanings as the previously described $R^r$ and $R^s$, respectively), $COR^{14x}$ (wherein $R^{14x}$ has the same meaning as the previously described $R^{14}$), $NR^{18x}R^{19x}$ (wherein $R^{18x}$ and $R^{19x}$ have the same meanings as the previously described $R^{18}$ and $R^{19}$, respectively), $NR^{20x}COR^{21x}$ (wherein $R^{20x}$ and $R^{21x}$ have the same meanings as the previously described $R^{20}$ and $R^{21}$, respectively) or $SO_2R^{22x}$ (wherein $R^{22x}$ has the same meaning as the previously described $R^{22}$), or $R^5$ and $R^6$ are joined to form a bond, and $R^7$ represents $X^1NR^{23}R^{24}$ [wherein $X^1$ represents C=O or C=S, $R^{23}$ represents a hydrogen atom, substituted or unsubstituted alkyl, substituted or unsubstitued lower cycloalkyl, substituted or unsubstituted aryl, or a substituted or unsubstituted heterocyclic group, and $R^{24}$ represents a hydrogen atom, substituted or unsubstituted alkyl, substituted or unsubstituted lower cycloalkyl, substituted or unsubstituted aryl, a substituted or unsubstituted heterocyclic group or $OR^{25}$ (wherein $R^{25}$ represents a hydrogen atom, substituted or unsubstituted alkyl, substituted or unsubstituted lower cycloalkyl, substituted or unsubstituted aryl, or a substituted or unsubstituted heterocyclic group)] or $COR^{26}$ [wherein $R^{26}$ represents a hydrogen atom, substituted or unsubstituted alkyl, substituted or unsubstituted lower cycloalkyl, substituted or unsubstituted aryl, a substituted or unsubstituted heterocyclic group or $OR^{27}$ (wherein $R^{27}$ represents a hydrogen atom, substituted or unsubstitued alkyl, substituted or unsubstituted lower cycloalkyl, substituted or unsubstituted aryl, or a substituted or unsubstituted heterocyclic group)] with the proviso that (1) when $-CR^5=CR^6R^7$ is placed on the benzene ring at the ortho position to $-OSO_2NR^rR^s$, one of $R^1$, $R^2$, $R^3$ and $R^4$ is $NH_2$ and is placed on the benzene ring at the para position to $-OSO_2NR^rR^s$, the rest of $R^1$, $R^2$, $R^3$ and $R^4$ as well as $R^5$ are hydrogen atoms and $R^6$ is cyano, then $R^7$ is not carboxy, (2) when one of $R^1$, $R^2$, $R^3$ and $R^4$ is hydroxy or $OSO_2NR^{ra}R^{sa}$ (wherein $R^{ra}$ and $R^{sa}$ have the same meanings as previously described, respectively) and the rest of $R^1$, $R^2$, $R^3$ and $R^4$ are hydrogen atoms, then said hydroxy or said $OSO_2NR^{ra}R^{sa}$ is only placed on the benzene ring at the meta position to $-OSO_2NR^rR^s$, and (3) when $R^5$ is hydroxy, then $R^7$ only represents $X^1NR^{23}R^{24}$ (wherein $X^1$, $R^{23}$ and $R^{24}$ have the same meanings as previously described, respectively) or $CO_2R^{27}$ (wherein $R^{27}$ has the same meaning as previously described)}, or a pharmaceutically acceptable salt thereof.

**2.** An aryl sulfamate derivative represented by Formula (IA)

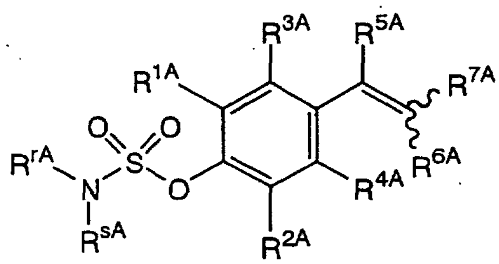

(IA)

[wherein $R^{rA}$, $R^{sA}$, $R^{3A}$, $R^{4A}$, $R^{5A}$, $R^{6A}$ and $R^{7A}$ have the same meanings as the previously described $R^r$, $R^s$, $R^3$, $R^4$, $R^5$, $R^6$ and $R^7$, respectively, and $R^{1A}$ and $R^{2A}$, which may be the same or different, each represent a hydrogen atom, lower alkyl, a halogen atom, nitro, cyano, azido, $OR^{8A}$ (wherein $R^{8A}$ represents lower alkyl or lower alkanoyl), $COR^{9A}$ (wherein $R^{9A}$ has the same meaning as the previously described $R^9$) or $NR^{12A}R^{13A}$ (wherein $R^{12A}$ and $R^{13A}$ have the same meanings as the previously described $R^{12}$ and $R^{13}$, respectively), with the proviso that when $R^{5A}$ is hydroxy, $R^{7A}$ only represents $X^{1A}NR^{23A}R^{24A}$ (wherein $X^{1A}$, $R^{23A}$ and $R^{24A}$ have the same meanings as the previously described $X^1$, $R^{23}$ and $R^{24}$, respectively) or $CO_2R^{27A}$ (wherein $R^{27A}$ has the same meaning as the previously described $R^{27}$)], or a pharmaceutically acceptable salt thereof.

**3.** The aryl sulfamate derivative or the pharmaceutically acceptable salt thereof according to claim 2, wherein $R^{1A}$ and $R^{2A}$ are hydrogen atoms.

**4.** The aryl sulfamate derivative or the pharmaceutically acceptable salt thereof according to claim 2, wherein at least one of $R^{1A}$ and $R^{2A}$ is a halogen atom.

**5.** The aryl sulfamate derivative or the pharmaceutically acceptable salt thereof according to claim 3 or 4, wherein $R^{5A}$ is a hydrogen atom, substituted or unsubstituted alkyl, substituted or unsubstituted lower cycloalkyl, a halogen atom, nitro, cyano, azido, isocyano, substituted or unsubstituted lower alkynyl, substituted or unsubstituted lower alkenyl, substituted or unsubstituted alkoxy, $OSO_2NR^{rc}R^{sc}$ (wherein $R^{rc}$ and $R^{sc}$ have the same meanings as previously described, respectively), $COR^{14}$ (wherein $R^{14}$ has the same meaning as previously described), $NR^{18}R^{19}$ (wherein $R^{18}$ and $R^{19}$ have the same meanings as previously described, respectively), $NR^{20}COR^{21}$ (wherein $R^{20}$ and $R^{21}$ have the same meanings as previously described, respectively) or $SO_2R^{22}$ (wherein $R^{22}$ has the same meaning as previously described), and $R^{6A}$ is a hydrogen atom, substituted or unsubstituted alkyl, substituted or unsubstituted lower cycloalkyl, a halogen atom, nitro, cyano, azido, isocyano, substituted or unsubstituted lower alkynyl, substituted or unsubstituted lower alkenyl, substituted or unsubstituted alkoxy, substituted or unsubstituted aryl, a substituted or unsubstituted heterocyclic group, $OSO_2NR^{rcx}R^{scx}$ (wherein $R^{rcx}$ and $R^{scx}$ have the same meanings as previously described, respectively), $COR^{14x}$ (wherein $R^{14x}$ has the same meaning as previously described), $NR^{18x}R^{19x}$ (wherein $R^{18x}$ and $R^{19x}$ have the same meanings as previously described, respectively), $NR^{20x}COR^{21x}$ (wherein, $R^{20x}$ and $R^{21x}$ have the same meanings as previously described, respectively) or $SO_2R^{22x}$ (wherein $R^{22x}$ has the same meaning as previously described).

**6.** The aryl sulfamate derivative or the pharmaceutically acceptable salt thereof according to claim 3 or 4, wherein $R^{5A}$ is hydroxy.

**7.** The aryl sulfamate derivative or the pharmaceutically acceptable salt thereof according to claim 3 or 4, wherein $R^{6A}$ is hydroxy.

**8.** The aryl sulfamate derivative or the pharmaceutically acceptable salt thereof according to any one of claims 3 to 5, wherein $R^{6A}$ is a hydrogen atom, substituted or unsubstituted alkyl, a halogen atom, nitro, cyano, azido, isocyano, substituted or unsubstituted alkoxy, $OSO_2NR^{rcx}R^{scx}$ (wherein $R^{rcx}$ and $R^{scx}$ have the same meanings as previously described, respectively), $COR^{14x}$ (wherein $R^{14x}$ has the same meaning as previously described), $NR^{20x}COR^{21x}$ (wherein $R^{20x}$ and $R^{21x}$ have the same meanings as previously described, respectively) or $SO_2R^{22x}$ (wherein $R^{22x}$ has the same meaning as previously described).

**9.** The aryl sulfamate derivative or the pharmaceutically acceptable salt thereof according to any one of claims 3 to 5, wherein $R^{6A}$ is a hydrogen atom or substituted or unsubstituted alkyl.

**10.** The aryl sulfamate derivative or the pharmaceutically acceptable salt thereof according to any one of claims 3, 4 and 6, wherein $R^{6A}$ is a hydrogen atom or substituted or unsubstituted alkyl.

**11.** The aryl sulfamate derivative or the pharmaceutically acceptable salt thereof according to claim 9, wherein $R^{5A}$ is cyano, a halogen atom, a hydrogen atom or substituted or unsubstituted alkyl.

**12.** The aryl sulfamate derivative or the pharmaceutically acceptable salt thereof according to claim 3 or 4, wherein $R^{5A}$ and $R^{6A}$ are joined to form a bond.

**13.** The aryl sulfamate derivative or the pharmaceutically acceptable salt thereof according to claim 8, wherein $R^{5A}$ is a hydrogen atom or substituted or unsubstituted alkyl.

**14.** The aryl sulfamate derivative or the pharmaceutically acceptable salt thereof according to any one of claims 3, 4 and 7, wherein $R^{5A}$ is a hydrogen atom or substituted or unsubstituted alkyl.

**15.** The aryl sulfamate derivative or the pharmaceutically acceptable salt thereof according to any one of claims 3 to 5, wherein $R^{6A}$ is cyano or a halogen atom.

**16.** The aryl sulfamate derivative or the pharmaceutically acceptable salt thereof according to claim 6, wherein $R^{6A}$ is cyano.

**17.** The aryl sulfamate derivative or the pharmaceutically acceptable salt thereof according to claim 15, wherein $R^{5A}$ is a hydrogen atom or substituted or unsubstituted alkyl.

**18.** The aryl sulfamate derivative or the pharmaceutically acceptable salt thereof according to claim 3 or 4, wherein $R^{5A}$ is a hydrogen atom or alkyl.

**19.** The aryl sulfamate derivative or the pharmaceutically acceptable salt thereof according to any one of claims 2 to 18, wherein $R^{7A}$ is $X^1NR^{23}R^{24}$ (wherein $X^1$, $R^{23}$ and $R^{24}$ have the same meanings as previously described, respectively).

**20.** The aryl sulfamate derivative or the pharmaceutically acceptable salt thereof according to any one of claims 2 to 18, wherein $R^{7A}$ is $CONR^{23}R^{24}$ (wherein $R^{23}$ and $R^{24}$ have the same meanings as previously described, respectively).

**21.** The aryl sulfamate derivative or the pharmaceutically acceptable salt thereof according to any one of claims 2 to 18, wherein $R^{7A}$ is $CONR^{23a}R^{24a}$ (wherein $R^{23a}$ and $R^{24a}$, which may be the same or different, each represent a hydrogen atom or substituted or unsubstituted alkyl).

**22.** The aryl sulfamate derivative or the pharmaceutically acceptable salt thereof according to claim 18, wherein $R^{7A}$ is $CONR^{23b}R^{24b}$ (wherein $R^{23b}$ and $R^{24b}$, which may be the same or different, each represent a hydrogen atom or alkyl having 7 to 20 carbon atoms).

**23.** The aryl sulfamate derivative or the pharmaceutically acceptable salt thereof according to claim 18, wherein $R^{7A}$ is $CONR^{23c}R^{24c}$ (wherein $R^{23c}$ and $R^{24c}$, which may be the same or different, each represent a hydrogen atom or alkyl having 1 to 6 carbon atoms).

**24.** The aryl sulfamate derivative or the pharmaceutically acceptable salt thereof according to any one of claims 2 to 23, wherein $R^{3A}$ and $R^{4A}$ are hydrogen atoms.

**25.** The aryl sulfamate derivative or the pharmaceutically acceptable salt thereof according to any one of claims 2 to 24, wherein $R^{rA}$ and $R^{sA}$ are hydrogen atoms.

**26.** A steroid sulfatase inhibitor comprising the aryl sulfamate derivative or the pharmaceutically acceptable salt thereof according to any one of claims 1 to 25.

**27.** A therapeutic or prophylactic agent for a disease selected from the group consisting of breast cancer, endometrial cancer, ovarian cancer, and prostate cancer, comprising at least one of the aryl sulfamate derivatives or the pharmaceutically acceptable salts thereof according to any one of claims 1 to 25.

**28.** An anti-tumor agent comprising at least one of the aryl sulfamate derivatives or the pharmaceutically acceptable salts thereof according to any one of claims 1 to 25.

**29.** A therapeutic or prophylactic agent for a steroid sulfatase-related disease, comprising at least one of the aryl sulfamate derivatives or the pharmaceutically acceptable salts thereof according to any one of claims 1 to 25.

**30.** A therapeutic or prophylactic agent for a steroid hormone dependent disease, comprising at least one of the aryl sulfamate derivatives or the pharmaceutically acceptable salts thereof according to any one of claims 1 to 25.

**31.** A pharmaceutical composition comprising at least one of the aryl sulfamate derivatives or the pharmaceutically acceptable salts thereof according to any one of claims 1 to 25.

**32.** A method for treating or preventing a malignant tumor, comprising a step of administering a therapeutically effective amount of the aryl sulfamate derivative or the pharmaceutically acceptable salt thereof according to any one of claims 1 to 25.

**33.** A method for treating or preventing a disease selected from the group consisting of breast cancer, endometrial cancer, ovarian cancer, and prostate cancer, comprising a step of administering a therapeutically effective amount of the aryl sulfamate derivative or the pharmaceutically acceptable salt thereof according to any one of claims 1 to 25.

**34.** A method for treating or preventing a steroid hormone dependent disease, comprising a step of administering a therapeutically effective amount of the aryl sulfamate derivative or the pharmaceutically acceptable salt thereof according to any one of claims 1 to 25.

**35.** A method for treating or preventing a steroid sulfatase-related disease, comprising a step of administering a therapeutically effective amount of the aryl sulfamate derivative or the pharmaceutically acceptable salt thereof according to any one of claims 1 to 25.

**36.** Use of the aryl sulfamate derivative or the pharmaceutically acceptable salt thereof according to any one of claims 1 to 25 for the manufacture of an anti-tumor agent.

**37.** Use of the aryl sulfamate derivative or the pharmaceutically acceptable salt thereof according to any one of claims 1 to 25 for the manufacture of a therapeutic or prophylactic agent for a disease selected from the group consisting of breast cancer, endometrial cancer, ovarian cancer, and prostate cancer.

**38.** Use of the aryl sulfamate derivative or the pharmaceutically acceptable salt thereof according to any one of claims 1 to 25 for the manufacture of a therapeutic or prophylactic agent for a steroid hormone dependent disease.

**39.** Use of the aryl sulfamate derivative or the pharmaceutically acceptable salt thereof according to any one of claims 1 to 25 for the manufacture of a therapeutic or prophylactic agent for a steroid sulfatase-related disease.

**40.** Use of the aryl sulfamate derivative or the pharmaceutically acceptable salt thereof according to any one of claims 1 to 25 for the manufacture of a steroid sulfatase inhibitor.

## INTERNATIONAL SEARCH REPORT

International application No.
PCT/JP02/08665

**A. CLASSIFICATION OF SUBJECT MATTER**

Int.Cl[7] C07C307/02, 327/44, 317/46, A61K31/18, 31/277, A61P5/24, 13/08, 35/00, 43/00

According to International Patent Classification (IPC) or to both national classification and IPC

**B. FIELDS SEARCHED**

Minimum documentation searched (classification system followed by classification symbols)

Int.Cl[7] C07C307/02, 327/44, 317/46, A61K31/18, 31/277, A61P5/24, 13/08, 35/00, 43/00

Documentation searched other than minimum documentation to the extent that such documents are included in the fields searched

Electronic data base consulted during the international search (name of data base and, where practicable, search terms used)

REGISTRY(STN), CA(STN)

**C. DOCUMENTS CONSIDERED TO BE RELEVANT**

| Category* | Citation of document, with indication, where appropriate, of the relevant passages | Relevant to claim No. |
|---|---|---|
| X | US 6011024 A (Imperial College of Science Technology & Medicine), 04 January, 2000 (04.01.00), & EP 1099706 A2 | 1-31,36-40 |
| X | WO 97/06793 A1 (Duguesne University of the Holy Ghost), 27 February, 1997 (27.02.97), & EP 845985 A1 & JP 11-510813 A & US 5567831 A | 1-31,36-40 |

☐ Further documents are listed in the continuation of Box C. ☐ See patent family annex.

* Special categories of cited documents:

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| 12 November, 2002 (12.11.02) | 26 November, 2002 (26.11.02) |
| Name and mailing address of the ISA/ Japanese Patent Office | Authorized officer |
| Facsimile No. | Telephone No. |

Form PCT/ISA/210 (second sheet) (July 1998)

INTERNATIONAL SEARCH REPORT

International application No.
PCT/JP02/08665

**Box I Observations where certain claims were found unsearchable (Continuation of item 1 of first sheet)**

This international search report has not been established in respect of certain claims under Article 17(2)(a) for the following reasons:

1. [X] Claims Nos.: 32-35
because they relate to subject matter not required to be searched by this Authority, namely:
Claims 32 to 35 pertain to methods for treatment of the human body by therapy and thus relates to a subject matter which this International Searching Authority is not required, under the provisions of Article 17(2)(a)(i) of the PCT and Rule 39.1(iv) of the Regulations under the PCT, to search.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box II Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.

2. [ ] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** [ ] The additional search fees were accompanied by the applicant's protest.

[ ] No protest accompanied the payment of additional search fees.

Form PCT/ISA/210 (continuation of first sheet (1)) (July 1998)